# EUROPEAN PATENT SPECIFICATION

(11) **EP 2 434 012 B1**
(45) Date of publication and mention of the grant of the patent: **13.12.2017**
(21) Application number: 10777759.1
(22) Date of filing: 18.05.2010
(51) Int. Cl.: C12N 15/09, C12N 5/10, C12N 7/06, C12N 15/113, C12N 15/63, C12N 5/074, C12N 15/11, C12N 15/86, C12N 15/877, C12N 5/071

(54) **VECTOR MATERIAL FOR CREATING PLURIPOTENT STEM CELLS, AND PLURIPOTENT STEM CELL CREATION METHOD USING SAID VECTOR MATERIAL**
VEKTORMATERIAL ZUR ERZEUGUNG PLURIPOTENTER STAMMZELLEN SOWIE VERFAHREN ZUR ERZEUGUNG PLURIPOTENTER STAMMZELLEN MITHILFE DIESES VEKTORMATERIALS
VECTEUR PERMETTANT DE PRODUIRE DES CELLULES SOUCHES PLURIPOTENTES, ET PROCÉDÉ DE PRODUCTION DE CELLULES SOUCHES PLURIPOTENTES UTILISANT CE VECTEUR

(30) Priority: 18.05.2009 JP 2009119745
(43) Date of publication of application: 28.03.2012
(73) Proprietor: National Institute of Advanced Industrial Science and Technology, Tokyo 100-8921 (JP)
(72) Inventor: NAKANISHI, Mahito, Tsukuba-shi, Ibaraki 305-8565 (JP); NISHIMURA, Ken, Intellectual Property Administration Office, Tsukuba Central 1, 1-1, Umezono 1-chome, Tsukuba-shi, Ibaraki 305-8560 (JP); OHTAKA, Manami, Intellectual Property Administration Office, Tsukuba Central 1, 1-1, Umezono 1-chome, Tsukuba-shi, Ibaraki 305-8560 (JP); SANO, Masayuki, Tsukuba-shi, Ibaraki 305-8565 (JP)
(74) Representative: Gibbs, Richard
(86) International application number: PCT/JP2010/058368
(87) International publication number: WO 2010/134526

(56) References cited:
- WO-A1-2006/084746
- WO-A1-2008/129971
- WO-A1-2010/008054
- KEISUKE KAJI ET AL: "Virus-free induction of pluripotency and subsequent excision of reprogramming factors", NATURE: INTERNATIONAL WEEKLY JOURNAL OF SCIENCE (AND SUPPLEMENTARY INFORMATION), NATURE PUBLISHING GROUP, UNITED KINGDOM, vol. 458, no. 7239, 9 April 2009 (2009-04-09), pages 771-775, XP002568858, ISSN: 0028-0836, DOI: 10.1038/NATURE07864 [retrieved on 2009-03-01]
- INOUE MAKOTO ET AL: "RECOMBINANT SENDAI VIRUS VECTORS DELETED IN BOTH THE MATRIX AND THE FUSION GENES: EFFICIENT GENE TRANSFER WITH PREFERABLE PROPERTIES", JOURNAL OF GENE MEDICINE, 2010 , JOHN WILEY & SONS, INC, US, vol. 6, no. 10, 5 May 2004 (2004-05-05), pages 1069-1081, XP009077374, ISSN: 1099-498X, DOI: 10.1002/JGM.597
- RAGHU R. CHIVUKULA ET AL: "Abate and Switch: miR-145 in Stem Cell Differentiation", CELL, vol. 137, no. 4, May 2009 (2009-05), pages 606-608, XP055077400, ISSN: 0092-8674, DOI: 10.1016/j.cell.2009.04.059
- CAREY B W ET AL: "Reprogramming of murine and human somatic cells using a single polycistronic vector", PROCEEDINGS OF THE NATIONAL ACADEMY OF SCIENCES, NATIONAL ACADEMY OF SCIENCES, vol. 106, no. 1, 24 December 2008 (2008-12-24), pages 157-162, XP002555953, ISSN: 0027-8424, DOI: 10.1073/PNAS.0811426106 [retrieved on 2009-01-06]
- MARIKO YOSHIZAKI ET AL: "Naked Sendai virus vector lacking all of the envelope-related genes: reduced cytopathogenicity and immunogenicity", THE JOURNAL OF GENE MEDICINE, vol. 8, no. 9, September 2006 (2006-09), pages 1151-1159, XP055048809, ISSN: 1099-498X, DOI: 10.1002/jgm.938
- NOEMI FUSAKI ET AL: "Efficient induction of transgene-free human pluripotent stem cells using a vector based on Sendai virus, an RNA virus that does not integrate into the host genome", PROCEEDINGS OF THE JAPAN ACADEMY. SERIES B, PHYSICAL ANDBIOLOGICAL SCIENCES, TOKYO, JP, vol. 85, no. 8, October 2009 (2009-10), pages 348-362, XP002663242, ISSN: 0386-2208, DOI: DOI:10.2183/PJAB.85.348
- TOMOHISA SEKI ET AL: "Generation of Induced Pluripotent Stem Cells from Human Terminally Differentiated Circulating T Cells", CELL STEM CELL, CELL PRESS, US, vol. 7, no. 1, 2 July 2010 (2010-07-02), pages 11-14, XP008162341, ISSN: 1934-5909, DOI: 10.1016/J.STEM.2010.06.003 [retrieved on 2010-07-01]
- NISHIMURA K ET AL: "Development of defective and persistent Sendai virus vector: a unique gene delivery/expression system ideal for cell reprogramming", JOURNAL OF BIOLOGICAL CHEMISTRY, FEBRUARY 11, 2011,AMERICAN SOCIETY FOR BIOCHEMISTRY AND MOLECULAR BIOLOGY, INC, BETHESDA, MD, USA, vol. 286, no. 6, 7 December 2010 (2010-12-07), pages 4760-4771, XP002684867, ISSN: 1083-351X, DOI: 10.1074/JBC.M110.183780 [retrieved on 2010-12-07]
- HIROSHI BAN ET AL: "Efficient generation of transgene-free human induced pluripotent stem cells (iPSCs) by temperature-sensitive Sendai virus vectors", PROCEEDINGS OF THE NATIONAL ACADEMY OF SCIENCES, NATIONAL ACADEMY OF SCIENCES, vol. 108, no. 34, 23 August 2011 (2011-08-23), pages 14234-14239, XP002684868, ISSN: 0027-8424, DOI: 10.1073/PNAS.1103509108 [retrieved on 2011-08-05]
- RUI-JUN SU ET AL: "Efficient Generation of Integration-Free iPS Cells from Human Adult Peripheral Blood Using BCL-XL Together with Yamanaka Factors", PLOS ONE, vol. 8, no. 5, 21 May 2013 (2013-05-21), pages e64496-e64496, XP55078067, ISSN: 1932-6203, DOI: 10.1371/journal.pone.0064496
- KEN NISHIMURA ET AL.: 'Saiboshitsu Jizoku Hatsugengata RNA Vector no Seishitsu Kento to Iryo Oyo ni Muketa Kenkyu' JOURNAL OF JAPANESE BIOCHEMICAL SOCIETY 2008, page 4T26-7, XP008138458
- YU J ET AL.: 'Induced Pluripotent Stem Cell Lines Derived from Human Somatic Cells' SCIENCE vol. 318, 2007, pages 1917 - 1920, XP009105055
- NISHIO M ET AL.: 'Recombinant Sendai viruses with L1618V mutation in their L polymerase protein establish persistent infection, but not temperature sensitivity.' VIROLOGY vol. 329, no. 2, 2004, pages 289 - 301, XP004613860
- MASAYUKI SANO ET AL.: 'SiRNA o Mochiita Saibo kara no Sendai Virus Vector no Sentakuteki Jokyo' JOURNAL OF JAPANESE BIOCHEMICAL SOCIETY 2008, page 2P-1429, XP008148138
- FUSAKI N ET AL.: 'Efficient induction of transgene-free human pluripotent stem cells using a vector based on Sendai virus, an RNA virus that does not integrate into the host genome' PROC JPN ACAD SER B PHYS BIOL SCI vol. 85, no. 8, 2009, pages 348 - 362, XP002663242
- NISHIMURA K ET AL.: 'Persistent and Stable Gene Expression by a Cytoplasmic RNA Replicon Based on a Noncytopathic Variant Sendai Virus' J. BIOL. CHEM. vol. 282, 2007, pages 27383 - 27391, XP008135575

### SEQUENCE LISTING

<110> National Institute of Advanced Industrial Science and thechnology
<120> Vector materials for making of iPS cells, and making method of iPS
   cells with using above materials
<130> 2008003929
<160> 46
<170> PatentIn version 3.1
<210> 1
   <211> 2652
   <212> DNA
   <213> Artificial
<220>
   <223> Codon-optimaized T7 RNA polymerase
<400> 1
   atgaacacca tcaacattgc caaaaacgat ttcagcgaca ttgagctggc cgccatcccc      60
   ttcaataccc tggccgatca ctacggggag cggctggcca gggagcagct ggccctggag     120
   cacgagtctt acgagatggg cgaggcccgg ttccggaaaa tgtttgaacg ccagctgaaa     180
   gccggagaag tggccgataa cgccgccgcc aagcctctga ttaccaccct gctgcccaag     240
   atgattgccc ggattaacga ttggttcgaa gaggtgaaag ccaagagggg caagagacct     300
   accgcctttc agtttctgca ggaaatcaaa cctgaagccg tggcctacat caccattaag     360
   accacactgg cctgcctgac cagcgccgac aacaccacag tgcaggccgt ggccagcgcc     420
   atcggcagag ccatcgagga cgaagccagg ttcgggagga tcagggatct ggaggccaag     480
   cacttcaaga aaaatgtgga agagcagctg aataagcggg tgggccacgt gtacaagaag     540
   gccttcatgc aggtggtgga agccgatatg ctgagcaaag gcctgctggg cggagaagcc     600
   tggagctctt ggcacaagga agatagcatt cacgtgggcg tgagatgtat tgaaatgctg     660
   attgagagca cagggatggt gtccctgcac cggcagaacg ccggagtggt gggccaggat     720
   agcgagacca tcgagctggc ccccgaatat gccgaggcca ttgccacaag agccggggcc     780
   ctggccggga tctctccaat gttccagcca tgcgtggtgc ctccaaagcc atggacaggc     840
   atcaccggag gggctactgg ggccaatggg cgcaggcctc tggccctggt gaggacccac     900
   agcaaaaagg ccctgatgcg ctacgaggac gtgtacatgc ctgaggtgta caaagccatc     960
   aacattgccc agaacaccgc ctggaagatc aacaagaaag tgctggccgt ggccaatgtg    1020
   attaccaagt ggaagcactg tccagtggaa gacatccctg ccatcgagcg cgaggaactg    1080
   cctatgaagc ccgaggacat tgatatgaac cccgaagccc tgacagcctg gaagagagcc    1140
   gccgccgccg tgtaccgcaa agatcgcgcc cggaagtcta ggagaatttc cctggagttc    1200
   atgctggagc aggccaataa gttcgccaac cacaaggcca tctggttccc ctacaatatg    1260
   gattggcgcg gccgggtgta tgccgtgtcc atgttcaatc cccagggcaa cgacatgacc    1320
   aaaggcctgc tgacactggc caagggcaag cccatcggca aggaaggata ttattggctg 1380
   aagatccacg gcgccaattg tgccggggtg gacaaagtgc cttttcctga aaggatcaag 1440
   ttcatcgagg agaaccacga gaacatcatg gcctgtgcca aatctcccct ggagaacacc 1500
   tggtgggccg aacaggactc tcctttctgc tttctggcct tttgtttcga gtacgccggg 1560
   gtgcagcacc acggcctgtc ctacaattgc tctctgcctc tggcctttga cggctcttgc 1620
   tccgggattc agcactttag cgccatgctg cgggacgaag tgggcggaag ggccgtgaat 1680
   ctgctgccct ccgaaaccgt gcaggatatc tacggcatcg tggccaagaa agtgaatgaa 1740
   atcctgcagg ccgatgccat caacgggaca gataacgaag tggtgaccgt gacagacgag 1800
   aatacaggcg agattagcga aaaagtgaaa ctggggacca aggccctggc cggccagtgg 1860
   ctggcccacg gcgtgacaag gtctgtgacc aagcgcagcg tgatgaccct ggcctacggc 1920
   tccaaagagt tcgggttcag acagcaggtg ctggaagaca caatccagcc tgccatcgac 1980
   agcggaaagg ggcccatgtt cacccagcca aaccaggccg ccggctatat ggccaagctg 2040
   atctgggaaa gcgtgtctgt gacagtggtg gccgccgtgg aggccatgaa ttggctgaag 2100
   agcgccgcca agctgctggc cgccgaagtg aaagacaaga agacaggaga gattctgagg 2160
   aagaggtgcg ccgtgcactg ggtgacccca gatggattcc ccgtgtggca ggagtacaag 2220
   aaaccaatcc agaccaggct gaatctgatg ttcctgggcc agtttcgcct gcagccaaca 2280
   attaacacca acaaggattc cgagattgat gcccacaagc aggaatctgg catcgccccc 2340
   aactttgtgc actctcagga tgggtctcac ctgaggaaga ccgtggtgtg ggcccacgaa 2400
   aaatatggaa ttgagtcctt tgccctgatt cacgattcct ttggcacaat ccctgccgac 2460
   gccgccaacc tgttcaaggc cgtgagagaa accatggtgg atacctacga atcttgcgat 2520
   gtgctggccg atttctacga ccagttcgcc gatcagctgc acgagtccca gctggacaag 2580
   atgcccgccc tgcccgccaa aggcaacctg aacctgcggg atattctgga gagcgatttt 2640
   gccttcgcct aa 2652
<210> 2
   <211> 2129
   <212> DNA
   <213> Artificial
<220>
   <223> Optimized human Oct4,human Sox2 and a part of Sendai virus genome cDNA
<400> 2
   cctaggttcc ccatggcggg acacctggct tcggatttcg ccttctcgcc ccctccaggt    60
   ggtggaggtg atgggccagg ggggccggag ccgggctggg ttgatcctcg gacctggcta   120
   agcttccaag gccctcctgg agggccagga atcgggccgg gggttgggcc aggctctgag   180
   gtgtggggga ttcccccatg ccccccgccg tatgagttct gtggggggat ggcgtactgt    240
   gggccccagg ttggagtggg gctagtgccc caaggcggct tggagacctc tcagcctgag 300
   ggcgaagcag gagtcggggt ggagagcaac tccgatgggg cctccccgga gccctgcacc 360
   gtcacccctg gtgccgtgaa gctggagaag gagaagctgg agcaaaaccc ggaggagtcc 420
   caggacatca aagctctgca gaaagaactc gagcaatttg ccaagctcct gaagcagaag 480
   aggatcaccc tgggatatac acaggccgat gtggggctca ccctgggggt tctatttggg 540
   aaggtattca gccaaacgac catctgccgc tttgaggctc tgcagcttag cttcaagaac 600
   atgtgtaagc tgcggccctt gctgcagaag tgggtggagg aagctgacaa caatgaaaat 660
   cttcaggaga tatgcaaagc agaaaccctc gtgcaggccc gaaagagaaa gcgaaccagt 720
   atcgagaacc gagtgagagg caacctggag aatttgttcc tgcagtgccc gaaacccaca 780
   ctgcagcaga tcagccacat cgcccagcag cttgggctcg agaaggatgt ggtccgagtg 840
   tggttctgta accggcgcca gaagggcaag cgatcaagca gcgactatgc acaacgagag 900
   gattttgagg ctgctgggtc tcctttctca gggggaccag tgtcctttcc tctggcccca 960
   gggccccatt ttggtacccc aggctatggg agccctcact tcactgcact gtactcctcg 1020
   gtccctttcc ctgaggggga agcctttccc cctgtctccg tcaccactct gggctctccc 1080
   atgcattcaa actgaggacg tcagatctgt atataataag aaaaacttag ggtgaaagtg 1140
   aggttgcgcg gtattttagc tagcccgcat gtacaacatg atggagacgg agctgaagcc 1200
   gccgggcccg cagcaaactt cggggggcgg cggcggcaac tccaccgcgg cggcggccgg 1260
   cggcaaccag aaaaacagcc cggaccgcgt caagcggccc atgaatgcct tcatggtgtg 1320
   gtcccgcggg cagcggcgca agatggccca ggagaacccc aagatgcaca actcggagat 1380
   cagcaagcgc ctgggcgccg agtggaaact tttgtcggag acggagaagc ggccgttcat 1440
   cgacgaggct aagcggctgc gagcgctgca catgaaggag cacccggatt ataaataccg 1500
   gccccggcgg aaaaccaaga cgctcatgaa gaaggataag tacacgctgc ccggcgggct 1560
   gctggccccc ggcggcaata gcatggcgag cggggtcggg gtgggcgccg gcctgggcgc 1620
   gggcgtgaac cagcgcatgg acagttacgc gcacatgaac ggctggagca acggcagcta 1680
   cagcatgatg caggaccagc tgggctaccc gcagcacccg ggcctcaatg cgcacggcgc 1740
   agcgcagatg cagcccatgc accgctacga cgtgagcgcc ctgcagtaca actccatgac 1800
   cagctcgcag acctacatga acggctcgcc cacctacagc atgtcctact cgcagcaggg 1860
   cacccctggc atggctcttg gctccatggg ttcggtggtc aagtccgagg ccagctccag 1920
   cccccctgtg gttacctctt cctcccactc cagggcgccc tgccaggccg gggacctccg 1980
   ggacatgatc agcatgtatc tccccggcgc cgaggtgccg gaacccgccg cccccagcag 2040
   acttcacatg tcccagcact accagagcgg cccggtgccc ggcacggcca ttaacggcac 2100
   actgcccctc tcacacatgt gagaccggt 2129
<210> 3
   <211> 5851
   <212> DNA
   <213> Artificial
<220>
   <223> pMO078
<400> 3
   gaattcgagt catcccgaga cgcgagttat gtgtttgcaa gacgtgccct aaagtctgca 60
   aactatgcag agatgacatt caatgtatgc ggcctgatcc tttctgccga gaaatcttcc 120
   gctcgtaagg tagatgagaa caaacaactg ctcaaacaga tccaagagag cgtggaatca 180
   ttccgggata tttacaagag attctctgag tatcagaaag aacagaactc attgctgatg 240
   tccaacctat ctacacttca tatcatcaca gatagaggtg gcaagactga caacacagac 300
   tcccttacaa ggtccccctc cgtttttgca aaatcaaaag agaacaagac taaggctacc 360
   aggtttgacc catctatgga gaccttagaa gatatgaagt acaaaccgga cctaatccga 420
   gaggatgaat ttagagatga gatccgcaac ccggtgtacc aagagaggga cacagaaccc 480
   agggcctcaa acgcatcacg tctcttcccc tccaaagaga agcacacaat gcactctctc 540
   aggctcgtca tagagagcag tcccctaagc agagctgaga aagcagcata tgtgaaatca 600
   ttatccaagt gcaagacaga ccaagaggtt aaggcagtca tggaactcgt agaagaggac 660
   atagagtcac tgaccaacta gatcccgggt gaggcatccc accatcctca gtcacagaga 720
   gacccaatct accatcagca tcagccagta aagattaaga aaaacttagg gtgaaagaaa 780
   tgcggccgct tggcgccaga atatatgaaa acatttaaca tttctcaaca agatctagaa 840
   ttagtagaag tagcgacaga gaagattaca atgctttatg aggataataa acatcatgtg 900
   ggagcggcaa ttcgtacgaa aacaggagaa atcatttcgg cagtacatat tgaagcgtat 960
   ataggacgag taactgtttg tgcagaagcc attgccattg gtagtgcagt ttcgaatgga 1020
   caaaaggatt ttgacacgat tgtagctgtt agacaccctt attctgacga agtagataga 1080
   agtattcgag tggtaagtcc ttgtggtatg tgtagggagt tgatttcaga ctatgcacca 1140
   gattgttttg tgttaataga aatgaatggc aagttagtca aaactacgat tgaagaactc 1200
   attccactca aatatacccg aaattaaaac gcgtcagaga cctgcaacaa tgtctcaagc 1260
   agacaccacc tggcagtcgg agccaccggg tcactccttg tcttaaataa gaaaaactta 1320
   gggataaagt cccttagatc tagcctaggg ggaccatggt gagcgtgatc aagcccgaga 1380
   tgaagatgaa gtacttcatg gacggcagcg tgaacggcca cgagttcacc gtggagggcg 1440
   agggcaccgg caagccctac gagggccacc aggagatgac cctgagggtg acaatggcca 1500
   agggcggccc catgcccttc agcttcgacc tggtgagcca caccttctgc tacggccaca 1560
   ggcccttcac caagtacccc gaggagatcc ccgactactt caagcaggcc ttccccgagg 1620
   gcctgagctg ggagaggagc ctccagttcg aggacggcgg cttcgccgcc gtgagcgccc 1680
   acatcagcct gaggggcaac tgcttcgagc acaagagcaa gttcgtgggc gtgaacttcc 1740
   ccgccgacgg ccccgtgatg cagaaccaga gcagcgactg ggagcccagc accgagaaga 1800
   tcaccacctg cgacggcgtg ctgaagggcg acgtgaccat gtacctgaag ctggccggcg 1860
   gcggcaacca caagtgccag ttcaagacca cctacaaggc cgccaagaag atcctgaaga 1920
   tgccccagag ccacttcatc ggccacaggc tggtgaggaa gaccgagggc aacatcaccg 1980
   agctggtgga ggacgccgtg gcccactgct gaagacgtca gatctgtata taataagaaa 2040
   aacttagggt gaaagtgagg ttgcgcggta ttttagctag ctgccaccat ggggaactgg 2100
   gctgtgaatg aggggctctc catttttgtc attctggttt ggctggggtt gaacgtcttc 2160
   ctctttgtct ggtattaccg ggtttatgat attccaccta agttctttta cacaagaaaa 2220
   cttcttgggt cagcactggc actggccagg gcccctgcag cctgcctgaa tttcaactgc 2280
   atgctgattc tcttgccagt ctgtcgaaat ctgctgtcct tcctcagggg ttccagtgcg 2340
   tgctgctcaa caagagttcg aagacaactg gacaggaatc tcacctttca taaaatggtg 2400
   gcatggatga ttgcacttca ctctgcgatt cacaccattg cacatctatt taatgtggaa 2460
   tggtgtgtga atgcccgagt caataattct gatccttatt cagtagcact ctctgaactt 2520
   ggagacaggc aaaatgaaag ttatctcaat tttgctcgaa agagaataaa gaaccctgaa 2580
   ggaggcctgt acctggctgt gaccctgttg gcaggcatca ctggagttgt catcacgctg 2640
   tgcctcatat taattatcac ttcctccacc aaaaccatcc ggaggtctta ctttgaagtc 2700
   ttttggtaca cacatcatct ctttgtgatc ttcttcattg gccttgccat ccatggagct 2760
   gaacgaattg tacgtgggca gaccgcagag agtttggctg tgcataatat aacagtttgt 2820
   gaacaaaaaa tctcagaatg ggaaaaaata aaggaatgcc caatccctca gtttgctgga 2880
   aaccctccta tgacttggaa atggatagtg ggtcccatgt ttctgtatct ctgtgagagg 2940
   ttggtgcggt tttggcgatc tcaacagaag gtggtcatca ccaaggtggt cactcaccct 3000
   ttcaaaacca tcgagctaca gatgaagaag aaggggttca aaatggaagt gggacaatac 3060
   atttttgtca agtgcccaaa ggtgtccaag ctggagtggc acccttttac actgacatcc 3120
   gcccctgagg aagacttctt tagtatccat atccgcatcg ttggggactg gacagagggg 3180
   ctgttcaatg cttgtggctg tgataagcag gagtttcaag atgcgtggaa actacctaag 3240
   atagcggttg atgggccctt tggcactgcc agtgaagatg tgttcagcta tgaggtggtg 3300
   atgttagtgg gagcagggat tggggtcaca cccttcgcat ccattctcaa gtcagtctgg 3360
   tacaaatatt gcaataacgc caccaatctg aagctcaaaa agatctactt ctactggctg 3420
   tgccgggaca cacatgcctt tgagtggttt gcagatctgc tgcaactgct ggagagccag 3480
   atgcaggaaa ggaacaatgc cggcttcctc agctacaaca tctacctcac tggctgggat 3540
   gagtctcagg ccaatcactt tgctgtgcac catgatgagg agaaagatgt gatcacaggc 3600
   ctgaaacaaa agactttgta tggacggccc aactgggata atgaattcaa gacaattgca 3660
   agtcaacacc ctaataccag aataggagtt ttcctctgtg gacctgaagc cttggctgaa 3720
   accctgagta aacaaagcat ctccaactct gagtctggcc ctcggggagt gcatttcatt 3780
   ttcaacaagg aaaacttcta acaccggtgt cggctttgct gacactagag tcatctccga 3840
   acatccacaa tatctctcag tctcttacgt ctctcacagt attaagaaaa acccagggtg 3900
   aatgggaagc ttgccatagg tcatggatgg gcaggagtcc tcccaaaacc cttctgacat 3960
   actctatcca gaatgccacc tgaactctcc catagtcagg gggaagatag cacagttgca 4020
   cgtcttgtta gatgtgaacc agccctacag actaaaggac gacagcataa taaatattac 4080
   aaagcacaaa attaggaacg gaggattgtc ccctcgtcaa attaagatca ggtctctggg 4140
   taaggctctt caacgcacaa taaaggattt agaccgatac acctttgaac cgtacccaac 4200
   ctactctcag gaattactta ggcttgatat accagagata tgtgacaaaa tccgatccgt 4260
   cttcgcggtc tcggatcggc tgaccaggga gttatctagt gggttccagg atctttggtt 4320
   gaatatcttc aagcaactag gcaatataga aggaagagag gggtacgatc cgttgcagga 4380
   tatcggcacc atcccggaga taactgataa atacagcagg aatagatggt ataggccatt 4440
   cctaacttgg ttcagcatca aatatgacat gcggtggatg cagaagacca gaccgggggg 4500
   acccctcgat acctctaatt cacataacct cctagaatgc aaatcataca ctctagtaac 4560
   atacggagat cttatcatga tactgaacaa gttgacattg acagggtata tcctaacccc 4620
   tgagctggtc ttgatgtatt gtgatgttgt agagggaagg tggaatatgt ctgctgcagg 4680
   gcatctagat aagaagtcca ttgggataac aagcaaaggt gaggaattat gggaactagt 4740
   ggattccctc ttctcaagtc ttggagagga aatatacaat gtcatcgcac tattggagcc 4800
   cctatcactt gctctcatac aactaaatga tccagttata cctctacgtg gggcatttat 4860
   gaggcatgtg ttgacagagc tacaggctgt tttaacaagt agggacgtgt acacagatgc 4920
   tgaagcagac actattgtgg agtcgttact cgccattttc catggaacct ctattgatga 4980
   gaaagcagag atcttttcct tctttaggac atttggccac cccagcttag aggctgtcac 5040
   tgccgccgac aaggtaaggg cccatatgta tgcacaaaag gcaataaagc ttaagaccct 5100
   atacgagtgt catgcagttt tttgcactat catcataaat gggtatagag agaggcatgg 5160
   cggacagtgg cccccctgtg acttccctga tcacgtgtgt ctagaactaa ggaacgctca 5220
   agggtccaat acggcaatct cttatgaatg tgctgtagac aactatacaa gtttcatagg 5280
   cttcaagttt cggaagttta tagaaccaca actagatgaa gatctcacaa tatatatgaa 5340
   agacaaagca ctatccccca ggaaggaggc atgggactct gtatacccgg atagtaatct 5400
   gtactataaa gccccagaat ctgaagagac ccggcggctt attgaagtgt tcataaatga 5460
   tgagaatttc aacccagaag aaattatcaa ttatgtggag tcaggagatt ggttgaaaga 5520
   cgagaagttc aacatctcgt acagtctcaa agagaaagag atcaagcaag agggtcgtct 5580
   attcgcaaaa atgacttata agatgcgagc cgtacaggtg ctggcagaga cactactggc   5640
   taaaggaata ggagagctgt tcagcgaaaa tgggatggtt aaaggagaga tagacctact   5700
   taaaagattg actactcttt ctgtctcagg agtccccagg actgattcag tgtacaataa   5760
   ctctaaatca tcagagaaga gaaacgaagg catgaaaaag aagaactctg gggggtactg   5820
   ggacgaaaag aagaggtcca gacatgaatt c                                  5851
<210> 4
   <211> 1478
   <212> DNA
   <213> Artificial
<220>
   <223> Optimized human Klf4 and a part of Sendai virus genome cDNA
<400> 4
   gctagcacct aggtctgaca tggctgtcag cgacgcgctg ctcccatctt tctccacgtt 60
   cgcgtctggc ccggcgggaa gggagaagac actgcgtcaa gcaggtgccc cgaataaccg 120
   ctggcgggag gagctctccc acatgaagcg acttccccca gtgcttcccg gccgccccta 180
   tgacctggcg gcggcgaccg tggccacaga cctggagagc ggcggagccg gtgcggcttg 240
   cggcggtagc aacctggcgc ccctacctcg gagagagacc gaggagttca acgatctcct 300
   ggacctggac tttattctct ccaattcgct gacccatcct ccggagtcag tggccgccac 360
   cgtgtcctcg tcagcgtcag cctcctcttc gtcgtcgccg tcgagcagcg gccctgccag 420
   cgcgccctcc acctgcagct tcacctatcc gatccgggcc gggaacgacc cgggcgtggc 480
   gccgggcggc acgggcggag gcctcctcta tggcagggag tccgctcccc ctccgacggc 540
   tcccttcaac ctggcggaca tcaacgacgt gagcccctcg ggcggcttcg tggccgagct 600
   cctgcggcca gaattggacc cggtgtacat tccgccgcag cagccgcagc cgccaggtgg 660
   cgggctgatg ggcaagttcg tgctgaaggc gtcgctgagc gcccctggca gcgagtacgg 720
   cagcccgtcg gtcatcagcg tcagcaaagg cagccctgac ggcagccacc cggtggtggt 780
   ggcgccctac aacggcgggc cgccgcgcac gtgccccaag atcaagcagg aggcggtctc 840
   ttcgtgcacc cacttgggcg ctggaccccc tctcagcaat ggccaccggc cggctgcaca 900
   cgacttcccc ctggggcggc agctccccag caggactacc ccgaccctgg gtcttgagga 960
   agtgctgagc agcagggact gtcaccctgc cctgccgctt cctcccggct tccatcccca 1020
   cccggggccc aattacccat ccttcctgcc cgatcagatg cagccgcaag tcccgccgct 1080
   ccattaccaa gagctcatgc cacccggttc ctgcatgcca gaggagccca agccaaagag 1140
   gggaagacga tcgtggcccc ggaaaaggac cgccacccac acttgtgatt acgcgggctg 1200
   cggcaaaacc tacacaaaga gttcccatct caaggcacac ctgcgaaccc acacaggtga 1260
   gaaaccttac cactgtgact gggacggctg tggatggaaa ttcgcccgct cagatgaact 1320
   gaccaggcac taccgtaaac acacggggca ccgcccgttc cagtgccaaa aatgcgaccg 1380
   agcattttcc aggtcggacc acctcgcctt acacatgaag aggcattttt aaagacgtcg 1440
   attaagaaaa acttagggtg aaagttcatc gcggccgc 1478
<210> 5
   <211> 3696
   <212> DNA
   <213> Artificial
<220>
   <223> A part of Sendai virus genome cDNA : Xhol-T7pro-SevcDNA-Nhel-Not1
<400> 5
   ctcgagtaat acgactcact atagggacca aacaagagaa gaaacatgta tggaatatat 60
   aatgaagtta gacaggattt tagggtcaaa gtatccaccc tgaggagcag gttccagatc 120
   cttttctttg ctgccaaagt tcacgatggc cgggttgttg agcaccttcg atacatttag 180
   ctctaggagg agcgaaagta ttaataagtc gggaggaggt gctgttatcc ccggccagag 240
   gagcacagtc tcagtgttca tactaggccc aagtgtgact gatgatgcag acaagttatt 300
   cattgcaaca accttcctag ctcactcatt ggacacagat aagcagcact ctcagagagg 360
   agggttcctc gtctctctgc ttgccatggc ttacagtagt ccagaattgt acttgacaac 420
   aaacggagta aacgccgatg tcaaatatgt gatctacaac atagagaaag accctaagag 480
   gacgaagaca gacggattca ttgtgaagac gagagatatg gaatatgaga ggaccacaga 540
   atggctgttt ggacctatgg tcaacaagag cccactcttc cagggtcaac gggatgctgc 600
   agaccctgac acactccttc aaatctatgg gtatcctgca tgcctaggag caataattgt 660
   ccaagtctgg attgtgctgg tgaaggccat cacaagcagc gccggcttaa ggaaagggtt 720
   cttcaacagg ttagaggcgt tcagacaaga cggcaccgtg aaaggtgcct tagttttcac 780
   tggggagaca gttgagggga taggctcggt tatgagatct cagcaaagcc ttgtatctct 840
   catggttgag acccttgtga ctatgaatac tgcaagatct gatctcacca cattagagaa 900
   gaacatccag atcgttggga actacatccg agatgcaggg ctggcttcct tcatgaacac 960
   tattaaatat ggggtggaga caaagatggc agctctaacg ttgtcaaacc tgaggcccga 1020
   tattaataag cttagaagcc tcatagacac ctacctgtca aaaggcccca gagctccctt 1080
   tatctgtatc ctcaaggacc ctgttcatgg tgaatttgct ccaggcaatt atcctgcact 1140
   atggagttac gccatgggag tcgccgtcgt acagaacaag tcaatgcagc agtacgtcac 1200
   agggaggaca taccttgata tggaaatgtt cttactagga caagccgtgg caaaggatgc 1260
   tgaatcgaag atcagcagtg ccttggaaga tgagttagga gtgacggata cagccaagga 1320
   gaggctcaga catcatctgg caaacttgtc cggtggggat ggtgcttacc acaaaccaac 1380
   aggcggtggt gcaattgagg tagctctaga caatgccgat atcgacctgg aaacagaagc 1440
   tcatgcggac caggacgcta ggggttgggg tggagatagt ggtgaaagat gggcacgtca 1500
   ggtgagtggt ggccactttg tcacactaca tggggctgaa cggttagagg aggaaaccaa 1560
   tgatgaggat gtatcagaca tagagagaag aatagccatg agactcgcag agagacggca 1620
   agaggattct gcaacccatg gagatgaagg ccgcaataac ggtgttgatc acgaagaaga 1680
   tgacgatgcc gcagcagcag ctgggatagg aggaatctag gatcatacga ggcctcaagg 1740
   tacttgatcc gcagtaagaa aaacttaggg tgaaagttca tccaccgatc ggctcaggca 1800
   aggccacacc caaccccacc gaccacaccc agcagtcgag acagccacgg cttcggctac 1860
   acttaccgca tggatcaaga tgccttcatt cttaaagaag attctgaagt tgagaggaag 1920
   gcgccaggag gacgagagtc gctctcggat gttatcggat tcctcgatgc tgtcctgtcg 1980
   aatgaaccaa ctgacatcgg aggggacaga agctggctcc acaacaccat caacactccc 2040
   caaggaccag gctctgctca tagagccaaa agtgagggcg aaggagaagt ctcaacaccg 2100
   tcgacccaag ataatcgatc aggtgaggag agtagagtct ctgggagaac aagcaagcca 2160
   gaggcagaag cacatgctgg aaaccttgat aaacaaaata tacactgggc ctttagggga 2220
   agaactggta caaactctgt atctcaggat ctggacgatg gaggagactc cggaatcctt 2280
   gaaaatcctc caaatgagag aggatatccg agatcaggta ttgaagatga aaacagagag 2340
   atggctgcgc accctgataa gaggggagaa gaccaagctg aaggacttcc agaagaggta 2400
   cgaggaggta catccctacc tgatgaagga gaaggtggag caagtaataa tggaagaagc 2460
   atggagcctg gcagctcaca tagtgcaaga gtaactgggg tcctggtgat tcctagcccc 2520
   gaacttgaag aggctgtgct acggaggaac aaaagaagac ctaccaacag tgggtccaaa 2580
   cctcttactc cagcaaccgt gcctggcacc cggtccccac cgctgaatcg ttacaacagc 2640
   acagggtcac caccaggaaa acccccatct acacaggatg agcacatcaa ctctggggac 2700
   acccccgccg tcagggtcaa agaccggaaa ccatcaatag ggactcgctc tgtctcagat 2760
   tgtccagcca acggccgccc aatccatccg ggtatagaga ccgactcaac aaaaaagggc 2820
   ataggagaga acacatcatc tatgaaagat atggctacat tgttgacgag tcttggtgta 2880
   atccagtctg ctcaagaatt cgagtcatcc cgagacgcga gttatgtgtt tgcaagacgt 2940
   gccctaaagt ctgcaaacta tgcagagatg acattcaatg tatgcggcct gatcctttct 3000
   gccgagaaat cttccgctcg taaggtagat gagaacaaac aactgctcaa acagatccaa 3060
   gagagcgtgg aatcattccg ggatatttac aagagattct ctgagtatca gaaagaacag 3120
   aactcattgc tgatgtccaa cctatctaca cttcatatca tcacagatag aggtggcaag 3180
   actgacaaca cagactccct tacaaggtcc ccctccgttt ttgcaaaatc aaaagagaac 3240
   aagactaagg ctaccaggtt tgacccatct atggagacct tagaagatat gaagtacaaa 3300
   ccggacctaa tccgagagga tgaatttaga gatgagatcc gcaacccggt gtaccaagag 3360
   agggacacag aacccagggc ctcaaacgca tcacgtctct tcccctccaa agagaagccc 3420
   acaatgcact ctctcaggct cgtcatagag agcagtcccc taagcagagc tgagaaagca   3480
   gcatatgtga aatcattatc caagtgcaag acagaccaag aggttaaggc agtcatggaa   3540
   ctcgtagaag aggacataga gtcactgacc aactagatcc cgggtgaggc atcccaccat   3600
   cctcagtcac agagagaccc aatctaccat cagcatcagc cagtaaagat taagaaaaac   3660
   ttagggtgaa agaaatttca ccgctagcgc ggccgc                             3696
<210> 6
   <211> 14760
   <212> DNA
   <213> Artificial
<220>
   <223> Template cDNA for SeVp(Mp+Klf4,delta-M::Bsr,delta-F::Oct4,delta-H N::Sox2)
<400> 6
   accaaacaag agaagaaaca tgtatggaat atataatgaa gttagacagg attttagggt      60
   caaagtatcc accctgagga gcaggttcca gatccttttc tttgctgcca aagttcacga      120
   tggccgggtt gttgagcacc ttcgatacat ttagctctag gaggagcgaa agtattaata      180
   agtcgggagg aggtgctgtt atccccggcc agaggagcac agtctcagtg ttcatactag      240
   gcccaagtgt gactgatgat gcagacaagt tattcattgc aacaaccttc ctagctcact      300
   cattggacac agataagcag cactctcaga gaggagggtt cctcgtctct ctgcttgcca      360
   tggcttacag tagtccagaa ttgtacttga caacaaacgg agtaaacgcc gatgtcaaat      420
   atgtgatcta caacatagag aaagacccta agaggacgaa gacagacgga ttcattgtga      480
   agacgagaga tatggaatat gagaggacca cagaatggct gtttggacct atggtcaaca      540
   agagcccact cttccagggt caacgggatg ctgcagaccc tgacacactc cttcaaatct      600
   atgggtatcc tgcatgccta ggagcaataa ttgtccaagt ctggattgtg ctggtgaagg      660
   ccatcacaag cagcgccggc ttaaggaaag ggttcttcaa caggttagag gcgttcagac      720
   aagacggcac cgtgaaaggt gccttagttt tcactgggga gacagttgag gggataggct      780
   cggttatgag atctcagcaa agccttgtat ctctcatggt tgagaccctt gtgactatga      840
   atactgcaag atctgatctc accacattag agaagaacat ccagatcgtt gggaactaca      900
   tccgagatgc agggctggct tccttcatga acactattaa atatggggtg gagacaaaga      960
   tggcagctct aacgttgtca aacctgaggc ccgatattaa taagcttaga agcctcatag     1020
   acacctacct gtcaaaaggc cccagagctc cctttatctg tatcctcaag gaccctgttc     1080
   atggtgaatt tgctccaggc aattatcctg cactatggag ttacgccatg ggagtcgccg     1140
   tcgtacagaa caagtcaatg cagcagtacg tcacagggag gacatacctt gatatggaaa     1200
   tgttcttact aggacaagcc gtggcaaagg atgctgaatc gaagatcagc agtgccttgg     1260
   aagatgagtt aggagtgacg gatacagcca aggagaggct cagacatcat ctggcaaact     1320
   tgtccggtgg ggatggtgct taccacaaac caacaggcgg tggtgcaatt gaggtagctc 1380
   tagacaatgc cgatatcgac ctggaaacag aagctcatgc ggaccaggac gctaggggtt 1440
   ggggtggaga tagtggtgaa agatgggcac gtcaggtgag tggtggccac tttgtcacac 1500
   tacatggggc tgaacggtta gaggaggaaa ccaatgatga ggatgtatca gacatagaga 1560
   gaagaatagc catgagactc gcagagagac ggcaagagga ttctgcaacc catggagatg 1620
   aaggccgcaa taacggtgtt gatcacgaag aagatgacga tgccgcagca gcagctggga 1680
   taggaggaat ctaggatcat acgaggcctc aaggtacttg atccgcagta agaaaaactt 1740
   agggtgaaag ttcatccacc gatcggctca ggcaaggcca cacccaaccc caccgaccac 1800
   acccagcagt cgagacagcc acggcttcgg ctacacttac cgcatggatc aagatgcctt 1860
   cattcttaaa gaagattctg aagttgagag gaaggcgcca ggaggacgag agtcgctctc 1920
   ggatgttatc ggattcctcg atgctgtcct gtcgaatgaa ccaactgaca tcggagggga 1980
   cagaagctgg ctccacaaca ccatcaacac tccccaagga ccaggctctg ctcatagagc 2040
   caaaagtgag ggcgaaggag aagtctcaac accgtcgacc caagataatc gatcaggtga 2100
   ggagagtaga gtctctggga gaacaagcaa gccagaggca gaagcacatg ctggaaacct 2160
   tgataaacaa aatatacact gggcctttag gggaagaact ggtacaaact ctgtatctca 2220
   ggatctggac gatggaggag actccggaat ccttgaaaat cctccaaatg agagaggata 2280
   tccgagatca ggtattgaag atgaaaacag agagatggct gcgcaccctg ataagagggg 2340
   agaagaccaa gctgaaggac ttccagaaga ggtacgagga ggtacatccc tacctgatga 2400
   aggagaaggt ggagcaagta ataatggaag aagcatggag cctggcagct cacatagtgc 2460
   aagagtaact ggggtcctgg tgattcctag ccccgaactt gaagaggctg tgctacggag 2520
   gaacaaaaga agacctacca acagtgggtc caaacctctt actccagcaa ccgtgcctgg 2580
   cacccggtcc ccaccgctga atcgttacaa cagcacaggg tcaccaccag gaaaaccccc 2640
   atctacacag gatgagcaca tcaactctgg ggacaccccc gccgtcaggg tcaaagaccg 2700
   gaaaccatca atagggactc gctctgtctc agattgtcca gccaacggcc gcccaatcca 2760
   tccgggtata gagaccgact caacaaaaaa gggcatagga gagaacacat catctatgaa 2820
   agatatggct acattgttga cgagtcttgg tgtaatccag tctgctcaag aattcgagtc 2880
   atcccgagac gcgagttatg tgtttgcaag acgtgcccta aagtctgcaa actatgcaga 2940
   gatgacattc aatgtatgcg gcctgatcct ttctgccgag aaatcttccg ctcgtaaggt 3000
   agatgagaac aaacaactgc tcaaacagat ccaagagagc gtggaatcat tccgggatat 3060
   ttacaagaga ttctctgagt atcagaaaga acagaactca ttgctgatgt ccaacctatc 3120
   tacacttcat atcatcacag atagaggtgg caagactgac aacacagact cccttacaag 3180
   gtccccctcc gtttttgcaa aatcaaaaga gaacaagact aaggctacca ggtttgaccc 3240
   atctatggag accttagaag atatgaagta caaaccggac ctaatccgag aggatgaatt 3300
   tagagatgag atccgcaacc cggtgtacca agagagggac acagaaccca gggcctcaaa 3360
   cgcatcacgt ctcttcccct ccaaagagaa gcccacaatg cactctctca ggctcgtcat 3420
   agagagcagt cccctaagca gagctgagaa agcagcatat gtgaaatcat tatccaagtg 3480
   caagacagac caagaggtta aggcagtcat ggaactcgta gaagaggaca tagagtcact 3540
   gaccaactag atcccgggtg aggcatccca ccatcctcag tcacagagag acccaatcta 3600
   ccatcagcat cagccagtaa agattaagaa aaacttaggg tgaaagaaat ttcaccgcta 3660
   gcacctaggt ctgacatggc tgtcagcgac gcgctgctcc catctttctc cacgttcgcg 3720
   tctggcccgg cgggaaggga gaagacactg cgtcaagcag gtgccccgaa taaccgctgg 3780
   cgggaggagc tctcccacat gaagcgactt cccccagtgc ttcccggccg cccctatgac 3840
   ctggcggcgg cgaccgtggc cacagacctg gagagcggcg gagccggtgc ggcttgcggc 3900
   ggtagcaacc tggcgcccct acctcggaga gagaccgagg agttcaacga tctcctggac 3960
   ctggacttta ttctctccaa ttcgctgacc catcctccgg agtcagtggc cgccaccgtg 4020
   tcctcgtcag cgtcagcctc ctcttcgtcg tcgccgtcga gcagcggccc tgccagcgcg 4080
   ccctccacct gcagcttcac ctatccgatc cgggccggga acgacccggg cgtggcgccg 4140
   ggcggcacgg gcggaggcct cctctatggc agggagtccg ctccccctcc gacggctccc 4200
   ttcaacctgg cggacatcaa cgacgtgagc ccctcgggcg gcttcgtggc cgagctcctg 4260
   cggccagaat tggacccggt gtacattccg ccgcagcagc cgcagccgcc aggtggcggg 4320
   ctgatgggca agttcgtgct gaaggcgtcg ctgagcgccc ctggcagcga gtacggcagc 4380
   ccgtcggtca tcagcgtcag caaaggcagc cctgacggca gccacccggt ggtggtggcg 4440
   ccctacaacg gcgggccgcc gcgcacgtgc cccaagatca agcaggaggc ggtctcttcg 4500
   tgcacccact tgggcgctgg accccctctc agcaatggcc accggccggc tgcacacgac 4560
   ttccccctgg ggcggcagct ccccagcagg actaccccga ccctgggtct tgaggaagtg 4620
   ctgagcagca gggactgtca ccctgccctg ccgcttcctc ccggcttcca tccccacccg 4680
   gggcccaatt acccatcctt cctgcccgat cagatgcagc cgcaagtccc gccgctccat 4740
   taccaagagc tcatgccacc cggttcctgc atgccagagg agcccaagcc aaagagggga 4800
   agacgatcgt ggccccggaa aaggaccgcc acccacactt gtgattacgc gggctgcggc 4860
   aaaacctaca caaagagttc ccatctcaag gcacacctgc gaacccacac aggtgagaaa 4920
   ccttaccact gtgactggga cggctgtgga tggaaattcg cccgctcaga tgaactgacc 4980
   aggcactacc gtaaacacac ggggcaccgc ccgttccagt gccaaaaatg cgaccgagca 5040
   ttttccaggt cggaccacct cgccttacac atgaagaggc atttttaaag acgtcgatta 5100
   agaaaaactt agggtgaaag ttcatcgcgg ccgcttggcg ccagaatata tgaaaacatt 5160
   taacatttct caacaagatc tagaattagt agaagtagcg acagagaaga ttacaatgct 5220
   ttatgaggat aataaacatc atgtgggagc ggcaattcgt acgaaaacag gagaaatcat 5280
   ttcggcagta catattgaag cgtatatagg acgagtaact gtttgtgcag aagccattgc 5340
   cattggtagt gcagtttcga atggacaaaa ggattttgac acgattgtag ctgttagaca 5400
   cccttattct gacgaagtag atagaagtat tcgagtggta agtccttgtg gtatgtgtag 5460
   ggagttgatt tcagactatg caccagattg ttttgtgtta atagaaatga atggcaagtt 5520
   agtcaaaact acgattgaag aactcattcc actcaaatat acccgaaatt aaaacgcgtc 5580
   agagacctgc aacaatgtct caagcagaca ccacctggca gtcggagcca ccgggtcact 5640
   ccttgtctta aataagaaaa acttagggat aaagtccctt agatctagcc taggttcccc 5700
   atggcgggac acctggcttc ggatttcgcc ttctcgcccc ctccaggtgg tggaggtgat 5760
   gggccagggg ggccggagcc gggctgggtt gatcctcgga cctggctaag cttccaaggc 5820
   cctcctggag ggccaggaat cgggccgggg gttgggccag gctctgaggt gtgggggatt 5880
   cccccatgcc ccccgccgta tgagttctgt ggggggatgg cgtactgtgg gccccaggtt 5940
   ggagtggggc tagtgcccca aggcggcttg gagacctctc agcctgaggg cgaagcagga 6000
   gtcggggtgg agagcaactc cgatggggcc tccccggagc cctgcaccgt cacccctggt 6060
   gccgtgaagc tggagaagga gaagctggag caaaacccgg aggagtccca ggacatcaaa 6120
   gctctgcaga aagaactcga gcaatttgcc aagctcctga agcagaagag gatcaccctg 6180
   ggatatacac aggccgatgt ggggctcacc ctgggggttc tatttgggaa ggtattcagc 6240
   caaacgacca tctgccgctt tgaggctctg cagcttagct tcaagaacat gtgtaagctg 6300
   cggcccttgc tgcagaagtg ggtggaggaa gctgacaaca atgaaaatct tcaggagata 6360
   tgcaaagcag aaaccctcgt gcaggcccga aagagaaagc gaaccagtat cgagaaccga 6420
   gtgagaggca acctggagaa tttgttcctg cagtgcccga aacccacact gcagcagatc 6480
   agccacatcg cccagcagct tgggctcgag aaggatgtgg tccgagtgtg gttctgtaac 6540
   cggcgccaga agggcaagcg atcaagcagc gactatgcac aacgagagga ttttgaggct 6600
   gctgggtctc ctttctcagg gggaccagtg tcctttcctc tggccccagg gccccatttt 6660
   ggtaccccag gctatgggag ccctcacttc actgcactgt actcctcggt ccctttccct 6720
   gagggggaag cctttccccc tgtctccgtc accactctgg gctctcccat gcattcaaac 6780
   tgaggacgtc agatctgtat ataataagaa aaacttaggg tgaaagtgag gttgcgcggt 6840
   attttagcta gcccgcatgt acaacatgat ggagacggag ctgaagccgc cgggcccgca 6900
   gcaaacttcg gggggcggcg gcggcaactc caccgcggcg gcggccggcg gcaaccagaa 6960
   aaacagcccg gaccgcgtca agcggcccat gaatgccttc atggtgtggt cccgcgggca 7020
   gcggcgcaag atggcccagg agaaccccaa gatgcacaac tcggagatca gcaagcgcct 7080
   gggcgccgag tggaaacttt tgtcggagac ggagaagcgg ccgttcatcg acgaggctaa 7140
   gcggctgcga gcgctgcaca tgaaggagca cccggattat aaataccggc cccggcggaa 7200
   aaccaagacg ctcatgaaga aggataagta cacgctgccc ggcgggctgc tggcccccgg 7260
   cggcaatagc atggcgagcg gggtcggggt gggcgccggc ctgggcgcgg gcgtgaacca 7320
   gcgcatggac agttacgcgc acatgaacgg ctggagcaac ggcagctaca gcatgatgca 7380
   ggaccagctg ggctacccgc agcacccggg cctcaatgcg cacggcgcag cgcagatgca 7440
   gcccatgcac cgctacgacg tgagcgccct gcagtacaac tccatgacca gctcgcagac 7500
   ctacatgaac ggctcgccca cctacagcat gtcctactcg cagcagggca cccctggcat 7560
   ggctcttggc tccatgggtt cggtggtcaa gtccgaggcc agctccagcc cccctgtggt 7620
   tacctcttcc tcccactcca gggcgccctg ccaggccggg gacctccggg acatgatcag 7680
   catgtatctc cccggcgccg aggtgccgga acccgccgcc cccagcagac ttcacatgtc 7740
   ccagcactac cagagcggcc cggtgcccgg cacggccatt aacggcacac tgcccctctc 7800
   acacatgtga gaccggtgtc ggctttgctg acactagagt catctccgaa catccacaat 7860
   atctctcagt ctcttacgtc tctcacagta ttaagaaaaa cccagggtga atgggaagct 7920
   tgccataggt catggatggg caggagtcct cccaaaaccc ttctgacata ctctatccag 7980
   aatgccacct gaactctccc atagtcaggg ggaagatagc acagttgcac gtcttgttag 8040
   atgtgaacca gccctacaga ctaaaggacg acagcataat aaatattaca aagcacaaaa 8100
   ttaggaacgg aggattgtcc cctcgtcaaa ttaagatcag gtctctgggt aaggctcttc 8160
   aacgcacaat aaaggattta gaccgataca cctttgaacc gtacccaacc tactctcagg 8220
   aattacttag gcttgatata ccagagatat gtgacaaaat ccgatccgtc ttcgcggtct 8280
   cggatcggct gaccagggag ttatctagtg ggttccagga tctttggttg aatatcttca 8340
   agcaactagg caatatagaa ggaagagagg ggtacgatcc gttgcaggat atcggcacca 8400
   tcccggagat aactgataaa tacagcagga atagatggta taggccattc ctaacttggt 8460
   tcagcatcaa atatgacatg cggtggatgc agaagaccag accgggggga cccctcgata 8520
   cctctaattc acataacctc ctagaatgca aatcatacac tctagtaaca tacggagatc 8580
   ttatcatgat actgaacaag ttgacattga cagggtatat cctaacccct gagctggtct 8640
   tgatgtattg tgatgttgta gagggaaggt ggaatatgtc tgctgcaggg catctagata 8700
   agaagtccat tgggataaca agcaaaggtg aggaattatg ggaactagtg gattccctct 8760
   tctcaagtct tggagaggaa atatacaatg tcatcgcact attggagccc ctatcacttg 8820
   ctctcataca actaaatgat ccagttatac ctctacgtgg ggcatttatg aggcatgtgt 8880
   tgacagagct acaggctgtt ttaacaagta gggacgtgta cacagatgct gaagcagaca 8940
   ctattgtgga gtcgttactc gccattttcc atggaacctc tattgatgag aaagcagaga 9000
   tcttttcctt ctttaggaca tttggccacc ccagcttaga ggctgtcact gccgccgaca 9060
   aggtaagggc ccatatgtat gcacaaaagg caataaagct taagacccta tacgagtgtc 9120
   atgcagtttt ttgcactatc atcataaatg ggtatagaga gaggcatggc ggacagtggc 9180
   ccccctgtga cttccctgat cacgtgtgtc tagaactaag gaacgctcaa gggtccaata 9240
   cggcaatctc ttatgaatgt gctgtagaca actatacaag tttcataggc ttcaagtttc 9300
   ggaagtttat agaaccacaa ctagatgaag atctcacaat atatatgaaa gacaaagcac 9360
   tatcccccag gaaggaggca tgggactctg tatacccgga tagtaatctg tactataaag 9420
   ccccagaatc tgaagagacc cggcggctta ttgaagtgtt cataaatgat gagaatttca 9480
   acccagaaga aattatcaat tatgtggagt caggagattg gttgaaagac gagaagttca 9540
   acatctcgta cagtctcaaa gagaaagaga tcaagcaaga gggtcgtcta ttcgcaaaaa 9600
   tgacttataa gatgcgagcc gtacaggtgc tggcagagac actactggct aaaggaatag 9660
   gagagctgtt cagcgaaaat gggatggtta aaggagagat agacctactt aaaagattga 9720
   ctactctttc tgtctcagga gtccccagga ctgattcagt gtacaataac tctaaatcat 9780
   cagagaagag aaacgaaggc atgaaaaaga agaactctgg ggggtactgg gacgaaaaga 9840
   agaggtccag acatgaattc aaggcaacag attcatcaac agacggctat gaaacgttaa 9900
   gttgcttcct cacaacagac ctcaagaaat actgcttaaa ctggagattt gaaagtactg 9960
   cattgtttgg tcagagatgc aacgagatat ttggcttcaa gaccttcttt aactggatgc 10020
   atccagtcct tgaaaggtgt acaatatatg ttggggatcc ttactgtcca gtcgccgacc 10080
   ggatgcatcg acaactccag gatcatgcag actctggcat tttcatacat aatcctaggg 10140
   ggggcataga aggttactgc cagaagctgt ggaccttaat ctcaatcagt gcaatccacc 10200
   tagcagctgt gagagtgggt gtcagggtct ctgcaatggt tcagggtgac aatcaagcta 10260
   tagccgtgac atcaagagta cctgtagctc agacttacaa gcagaagaaa aatcatgtct 10320
   ataaggagat caccaaatat tttggtgctc taagacacgt catgtttgat gtagggcacg 10380
   agctaaaatt gaacgagacc atcattagta gcaagatgtt tgtctatagt aaaagaatat 10440
   actatgatgg gaagatttta ccacagtgcc tgaaagcctt gaccaggtgt gtattctggt 10500
   ccgagacact ggtagatgaa aacagatctg cttgttcgaa catctcaaca tccatagcaa 10560
   aagctatcga aaatgggtat tctcctatac taggctactg cattgcgttg tataagacct 10620
   gtcagcaggt gtgcatatca ctagggatga ctataaatcc aactatcagc ccgaccgtaa 10680
   gagatcaata ctttaagggt aagaattggc tgagatgtgc agtgttgatt ccagcaaatg 10740
   ttggaggatt caactacatg tctacatcta gatgctttgt tagaaatatt ggagaccccg 10800
   cagtagcagc cctagctgat ctcaaaagat tcatcagagc ggatctgtta gacaagcagg 10860
   tactatacag ggtcatgaat caagaacccg gtgactctag ctttctagat tgggcttcag 10920
   acccttattc atgtaacctc ccgcattctc agagtataac tacgattata aagaatatca 10980
   ctgctagatc tgtgctgcag gaatccccga atcctctact gtctggtctc ttcaccgaga 11040
   ctagtggaga agaggatctc aacctggcct cgttccttat ggaccggaaa gtcatcctgc 11100
   cgagagtggc tcatgagatc ctgggtaatt ccttaactgg agttagggag gcgattgcag 11160
   ggatgcttga tacgaccaag tctctagtga gatccagcgt taagaaagga ggattatcat 11220
   atgggatatt gaggaggctt gtcaattatg atctattgca gtacgagaca ctgactagaa 11280
   ctctcaggaa accggtgaaa gacaacatcg aatatgagta tatgtgttca gttgagctag 11340
   ctgtcggtct aaggcagaaa atgtggatcc acctaactta cgggagaccc atacatgggc 11400
   tagaaacacc agacccttta gagctcttga ggggaacatt tatcgaaggt tcagaggtgt 11460
   gcaagctttg caggtctgag ggagcagacc ccatctatac atggttctat ctccctgaca 11520
   atatagacct ggacacgctt acaaacggat gtccggctat aagaatcccc tattttggat 11580
   cagccactga tgaaaggtcg gaagcccaac tcgggtatgt aagaaatcta agcaaacccg 11640
   caaaggctgc catccggata gctatggtgt atacgtgggc ctacgggact gatgagatat 11700
   cgtggatgga agccgctctt atagcccaaa caagagctaa tctgagctta gagaatctaa 11760
   agctgctgac tcctgtttca acctccacta atctatctca taggttgaaa gatacggcaa 11820
   cccagatgaa gttctctagt gcaacactag tccgtgcaag tcggttcata acaatatcaa 11880
   atgataacat ggcactcaaa gaagcagggg agtcgaagga tactaatctc gtgtatcagc 11940
   agattatgct aactgggcta agcttgttcg agttcaatat gagatataag aaaggttcct 12000
   tagggaagcc actgatattg cacttacatc ttaataacgg gtgctgtata atggagtccc 12060
   cacaggaggc gaatatcccc ccaaggtcca cattagattt agagattaca caagagaaca 12120
   ataaattgat ctatgatcct gatccactca aggatgtgga ccttgagcta tttagcaagg 12180
   tcagagatgt tgtacataca gttgacatga cttattggtc agatgatgaa gttatcagag 12240
   caaccagtat ctgtactgca atgacgatag ctgatacaat gtctcaatta gatagagaca 12300
   acctaaaaga gatgatcgcg ctagtaaatg acgatgatgt caacagcctg attactgagt 12360
   ttatggtgat tgatgttcct ttattttgct caacgttcgg gggtattcta gtcaatcagt 12420
   ttgcatactc actctacggc ttaaacatca gaggaaggga agaaatatgg ggacatgtag 12480
   tccggattct taaagatacc tcccacgcag ttctaaaagt cttatctaat gctctatctc 12540
   atcccaaaat cttcaaacga ttctggaatg caggtgtcgt ggaacctgtg tatgggccta 12600
   acctctcaaa tcaggacaag atactcttgg ccctctctgt ctgtgaatat tctgtggatc 12660
   tattcatgca cgattggcaa gggggtgtac cgcttgagat ctttatctgt gacaatgacc 12720
   cagatgtggc cgacatgagg aggtcctctt tcttggcaag acatcttgca tacctatgca 12780
   gcgtggcaga gatatctagg gatgggccaa gattagaatc aatgaactct ctagagaggc 12840
   tcgagtcact aaagagttac ctggaactca catttcttga tgacccggta ctgaggtaca 12900
   gtcagttgac tggcctagtc atcaaagtat tcccatctac tttgacctat atccggaagt 12960
   catctataaa agtgttaagg acaagaggta taggagtccc tgaagtctta gaagattggg 13020
   atcccgaggc agataatgca ctgttagatg gtatcgcggc agaaatacaa cagaatattc 13080
   ctttgggaca tcagactaga gccccttttt gggggttgag agtatccaag tcacaggtac 13140
   tgcgtctccg ggggtacaag gagatcacaa gaggtgagat aggcagatca ggcgttggtc 13200
   tgacgttacc attcgatgga agatatctat ctcaccagct gaggctcttt ggcatcaaca 13260
   gtactagctg cttgaaagca cttgaactta cctacctatt gagcccctta gttgacaagg 13320
   ataaagatag gctatattta ggggaaggag ctggggccat gctttcctgt tatgacgcta 13380
   ctcttggccc atgcatcaac tattataact caggggtata ctcttgtgat gtcaatgggc 13440
   agagagagtt aaatatatat cctgctgagg tggcactggt gggaaagaaa ttaaacaatg 13500
   ttactagtct gggtcaaaga gttaaagtgt tattcaacgg gaatcctggc tcgacatgga 13560
   ttggaaatga tgagtgtgag gctttgattt ggaatgaatt gcagaatagc tcgataggcc 13620
   tagtccactg tgacatggag ggaggagatc ataaggatga tcaagttgta ctgcatgagc 13680
   attacagtgt aatccggatc gcgtatctgg tgggggatcg agacgttgtg cttataagca 13740
   agattgctcc taggctgggc acggattgga ccaggcagct cagcctatat ctgagatact 13800
   gggacgaggt taacctaata gtgcttaaaa catctaaccc tgcttccaca gagatgtatc 13860
   tcctatcgag gcatcccaaa tctgacatta tagaggacag caagacggtg ttagctagtc 13920
   tcctcccttt gtcaaaagaa gatagcatca agatagaaaa gtggatctta atagagaagg 13980
   caaaggctca cgaatgggtt actcgggaat tgagagaagg aagctcttca tcagggatgc 14040
   ttagacctta ccatcaagca ctgcagacgt ttggctttga accaaacttg tataaattga 14100
   gcagagattt cttgtccacc atgaacatag ctgatacaca caactgcatg atagctttca 14160
   acagggtttt gaaggataca atcttcgaat gggctagaat aactgagtca gataaaaggc 14220
   ttaaactaac tggtaagtat gacctgtatc ctgtgagaga ttcaggcaaa ttgaagacag 14280
   tttctagaag acttgtgcta tcttggatat ctttatctat gtccacaaga ttggtaactg 14340
   ggtcattccc tgaccagaag tttgaagcaa gacttcaatt gggaatagtt tcattatcat 14400
   cccgtgaaat caggaacctg agggttatca caaaaacttt attagaccgg tttgaggata 14460
   ttatacatag tataacgtac agattcctca ccaaagaaat aaagattttg atgaagattt 14520
   taggggcagt caagatgttc ggggccaggc aaaatgaata cacgaccgtg attgatgatg 14580
   gatcactggg tgatatcgag ccatatgaca gctcgtaata attagtccct atcgtgcaga 14640
   acgatcgaag ctccgcggta cctggaagtc ttggactgat ccatatgaca atagtaagaa 14700
   aaacttacaa gaagacaaga aaatttaaaa gaatacatat ctcttaaact cttgtctggt 14760
<210> 7
   <211> 38
   <212> DNA
   <213> Artificial
<220>
   <223> Primer
<400> 7
   actagctagc agtctgacat ggctgtcagc gacgcgct 38
<210> 8
   <211> 32
   <212> DNA
   <213> Artificial
<220>
   <223> Primer
<400> 8
   ggtccacgcg tttaaaaatg cctcttcatg tg 32
<210> 9
   <211> 1845
   <212> DNA
   <213> Artificial
<220>
   <223> pMO026
<400> 9
   gaattcgagt catcccgaga cgcgagttat gtgtttgcaa gacgtgccct aaagtctgca 60
   aactatgcag agatgacatt caatgtatgc ggcctgatcc tttctgccga gaaatcttcc 120
   gctcgtaagg tagatgagaa caaacaactg ctcaaacaga tccaagagag cgtggaatca 180
   ttccgggata tttacaagag attctctgag tatcagaaag aacagaactc attgctgatg 240
   tccaacctat ctacacttca tatcatcaca gatagaggtg gcaagactga caacacagac 300
   tcccttacaa ggtccccctc cgtttttgca aaatcaaaag agaacaagac taaggctacc 360
   aggtttgacc catctatgga gaccttagaa gatatgaagt acaaaccgga cctaatccga 420
   gaggatgaat ttagagatga gatccgcaac ccggtgtacc aagagaggga cacagaaccc 480
   agggcctcaa acgcatcacg tctcttcccc tccaaagaga agcccacaat gcactctctc 540
   aggctcgtca tagagagcag tcccctaagc agagctgaga aagcagcata tgtgaaatca 600
   ttatccaagt gcaagacaga ccaagaggtt aaggcagtca tggaactcgt agaagaggac 660
   atagagtcac tgaccaacta gatcccgggt gaggcatccc accatcctca gtcacagaga 720
   gacccaatct accatcagca tcagccagta aagattaaga aaaacttagg gtgaaagaaa 780
   tgcggccgct tgctagcaga atatatgaaa acatttaaca tttctcaaca agatctagaa 840
   ttagtagaag tagcgacaga gaagattaca atgctttatg aggataataa acatcatgtg 900
   ggagcggcaa ttcgtacgaa aacaggagaa atcatttcgg cagtacatat tgaagcgtat 960
   ataggacgag taactgtttg tgcagaagcc attgccattg gtagtgcagt ttcgaatgga 1020
   caaaaggatt ttgacacgat tgtagctgtt agacaccctt attctgacga agtagataga 1080
   agtattcgag tggtaagtcc ttgtggtatg tgtagggagt tgatttcaga ctatgcacca 1140
   gattgttttg tgttaataga aatgaatggc aagttagtca aaactacgat tgaagaactc 1200
   attccactca aatatacccg aaattaaaac gcgtcagaga cctgcaacaa tgtctcaagc 1260
   agacaccacc tggcagtcgg agccaccggg tcactccttg tcttaaataa gaaaaactta 1320
   gggataaagt cccttgtgag tgcttggttg caaaactctc cccttgggaa acatgacagc 1380
   atatatccgg aggtcacagt gcatctcaac atcactactg gttgttctca ccacattggt 1440
   ctcgtgtcag attcccaggg atatgctctc taacataggg gtcatagtcg atgaagggaa 1500
   atcactgaag atagctgggt cccacgaatc gaggtacata gtactgagtc tagttccggg 1560
   ggtagacctt gagaatggat gcggaacagc tcaggttatc cagtacaaga gcctactgaa 1620
   caggctgtta atcccattga gggatgcctt agatcttcag gaggctctga taactgtcac 1680
   caatgatacg acacaaaatg ccggtgttcc acagttgaga ttcttcggtg ctgtgattgg 1740
   tactatcgca cttggagtgg cgacatcagc acagatcacc acagggattg cactagccga 1800
   agcgagggag gccaaaagag acatagcgct catcaaggaa tcgat 1845
<210> 10
   <211> 39
   <212> DNA
   <213> Artificial
<220>
   <223> Primer
<400> 10
   actagctagc ttagacgctg gatttttttc gggtagtgg 39
<210> 11
   <211> 30
   <212> DNA
   <213> Artificial
<220>
   <223> Primer
<400> 11
   gtccgacgtc cttacgcaca agagttccgt 30
<210> 12
   <211> 5160
   <212> DNA
   <213> Artificial
<220>
   <223> pM0094(+E)Mp;Klf4 Xhol-
<400> 12
   ctcgagtaat acgactcact atagggacca aacaagagaa gaaacatgta tggaatatat      60
   aatgaagttt aagaaaaact tagggtcaaa gtatccaccc tgaggagcag gttccagatc     120
   cttttctttg ctgccaaagt tcacgatggc cgggttgttg agcaccttcg atacatttag     180
   ctctaggagg agcgaaagta ttaataagtc gggaggaggt gctgttatcc ccggccagag 240
   gagcacagtc tcagtgttca tactaggccc aagtgtgact gatgatgcag acaagttatt 300
   cattgcaaca accttcctag ctcactcatt ggacacagat aagcagcact ctcagagagg 360
   agggttcctc gtctctctgc ttgccatggc ttacagtagt ccagaattgt acttgacaac 420
   aaacggagta aacgccgatg tcaaatatgt gatctacaac atagagaaag accctaagag 480
   gacgaagaca gacggattca ttgtgaagac gagagatatg gaatatgaga ggaccacaga 540
   atggctgttt ggacctatgg tcaacaagag cccactcttc cagggtcaac gggatgctgc 600
   agaccctgac acactccttc aaatctatgg gtatcctgca tgcctaggag caataattgt 660
   ccaagtctgg attgtgctgg tgaaggccat cacaagcagc gccggcttaa ggaaagggtt 720
   cttcaacagg ttagaggcgt tcagacaaga cggcaccgtg aaaggtgcct tagttttcac 780
   tggggagaca gttgagggga taggctcggt tatgagatct cagcaaagcc ttgtatctct 840
   catggttgag acccttgtga ctatgaatac tgcaagatct gatctcacca cattagagaa 900
   gaacatccag atcgttggga actacatccg agatgcaggg ctggcttcct tcatgaacac 960
   tattaaatat ggggtggaga caaagatggc agctctaacg ttgtcaaacc tgaggcccga 1020
   tattaataag cttagaagcc tcatagacac ctacctgtca aaaggcccca gagctcccct 1080
   tatctgtatc ctcaaggacc ctgttcatgg tgaatttgct ccaggcaatt atcctgcact 1140
   atggagttac gccatgggag tcgccgtcgt acagaacaag tcaatgcagc agtacgtcac 1200
   agggaggaca taccttgata tggaaatgtt cttactagga caagccgtgg caaaggatgc 1260
   tgaatcgaag atcagcagtg ccttggaaga tgagttagga gtgacggata cagccaagga 1320
   gaggctcaga catcatctgg caaacttgtc cggtggggat ggtgcttacc acaaaccaac 1380
   aggcggtggt gcaattgagg tagctctaga caatgccgat atcgacctgg aaacagaagc 1440
   tcatgcggac caggacgcta ggggttgggg tggagatagt ggtgaaagat gggcacgtca 1500
   ggtgagtggt ggccactttg tcacactaca tggggctgaa cggttagagg aggaaaccaa 1560
   tgatgaggat gtatcagaca tagagagaag aatagccatg agactcgcag agagacggca 1620
   agaggattct gcaacccatg gagatgaagg ccgcaataac ggtgttgatc acgaagaaga 1680
   tgacgatgcc gcagcagcag ctgggatagg aggaatctag gatcatacga ggcctcaagg 1740
   tacttgatcc gcagtaagaa aaacttaggg tgaaagttca tccaccgatc ggctcaggca 1800
   aggccacacc caaccccacc gaccacaccc agcagtcgag acagccacgg cttcggctac 1860
   acttaccgca tggatcaaga tgccttcatt cttaaagaag attctgaagt tgagaggaag 1920
   gcgccaggag gacgagagtc gctctcggat gttatcggat tcctcgatgc tgtcctgtcg 1980
   aatgaaccaa ctgacatcgg aggggacaga agctggctcc acaacaccat caacactccc 2040
   caaggaccag gctctgctca tagagccaaa agtgagggcg aaggagaagt ctcaacaccg 2100
   tcgacccaag ataatcgatc aggtgaggag agtagagtct ctgggagaac aagcaagcca 2160
   gaggcagaag cacatgctgg aaaccttgat aaacaaaata tacactgggc ctttagggga 2220
   agaactggta caaactctgt atctcaggat ctggacgatg gaggagactc cggaatcctt 2280
   gaaaatcctc caaatgagag aggatatccg agatcaggta ttgaagatga aaacagagag 2340
   atggctgcgc accctgataa gaggggagaa gaccaagctg aaggacttcc agaagaggta 2400
   cgaggaggta catccctacc tgatgaagga gaaggtggag caagtaataa tggaagaagc 2460
   atggagcctg gcagctcaca tagtgcaaga gtaactgggg tcctggtgat tcctagcccc 2520
   gaacttgaag aggctgtgct acggaggaac aaaagaagac ctaccaacag tgggtccaaa 2580
   cctcttactc cagcaaccgt gcctggcacc cggtccccac cgctgaatcg ttacaacagc 2640
   acagggtcac caccaggaaa acccccatct acacaggatg agcacatcaa ctctggggac 2700
   acccccgccg tcagggtcaa agaccggaaa ccatcaatag ggactcgctc tgtctcagat 2760
   tgtccagcca acggccgccc aatccatccg ggtatagaga ccgactcaac aaaaaagggc 2820
   ataggagaga acacatcatc tatgaaagat atggctacat tgttgacgag tcttggtgta 2880
   atccagtctg ctcaagaatt cgagtcatcc cgagacgcga gttatgtgtt tgcaagacgt 2940
   gccctaaagt ctgcaaacta tgcagagatg acattcaatg tatgcggcct gatcctttct 3000
   gccgagaaat cttccgctcg taaggtagat gagaacaaac aactgctcaa acagatccaa 3060
   gagagcgtgg aatcattccg ggatatttac aagagattct ctgagtatca gaaagaacag 3120
   aactcattgc tgatgtccaa cctatctaca cttcatatca tcacagatag aggtggcaag 3180
   actgacaaca cagactccct tacaaggtcc ccctccgttt ttgcaaaatc aaaagagaac 3240
   aagactaagg ctaccaggtt tgacccatct atggagacct tagaagatat gaagtacaaa 3300
   ccggacctaa tccgagagga tgaatttaga gatgagatcc gcaacccggt gtaccaagag 3360
   agggacacag aacccagggc ctcaaacgca tcacgtctct tcccctccaa agagaagccc 3420
   acaatgcact ctctcaggct cgtcatagag agcagtcccc taagcagagc tgagaaagca 3480
   gcatatgtga aatcattatc caagtgcaag acagaccaag aggttaaggc agtcatggaa 3540
   ctcgtagaag aggacataga gtcactgacc aactagatcc cgggtgaggc atcccaccat 3600
   cctcagtcac agagagaccc aatctaccat cagcatcagc cagtaaagat taagaaaaac 3660
   ttagggtgaa agaaatttca ccgctagcac ctaggtctga catggctgtc agcgacgcgc 3720
   tgctcccatc tttctccacg ttcgcgtctg gcccggcggg aagggagaag acactgcgtc 3780
   aagcaggtgc cccgaataac cgctggcggg aggagctctc ccacatgaag cgacttcccc 3840
   cagtgcttcc cggccgcccc tatgacctgg cggcggcgac cgtggccaca gacctggaga 3900
   gcggcggagc cggtgcggct tgcggcggta gcaacctggc gcccctacct cggagagaga 3960
   ccgaggagtt caacgatctc ctggacctgg actttattct ctccaattcg ctgacccatc 4020
   ctccggagtc agtggccgcc accgtgtcct cgtcagcgtc agcctcctct tcgtcgtcgc 4080
   cgtcgagcag cggccctgcc agcgcgccct ccacctgcag cttcacctat ccgatccggg 4140
   ccgggaacga cccgggcgtg gcgccgggcg gcacgggcgg aggcctcctc tatggcaggg 4200
   agtccgctcc ccctccgacg gctcccttca acctggcgga catcaacgac gtgagcccct 4260
   cgggcggctt cgtggccgag ctcctgcggc cagaattgga cccggtgtac attccgccgc 4320
   agcagccgca gccgccaggt ggcgggctga tgggcaagtt cgtgctgaag gcgtcgctga 4380
   gcgcccctgg cagcgagtac ggcagcccgt cggtcatcag cgtcagcaaa ggcagccctg 4440
   acggcagcca cccggtggtg gtggcgccct acaacggcgg gccgccgcgc acgtgcccca 4500
   agatcaagca ggaggcggtc tcttcgtgca cccacttggg cgctggaccc cctctcagca 4560
   atggccaccg gccggctgca cacgacttcc ccctggggcg gcagctcccc agcaggacta 4620
   ccccgaccct gggtcttgag gaagtgctga gcagcaggga ctgtcaccct gccctgccgc 4680
   ttcctcccgg cttccatccc cacccggggc ccaattaccc atccttcctg cccgatcaga 4740
   tgcagccgca agtcccgccg ctccattacc aagagctcat gccacccggt tcctgcatgc 4800
   cagaggagcc caagccaaag aggggaagac gatcgtggcc ccggaaaagg accgccaccc 4860
   acacttgtga ttacgcgggc tgcggcaaaa cctacacaaa gagttcccat ctcaaggcac 4920
   acctgcgaac ccacacaggt gagaaacctt accactgtga ctgggacggc tgtggatgga 4980
   aattcgcccg ctcagatgaa ctgaccaggc actaccgtaa acacacgggg caccgcccgt 5040
   tccagtgcca aaaatgcgac cgagcatttt ccaggtcgga ccacctcgcc ttacacatga 5100
   agaggcattt ttaaagacgt cgattaagaa aaacttaggg tgaaagttca tcgcggccgc 5160
<210> 13
   <211> 15696
   <212> DNA
   <213> Artificial
<220>
   <223> Template cDNA for SeVp(Mp+myc,delta-M::Klf4,delta-F::Oct4,delta-H N::Sox2)
<400> 13
   accaaacaag agaagaaaca tgtatggaat atataatgaa gtttaagaaa aacttagggt     60
   caaagtatcc accctgagga gcaggttcca gatccttttc tttgctgcca aagttcacga    120
   tggccgggtt gttgagcacc ttcgatacat ttagctctag gaggagcgaa agtattaata    180
   agtcgggagg aggtgctgtt atccccggcc agaggagcac agtctcagtg ttcatactag    240
   gcccaagtgt gactgatgat gcagacaagt tattcattgc aacaaccttc ctagctcact    300
   cattggacac agataagcag cactctcaga gaggagggtt cctcgtctct ctgcttgcca    360
   tggcttacag tagtccagaa ttgtacttga caacaaacgg agtaaacgcc gatgtcaaat    420
   atgtgatcta caacatagag aaagacccta agaggacgaa gacagacgga ttcattgtga    480
   agacgagaga tatggaatat gagaggacca cagaatggct gtttggacct atggtcaaca    540
   agagcccact cttccagggt caacgggatg ctgcagaccc tgacacactc cttcaaatct 600
   atgggtatcc tgcatgccta ggagcaataa ttgtccaagt ctggattgtg ctggtgaagg 660
   ccatcacaag cagcgccggc ttaaggaaag ggttcttcaa caggttagag gcgttcagac 720
   aagacggcac cgtgaaaggt gccttagttt tcactgggga gacagttgag gggataggct 780
   cggttatgag atctcagcaa agccttgtat ctctcatggt tgagaccctt gtgactatga 840
   atactgcaag atctgatctc accacattag agaagaacat ccagatcgtt gggaactaca 900
   tccgagatgc agggctggct tccttcatga acactattaa atatggggtg gagacaaaga 960
   tggcagctct aacgttgtca aacctgaggc ccgatattaa taagcttaga agcctcatag 1020
   acacctacct gtcaaaaggc cccagagctc cctttatctg tatcctcaag gaccctgttc 1080
   atggtgaatt tgctccaggc aattatcctg cactatggag ttacgccatg ggagtcgccg 1140
   tcgtacagaa caagtcaatg cagcagtacg tcacagggag gacatacctt gatatggaaa 1200
   tgttcttact aggacaagcc gtggcaaagg atgctgaatc gaagatcagc agtgccttgg 1260
   aagatgagtt aggagtgacg gatacagcca aggagaggct cagacatcat ctggcaaact 1320
   tgtccggtgg ggatggtgct taccacaaac caacaggcgg tggtgcaatt gaggtagctc 1380
   tagacaatgc cgatatcgac ctggaaacag aagctcatgc ggaccaggac gctaggggtt 1440
   ggggtggaga tagtggtgaa agatgggcac gtcaggtgag tggtggccac tttgtcacac 1500
   tacatggggc tgaacggtta gaggaggaaa ccaatgatga ggatgtatca gacatagaga 1560
   gaagaatagc catgagactc gcagagagac ggcaagagga ttctgcaacc catggagatg 1620
   aaggccgcaa taacggtgtt gatcacgaag aagatgacga tgccgcagca gcagctggga 1680
   taggaggaat ctaggatcat acgaggcctc aaggtacttg atccgcagta agaaaaactt 1740
   agggtgaaag ttcatccacc gatcggctca ggcaaggcca cacccaaccc caccgaccac 1800
   acccagcagt cgagacagcc acggcttcgg ctacacttac cgcatggatc aagatgcctt 1860
   cattcttaaa gaagattctg aagttgagag gaaggcgcca ggaggacgag agtcgctctc 1920
   ggatgttatc ggattcctcg atgctgtcct gtcgaatgaa ccaactgaca tcggagggga 1980
   cagaagctgg ctccacaaca ccatcaacac tccccaagga ccaggctctg ctcatagagc 2040
   caaaagtgag ggcgaaggag aagtctcaac accgtcgacc caagataatc gatcaggtga 2100
   ggagagtaga gtctctggga gaacaagcaa gccagaggca gaagcacatg ctggaaacct 2160
   tgataaacaa aatatacact gggcctttag gggaagaact ggtacaaact ctgtatctca 2220
   ggatctggac gatggaggag actccggaat ccttgaaaat cctccaaatg agagaggata 2280
   tccgagatca ggtattgaag atgaaaacag agagatggct gcgcaccctg ataagagggg 2340
   agaagaccaa gctgaaggac ttccagaaga ggtacgagga ggtacatccc tacctgatga 2400
   aggagaaggt ggagcaagta ataatggaag aagcatggag cctggcagct cacatagtgc 2460
   aagagtaact ggggtcctgg tgattcctag ccccgaactt gaagaggctg tgctacggag 2520
   gaacaaaaga agacctacca acagtgggtc caaacctctt actccagcaa ccgtgcctgg 2580
   cacccggtcc ccaccgctga atcgttacaa cagcacaggg tcaccaccag gaaaaccccc 2640
   atctacacag gatgagcaca tcaactctgg ggacaccccc gccgtcaggg tcaaagaccg 2700
   gaaaccatca atagggactc gctctgtctc agattgtcca gccaacggcc gcccaatcca 2760
   tccgggtata gagaccgact caacaaaaaa gggcatagga gagaacacat catctatgaa 2820
   agatatggct acattgttga cgagtcttgg tgtaatccag tctgctcaag aattcgagtc 2880
   atcccgagac gcgagttatg tgtttgcaag acgtgcccta aagtctgcaa actatgcaga 2940
   gatgacattc aatgtatgcg gcctgatcct ttctgccgag aaatcttccg ctcgtaaggt 3000
   agatgagaac aaacaactgc tcaaacagat ccaagagagc gtggaatcat tccgggatat 3060
   ttacaagaga ttctctgagt atcagaaaga acagaactca ttgctgatgt ccaacctatc 3120
   tacacttcat atcatcacag atagaggtgg caagactgac aacacagact cccttacaag 3180
   gtccccctcc gtttttgcaa aatcaaaaga gaacaagact aaggctacca ggtttgaccc 3240
   atctatggag accttagaag atatgaagta caaaccggac ctaatccgag aggatgaatt 3300
   tagagatgag atccgcaacc cggtgtacca agagagggac acagaaccca gggcctcaaa 3360
   cgcatcacgt ctcttcccct ccaaagagaa gcccacaatg cactctctca ggctcgtcat 3420
   agagagcagt cccctaagca gagctgagaa agcagcatat gtgaaatcat tatccaagtg 3480
   caagacagac caagaggtta aggcagtcat ggaactcgta gaagaggaca tagagtcact 3540
   gaccaactag atcccgggtg aggcatccca ccatcctcag tcacagagag acccaatcta 3600
   ccatcagcat cagccagtaa agattaagaa aaacttaggg tgaaagaaat ttcaccgcta 3660
   gcttagacgc tggatttttt tcgggtagtg gaaaaccagc agcctcccgc gacgatgccc 3720
   ctcaacgtta gcttcaccaa caggaactat gacctcgact acgactcggt gcagccgtat 3780
   ttctactgcg acgaggagga gaacttctac cagcagcagc agcagagcga gctgcagccc 3840
   ccggcgccca gcgaggatat ctggaagaaa ttcgagctgc tgcccacccc gcccctgtcc 3900
   cctagccgcc gctccgggct ctgctcgccc tcctacgttg cggtcacacc cttctccctt 3960
   cggggagaca acgacggcgg tggcgggagc ttctccacgg ccgaccagct ggagatggtg 4020
   accgagctgc tgggaggaga catggtgaac cagagtttca tctgcgaccc ggacgacgag 4080
   accttcatca aaaacatcat catccaggac tgtatgtgga gcggcttctc ggccgccgcc 4140
   aagctcgtct cagagaagct ggcctcctac caggctgcgc gcaaagacag cggcagcccg 4200
   aaccccgccc gcggccacag cgtctgctcc acctccagct tgtacctgca ggatctgagc 4260
   gccgccgcct cagagtgcat cgacccctcg gtggtcttcc cctaccctct caacgacagc 4320
   agctcgccca agtcctgcgc ctcgcaagac tccagcgcct tctctccgtc ctcggattct 4380
   ctgctctcct cgacggagtc ctccccgcag ggcagccccg agcccctggt gctccatgag 4440
   gagacaccgc ccaccaccag cagcgactct gaggaggaac aagaagatga ggaagaaatc 4500
   gatgttgttt ctgtggaaaa gaggcaggct cctggcaaaa ggtcagagtc tggatcacct 4560
   tctgctggag gccacagcaa acctcctcac agcccactgg tcctcaagag gtgccacgtc 4620
   tccacacatc agcacaacta cgcagcgcct ccctccactc ggaaggacta tcctgctgcc 4680
   aagagggtca agttggacag tgtcagagtc ctgagacaga tcagcaacaa ccgaaaatgc 4740
   accagcccca ggtcctcgga caccgaggag aatgtcaaga ggcgaacaca caacgtcttg 4800
   gagcgccaga ggaggaacga gctaaaacgg agcttttttg ccctgcgtga ccagatcccg 4860
   gagttggaaa acaatgaaaa ggcccccaag gtagttatcc ttaaaaaagc cacagcatac 4920
   atcctgtccg tccaagcaga ggagcaaaag ctcatttctg aagaggactt gttgcggaaa 4980
   cgacgagaac agttgaaaca caaacttgaa cagctacgga actcttgtgc gtaaggacgt 5040
   cgattaagaa aaacttaggg tgaaagttca tcgcggccgc ttgctagcag tctgacatgg 5100
   ctgtcagcga cgcgctgctc ccatctttct ccacgttcgc gtctggcccg gcgggaaggg 5160
   agaagacact gcgtcaagca ggtgccccga ataaccgctg gcgggaggag ctctcccaca 5220
   tgaagcgact tcccccagtg cttcccggcc gcccctatga cctggcggcg gcgaccgtgg 5280
   ccacagacct ggagagcggc ggagccggtg cggcttgcgg cggtagcaac ctggcgcccc 5340
   tacctcggag agagaccgag gagttcaacg atctcctgga cctggacttt attctctcca 5400
   attcgctgac ccatcctccg gagtcagtgg ccgccaccgt gtcctcgtca gcgtcagcct 5460
   cctcttcgtc gtcgccgtcg agcagcggcc ctgccagcgc gccctccacc tgcagcttca 5520
   cctatccgat ccgggccggg aacgacccgg gcgtggcgcc gggcggcacg ggcggaggcc 5580
   tcctctatgg cagggagtcc gctccccctc cgacggctcc cttcaacctg gcggacatca 5640
   acgacgtgag cccctcgggc ggcttcgtgg ccgagctcct gcggccagaa ttggacccgg 5700
   tgtacattcc gccgcagcag ccgcagccgc caggtggcgg gctgatgggc aagttcgtgc 5760
   tgaaggcgtc gctgagcgcc cctggcagcg agtacggcag cccgtcggtc atcagcgtca 5820
   gcaaaggcag ccctgacggc agccacccgg tggtggtggc gccctacaac ggcgggccgc 5880
   cgcgcacgtg ccccaagatc aagcaggagg cggtctcttc gtgcacccac ttgggcgctg 5940
   gaccccctct cagcaatggc caccggccgg ctgcacacga cttccccctg gggcggcagc 6000
   tccccagcag gactaccccg accctgggtc ttgaggaagt gctgagcagc agggactgtc 6060
   accctgccct gccgcttcct cccggcttcc atccccaccc ggggcccaat tacccatcct 6120
   tcctgcccga tcagatgcag ccgcaagtcc cgccgctcca ttaccaagag ctcatgccac 6180
   ccggttcctg catgccagag gagcccaagc caaagagggg aagacgatcg tggccccgga 6240
   aaaggaccgc cacccacact tgtgattacg cgggctgcgg caaaacctac acaaagagtt 6300
   cccatctcaa ggcacacctg cgaacccaca caggtgagaa accttaccac tgtgactggg 6360
   acggctgtgg atggaaattc gcccgctcag atgaactgac caggcactac cgtaaacaca 6420
   cggggcaccg cccgttccag tgccaaaaat gcgaccgagc attttccagg tcggaccacc 6480
   tcgccttaca catgaagagg catttttaaa cgcgtcagag acctgcaaca atgtctcaag 6540
   cagacaccac ctggcagtcg gagccaccgg gtcactcctt gtcttaaata agaaaaactt 6600
   agggataaag tcccttagat ctagcctagg ttccccatgg cgggacacct ggcttcggat 6660
   ttcgccttct cgccccctcc aggtggtgga ggtgatgggc caggggggcc ggagccgggc 6720
   tgggttgatc ctcggacctg gctaagcttc caaggccctc ctggagggcc aggaatcggg 6780
   ccgggggttg ggccaggctc tgaggtgtgg gggattcccc catgcccccc gccgtatgag 6840
   ttctgtgggg ggatggcgta ctgtgggccc caggttggag tggggctagt gccccaaggc 6900
   ggcttggaga cctctcagcc tgagggcgaa gcaggagtcg gggtggagag caactccgat 6960
   ggggcctccc cggagccctg caccgtcacc cctggtgccg tgaagctgga gaaggagaag 7020
   ctggagcaaa acccggagga gtcccaggac atcaaagctc tgcagaaaga actcgagcaa 7080
   tttgccaagc tcctgaagca gaagaggatc accctgggat atacacaggc cgatgtgggg 7140
   ctcaccctgg gggttctatt tgggaaggta ttcagccaaa cgaccatctg ccgctttgag 7200
   gctctgcagc ttagcttcaa gaacatgtgt aagctgcggc ccttgctgca gaagtgggtg 7260
   gaggaagctg acaacaatga aaatcttcag gagatatgca aagcagaaac cctcgtgcag 7320
   gcccgaaaga gaaagcgaac cagtatcgag aaccgagtga gaggcaacct ggagaatttg 7380
   ttcctgcagt gcccgaaacc cacactgcag cagatcagcc acatcgccca gcagcttggg 7440
   ctcgagaagg atgtggtccg agtgtggttc tgtaaccggc gccagaaggg caagcgatca 7500
   agcagcgact atgcacaacg agaggatttt gaggctgctg ggtctccttt ctcaggggga 7560
   ccagtgtcct ttcctctggc cccagggccc cattttggta ccccaggcta tgggagccct 7620
   cacttcactg cactgtactc ctcggtccct ttccctgagg gggaagcctt tccccctgtc 7680
   tccgtcacca ctctgggctc tcccatgcat tcaaactgag gacgtcagat ctgtatataa 7740
   taagaaaaac ttagggtgaa agtgaggttg cgcggtattt tagctagccc gcatgtacaa 7800
   catgatggag acggagctga agccgccggg cccgcagcaa acttcggggg gcggcggcgg 7860
   caactccacc gcggcggcgg ccggcggcaa ccagaaaaac agcccggacc gcgtcaagcg 7920
   gcccatgaat gccttcatgg tgtggtcccg cgggcagcgg cgcaagatgg cccaggagaa 7980
   ccccaagatg cacaactcgg agatcagcaa gcgcctgggc gccgagtgga aacttttgtc 8040
   ggagacggag aagcggccgt tcatcgacga ggctaagcgg ctgcgagcgc tgcacatgaa 8100
   ggagcacccg gattataaat accggccccg gcggaaaacc aagacgctca tgaagaagga 8160
   taagtacacg ctgcccggcg ggctgctggc ccccggcggc aatagcatgg cgagcggggt 8220
   cggggtgggc gccggcctgg gcgcgggcgt gaaccagcgc atggacagtt acgcgcacat 8280
   gaacggctgg agcaacggca gctacagcat gatgcaggac cagctgggct acccgcagca 8340
   cccgggcctc aatgcgcacg gcgcagcgca gatgcagccc atgcaccgct acgacgtgag 8400
   cgccctgcag tacaactcca tgaccagctc gcagacctac atgaacggct cgcccaccta 8460
   cagcatgtcc tactcgcagc agggcacccc tggcatggct cttggctcca tgggttcggt 8520
   ggtcaagtcc gaggccagct ccagcccccc tgtggttacc tcttcctccc actccagggc 8580
   gccctgccag gccggggacc tccgggacat gatcagcatg tatctccccg gcgccgaggt 8640
   gccggaaccc gccgccccca gcagacttca catgtcccag cactaccaga gcggcccggt 8700
   gcccggcacg gccattaacg gcacactgcc cctctcacac atgtgagacc ggtgtcggct 8760
   ttgctgacac tagagtcatc tccgaacatc cacaatatct ctcagtctct tacgtctctc 8820
   acagtattaa gaaaaaccca gggtgaatgg gaagcttgcc ataggtcatg gatgggcagg 8880
   agtcctccca aaacccttct gacatactct atccagaatg ccacctgaac tctcccatag 8940
   tcagggggaa gatagcacag ttgcacgtct tgttagatgt gaaccagccc tacagactaa 9000
   aggacgacag cataataaat attacaaagc acaaaattag gaacggagga ttgtcccctc 9060
   gtcaaattaa gatcaggtct ctgggtaagg ctcttcaacg cacaataaag gatttagacc 9120
   gatacacctt tgaaccgtac ccaacctact ctcaggaatt acttaggctt gatataccag 9180
   agatatgtga caaaatccga tccgtcttcg cggtctcgga tcggctgacc agggagttat 9240
   ctagtgggtt ccaggatctt tggttgaata tcttcaagca actaggcaat atagaaggaa 9300
   gagaggggta cgatccgttg caggatatcg gcaccatccc ggagataact gataaataca 9360
   gcaggaatag atggtatagg ccattcctaa cttggttcag catcaaatat gacatgcggt 9420
   ggatgcagaa gaccagaccg gggggacccc tcgatacctc taattcacat aacctcctag 9480
   aatgcaaatc atacactcta gtaacatacg gagatcttat catgatactg aacaagttga 9540
   cattgacagg gtatatccta acccctgagc tggtcttgat gtattgtgat gttgtagagg 9600
   gaaggtggaa tatgtctgct gcagggcatc tagataagaa gtccattggg ataacaagca 9660
   aaggtgagga attatgggaa ctagtggatt ccctcttctc aagtcttgga gaggaaatat 9720
   acaatgtcat cgcactattg gagcccctat cacttgctct catacaacta aatgatccag 9780
   ttatacctct acgtggggca tttatgaggc atgtgttgac agagctacag gctgttttaa 9840
   caagtaggga cgtgtacaca gatgctgaag cagacactat tgtggagtcg ttactcgcca 9900
   ttttccatgg aacctctatt gatgagaaag cagagatctt ttccttcttt aggacatttg 9960
   gccaccccag cttagaggct gtcactgccg ccgacaaggt aagggcccat atgtatgcac 10020
   aaaaggcaat aaagcttaag accctatacg agtgtcatgc agttttttgc actatcatca 10080
   taaatgggta tagagagagg catggcggac agtggccccc ctgtgacttc cctgatcacg 10140
   tgtgtctaga actaaggaac gctcaagggt ccaatacggc aatctcttat gaatgtgctg 10200
   tagacaacta tacaagtttc ataggcttca agtttcggaa gtttatagaa ccacaactag 10260
   atgaagatct cacaatatat atgaaagaca aagcactatc ccccaggaag gaggcatggg 10320
   actctgtata cccggatagt aatctgtact ataaagcccc agaatctgaa gagacccggc 10380
   ggcttattga agtgttcata aatgatgaga atttcaaccc agaagaaatt atcaattatg 10440
   tggagtcagg agattggttg aaagacgaga agttcaacat ctcgtacagt ctcaaagaga 10500
   aagagatcaa gcaagagggt cgtctattcg caaaaatgac ttataagatg cgagccgtac 10560
   aggtgctggc agagacacta ctggctaaag gaataggaga gctgttcagc gaaaatggga 10620
   tggttaaagg agagatagac ctacttaaaa gattgactac tctttctgtc tcaggagtcc 10680
   ccaggactga ttcagtgtac aataactcta aatcatcaga gaagagaaac gaaggcatga 10740
   aaaagaagaa ctctgggggg tactgggacg aaaagaagag gtccagacat gaattcaagg 10800
   caacagattc atcaacagac ggctatgaaa cgttaagttg cttcctcaca acagacctca 10860
   agaaatactg cttaaactgg agatttgaaa gtactgcatt gtttggtcag agatgcaacg 10920
   agatatttgg cttcaagacc ttctttaact ggatgcatcc agtccttgaa aggtgtacaa 10980
   tatatgttgg ggatccttac tgtccagtcg ccgaccggat gcatcgacaa ctccaggatc 11040
   atgcagactc tggcattttc atacataatc ctaggggggg catagaaggt tactgccaga 11100
   agctgtggac cttaatctca atcagtgcaa tccacctagc agctgtgaga gtgggtgtca 11160
   gggtctctgc aatggttcag ggtgacaatc aagctatagc cgtgacatca agagtacctg 11220
   tagctcagac ttacaagcag aagaaaaatc atgtctataa ggagatcacc aaatattttg 11280
   gtgctctaag acacgtcatg tttgatgtag ggcacgagct aaaattgaac gagaccatca 11340
   ttagtagcaa gatgtttgtc tatagtaaaa gaatatacta tgatgggaag attttaccac 11400
   agtgcctgaa agccttgacc aggtgtgtat tctggtccga gacactggta gatgaaaaca 11460
   gatctgcttg ttcgaacatc tcaacatcca tagcaaaagc tatcgaaaat gggtattctc 11520
   ctatactagg ctactgcatt gcgttgtata agacctgtca gcaggtgtgc atatcactag 11580
   ggatgactat aaatccaact atcagcccga ccgtaagaga tcaatacttt aagggtaaga 11640
   attggctgag atgtgcagtg ttgattccag caaatgttgg aggattcaac tacatgtcta 11700
   catctagatg ctttgttaga aatattggag accccgcagt agcagcccta gctgatctca 11760
   aaagattcat cagagcggat ctgttagaca agcaggtact atacagggtc atgaatcaag 11820
   aacccggtga ctctagcttt ctagattggg cttcagaccc ttattcatgt aacctcccgc 11880
   attctcagag tataactacg attataaaga atatcactgc tagatctgtg ctgcaggaat 11940
   ccccgaatcc tctactgtct ggtctcttca ccgagactag tggagaagag gatctcaacc 12000
   tggcctcgtt ccttatggac cggaaagtca tcctgccgag agtggctcat gagatcctgg 12060
   gtaattcctt aactggagtt agggaggcga ttgcagggat gcttgatacg accaagtctc 12120
   tagtgagatc cagcgttaag aaaggaggat tatcatatgg gatattgagg aggcttgtca 12180
   attatgatct attgcagtac gagacactga ctagaactct caggaaaccg gtgaaagaca 12240
   acatcgaata tgagtatatg tgttcagttg agctagctgt cggtctaagg cagaaaatgt 12300
   ggatccacct aacttacggg agacccatac atgggctaga aacaccagac cctttagagc 12360
   tcttgagggg aacatttatc gaaggttcag aggtgtgcaa gctttgcagg tctgagggag 12420
   cagaccccat ctatacatgg ttctatctcc ctgacaatat agacctggac acgcttacaa 12480
   acggatgtcc ggctataaga atcccctatt ttggatcagc cactgatgaa aggtcggaag 12540
   cccaactcgg gtatgtaaga aatctaagca aacccgcaaa ggctgccatc cggatagcta 12600
   tggtgtatac gtgggcctac gggactgatg agatatcgtg gatggaagcc gctcttatag 12660
   cccaaacaag agctaatctg agcttagaga atctaaagct gctgactcct gtttcaacct 12720
   ccactaatct atctcatagg ttgaaagata cggcaaccca gatgaagttc tctagtgcaa 12780
   cactagtccg tgcaagtcgg ttcataacaa tatcaaatga taacatggca ctcaaagaag 12840
   caggggagtc gaaggatact aatctcgtgt atcagcagat tatgctaact gggctaagct 12900
   tgttcgagtt caatatgaga tataagaaag gttccttagg gaagccactg atattgcact 12960
   tacatcttaa taacgggtgc tgtataatgg agtccccaca ggaggcgaat atccccccaa 13020
   ggtccacatt agatttagag attacacaag agaacaataa attgatctat gatcctgatc 13080
   cactcaagga tgtggacctt gagctattta gcaaggtcag agatgttgta catacagttg 13140
   acatgactta ttggtcagat gatgaagtta tcagagcaac cagtatctgt actgcaatga 13200
   cgatagctga tacaatgtct caattagata gagacaacct aaaagagatg atcgcgctag 13260
   taaatgacga tgatgtcaac agcctgatta ctgagtttat ggtgattgat gttcctttat 13320
   tttgctcaac gttcgggggt attctagtca atcagtttgc atactcactc tacggcttaa 13380
   acatcagagg aagggaagaa atatggggac atgtagtccg gattcttaaa gatacctccc 13440
   acgcagttct aaaagtctta tctaatgctc tatctcatcc caaaatcttc aaacgattct 13500
   ggaatgcagg tgtcgtggaa cctgtgtatg ggcctaacct ctcaaatcag gacaagatac 13560
   tcttggccct ctctgtctgt gaatattctg tggatctatt catgcacgat tggcaagggg 13620
   gtgtaccgct tgagatcttt atctgtgaca atgacccaga tgtggccgac atgaggaggt 13680
   cctctttctt ggcaagacat cttgcatacc tatgcagcgt ggcagagata tctagggatg 13740
   ggccaagatt agaatcaatg aactctctag agaggctcga gtcactaaag agttacctgg 13800
   aactcacatt tcttgatgac ccggtactga ggtacagtca gttgactggc ctagtcatca 13860
   aagtattccc atctactttg acctatatcc ggaagtcatc tataaaagtg ttaaggacaa 13920
   gaggtatagg agtccctgaa gtcttagaag attgggatcc cgaggcagat aatgcactgt 13980
   tagatggtat cgcggcagaa atacaacaga atattccttt gggacatcag actagagccc 14040
   ctttttgggg gttgagagta tccaagtcac aggtactgcg tctccggggg tacaaggaga 14100
   tcacaagagg tgagataggc agatcaggcg ttggtctgac gttaccattc gatggaagat 14160
   atctatctca ccagctgagg ctctttggca tcaacagtac tagctgcttg aaagcacttg 14220
   aacttaccta cctattgagc cccttagttg acaaggataa agataggcta tatttagggg  14280
   aaggagctgg ggccatgctt tcctgttatg acgctactct tggcccatgc atcaactatt  14340
   ataactcagg ggtatactct tgtgatgtca atgggcagag agagttaaat atatatcctg  14400
   ctgaggtggc actggtggga aagaaattaa acaatgttac tagtctgggt caaagagtta  14460
   aagtgttatt caacgggaat cctggctcga catggattgg aaatgatgag tgtgaggctt  14520
   tgatttggaa tgaattgcag aatagctcga taggcctagt ccactgtgac atggagggag  14580
   gagatcataa ggatgatcaa gttgtactgc atgagcatta cagtgtaatc cggatcgcgt  14640
   atctggtggg ggatcgagac gttgtgctta taagcaagat tgctcctagg ctgggcacgg  14700
   attggaccag gcagctcagc ctatatctga gatactggga cgaggttaac ctaatagtgc  14760
   ttaaaacatc taaccctgct tccacagaga tgtatctcct atcgaggcat cccaaatctg  14820
   acattataga ggacagcaag acggtgttag ctagtctcct ccctttgtca aaagaagata  14880
   gcatcaagat agaaaagtgg atcttaatag agaaggcaaa ggctcacgaa tgggttactc  14940
   gggaattgag agaaggaagc tcttcatcag ggatgcttag accttaccat caagcactgc  15000
   agacgtttgg ctttgaacca aacttgtata aattgagcag agatttcttg tccaccatga  15060
   acatagctga tacacacaac tgcatgatag ctttcaacag ggttttgaag gatacaatct  15120
   tcgaatgggc tagaataact gagtcagata aaaggcttaa actaactggt aagtatgacc  15180
   tgtatcctgt gagagattca ggcaaattga agacagtttc tagaagactt gtgctatctt  15240
   ggatatcttt atctatgtcc acaagattgg taactgggtc attccctgac cagaagtttg  15300
   aagcaagact tcaattggga atagtttcat tatcatcccg tgaaatcagg aacctgaggg  15360
   ttatcacaaa aactttatta gaccggtttg aggatattat acatagtata acgtacagat  15420
   tcctcaccaa agaaataaag attttgatga agattttagg ggcagtcaag atgttcgggg  15480
   ccaggcaaaa tgaatacacg accgtgattg atgatggatc actgggtgat atcgagccat  15540
   atgacagctc gtaataatta gtccctatcg tgcagaacga tcgaagctcc gcggtacctg  15600
   gaagtcttgg actgatccat atgacaatag taagaaaaac ttacaagaag acaagaaaat  15660
   ttaaaagaat acatatctct taaactcttg tctggt                            15696
<210> 14
   <211> 21
   <212> RNA
   <213> Artificial
<220>
   <223> siRNA:Sense strand
<400> 14
   gguucagcau caaauaugaa g 21
<210> 15
   <211> 21
   <212> RNA
   <213> Artificial
<220>
   <223> siRNA:Anti-sense strand
<400> 15
   ucauauuuga ugcugaacca u 21
<210> 16
   <211> 21
   <212> RNA
   <213> Artificial
<220>
   <223> siRNA:Sense strand
<400> 16
   gguccagaca ugaauucaaa g 21
<210> 17
   <211> 21
   <212> RNA
   <213> Artificial
<220>
   <223> siRNA:Anti-sense strand
<400> 17
   uugaauucau gucuggacca u 21
<210> 18
   <211> 20
   <212> DNA
   <213> Artificial
<220>
   <223> Primer
<400> 18
   ggaagcatcg aattctggga 20
<210> 19
   <211> 20
   <212> DNA
   <213> Artificial
<220>
   <223> Primer
<400> 19
   cggagcagca ttccaaggct 20
<210> 20
   <211> 20
   <212> DNA
   <213> Artificial
<220>
   <223> Primer
<400> 20
   tgagccgtct ttccaccagg 20
<210> 21
   <211> 20
   <212> DNA
   <213> Artificial
<220>
   <223> Primer
<400> 21
   acatggtctc cagactccac 20
<210> 22
   <211> 20
   <212> DNA
   <213> Artificial
<220>
   <223> Primer
<400> 22
   agcatccgac tgtaaagaat 20
<210> 23
   <211> 20
   <212> DNA
   <213> Artificial
<220>
   <223> Primer
<400> 23
   cctctccaca gttatagaag 20
<210> 24
   <211> 20
   <212> DNA
   <213> Artificial
<220>
   <223> Primer
<400> 24
   aagaccctaa gaggacgaag 20
<210> 25
   <211> 25
   <212> DNA
   <213> Artificial
<220>
   <223> Primer
<400> 25
   actcccatgg cgtaactcca tagtg 25
<210> 26
   <211> 18
   <212> DNA
   <213> Artificial
<220>
   <223> Primer
<400> 26
   actgttgctg gggaatgg 18
<210> 27
   <211> 19
   <212> DNA
   <213> Artificial
<220>
   <223> Primer
<400> 27
   ccaagttcaa agctgctgg 19
<210> 28
   <211> 22
   <212> DNA
   <213> Artificial
<220>
   <223> Primer
<400> 28
   ttctggcctc tgtgaagtag tg 22
<210> 29
   <211> 20
   <212> DNA
   <213> Artificial
<220>
   <223> Primer
<400> 29
   gtgaaaccat ggtgcagatg 20
<210> 30
   <211> 24
   <212> DNA
   <213> Artificial
<220>
   <223> Primer
<400> 30
   aggaatactg aatgtggact ttcc 24
<210> 31
   <211> 23
   <212> DNA
   <213> Artificial
<220>
   <223> Primer
<400> 31
   tcccttgatt aacagaagac ctg 23
<210> 32
   <211> 39
   <212> DNA
   <213> Artificial
<220>
   <223> Primer
<400> 32
   actagctagc ttagacgctg gatttttttc gggtagtgg 39
<210> 33
   <211> 30
   <212> DNA
   <213> Artificial
<220>
   <223> Primer
<400> 33
   gtccaccggt cttacgcaca agagttccgt 30
<210> 34
   <211> 35
   <212> DNA
   <213> Artificial
<220>
   <223> Prmer
<400> 34
   agtacctagg cgcatgtaca acatgatgga gacgg 35
<210> 35
   <211> 33
   <212> DNA
   <213> Artificial
<220>
   <223> Primer
<400> 35
   gtccgacgtc ctcacatgtg tgagaggggc agt 33
<210> 36
   <211> 40
   <212> DNA
   <213> Artificial
<220>
   <223> Primer
<400> 36
   actagctagc ggttccccat ggcgggacac ctggcttcgg 40
<210> 37
   <211> 35
   <212> DNA
   <213> Artificial
<220>
   <223> Primer
<400> 37
   ggtccacgcg ttcagtttga atgcatggga gagcc 35
<210> 38
   <211> 15702
   <212> DNA
   <213> Artificial
<220>
   <223> Full length genome cDNA of Sendai virus vector-version2
<400> 38
   accaaacaag agaagaaaca tgtatggaat atataatgaa gttagacagg attttagggt     60
   caaagtatcc accctgagga gcaggttcca gatccttttc tttgctgcca aagttcacga    120
   tggccgggtt gttgagcacc ttcgatacat ttagctctag gaggagcgaa agtattaata    180
   agtcgggagg aggtgctgtt atccccggcc agaggagcac agtctcagtg ttcatactag    240
   gcccaagtgt gactgatgat gcagacaagt tattcattgc aacaaccttc ctagctcact    300
   cattggacac agataagcag cactctcaga gaggagggtt cctcgtctct ctgcttgcca    360
   tggcttacag tagtccagaa ttgtacttga caacaaacgg agtaaacgcc gatgtcaaat    420
   atgtgatcta caacatagag aaagacccta agaggacgaa gacagacgga ttcattgtga    480
   agacgagaga tatggaatat gagaggacca cagaatggct gtttggacct atggtcaaca    540
   agagcccact cttccagggt caacgggatg ctgcagaccc tgacacactc cttcaaatct    600
   atgggtatcc tgcatgccta ggagcaataa ttgtccaagt ctggattgtg ctggtgaagg    660
   ccatcacaag cagcgccggc ttaaggaaag ggttcttcaa caggttagag gcgttcagac    720
   aagacggcac cgtgaaaggt gccttagttt tcactgggga gacagttgag gggataggct    780
   cggttatgag atctcagcaa agccttgtat ctctcatggt tgagaccctt gtgactatga    840
   atactgcaag atctgatctc accacattag agaagaacat ccagatcgtt gggaactaca    900
   tccgagatgc agggctggct tccttcatga acactattaa atatggggtg gagacaaaga    960
   tggcagctct aacgttgtca aacctgaggc ccgatattaa taagcttaga agcctcatag   1020
   acacctacct gtcaaaaggc cccagagctc cctttatctg tatcctcaag gaccctgttc   1080
   atggtgaatt tgctccaggc aattatcctg cactatggag ttacgccatg ggagtcgccg   1140
   tcgtacagaa caagtcaatg cagcagtacg tcacagggag gacatacctt gatatggaaa   1200
   tgttcttact aggacaagcc gtggcaaagg atgctgaatc gaagatcagc agtgccttgg   1260
   aagatgagtt aggagtgacg gatacagcca aggagaggct cagacatcat ctggcaaact 1320
   tgtccggtgg ggatggtgct taccacaaac caacaggcgg tggtgcaatt gaggtagctc 1380
   tagacaatgc cgatatcgac ctggaaacag aagctcatgc ggaccaggac gctaggggtt 1440
   ggggtggaga tagtggtgaa agatgggcac gtcaggtgag tggtggccac tttgtcacac 1500
   tacatggggc tgaacggtta gaggaggaaa ccaatgatga ggatgtatca gacatagaga 1560
   gaagaatagc catgagactc gcagagagac ggcaagagga ttctgcaacc catggagatg 1620
   aaggccgcaa taacggtgtt gatcacgaag aagatgacga tgccgcagca gcagctggga 1680
   taggaggaat ctaggatcat acgaggcctc aaggtacttg atccgcagta agaaaaactt 1740
   agggtgaaag ttcatccacc gatcggctca ggcaaggcca cacccaaccc caccgaccac 1800
   acccagcagt cgagacagcc acggcttcgg ctacacttac cgcatggatc aagatgcctt 1860
   cattcttaaa gaagattctg aagttgagag gaaggcgcca ggaggacgag agtcgctctc 1920
   ggatgttatc ggattcctcg atgctgtcct gtcgaatgaa ccaactgaca tcggagggga 1980
   cagaagctgg ctccacaaca ccatcaacac tccccaagga ccaggctctg ctcatagagc 2040
   caaaagtgag ggcgaaggag aagtctcaac accgtcgacc caagataatc gatcaggtga 2100
   ggagagtaga gtctctggga gaacaagcaa gccagaggca gaagcacatg ctggaaacct 2160
   tgataaacaa aatatacact gggcctttag gggaagaact ggtacaaact ctgtatctca 2220
   ggatctggac gatggaggag actccggaat ccttgaaaat cctccaaatg agagaggata 2280
   tccgagatca ggtattgaag atgaaaacag agagatggct gcgcaccctg ataagagggg 2340
   agaagaccaa gctgaaggac ttccagaaga ggtacgagga ggtacatccc tacctgatga 2400
   aggagaaggt ggagcaagta ataatggaag aagcatggag cctggcagct cacatagtgc 2460
   aagagtaact ggggtcctgg tgattcctag ccccgaactt gaagaggctg tgctacggag 2520
   gaacaaaaga agacctacca acagtgggtc caaacctctt actccagcaa ccgtgcctgg 2580
   cacccggtcc ccaccgctga atcgttacaa cagcacaggg tcaccaccag gaaaaccccc 2640
   atctacacag gatgagcaca tcaactctgg ggacaccccc gccgtcaggg tcaaagaccg 2700
   gaaaccatca atagggactc gctctgtctc agattgtcca gccaacggcc gcccaatcca 2760
   tccgggtata gagaccgact caacaaaaaa gggcatagga gagaacacat catctatgaa 2820
   agatatggct acattgttga cgagtcttgg tgtaatccag tctgctcaag aattcgagtc 2880
   atcccgagac gcgagttatg tgtttgcaag acgtgcccta aagtctgcaa actatgcaga 2940
   gatgacattc aatgtatgcg gcctgatcct ttctgccgag aaatcttccg ctcgtaaggt 3000
   agatgagaac aaacaactgc tcaaacagat ccaagagagc gtggaatcat tccgggatat 3060
   ttacaagaga ttctctgagt atcagaaaga acagaactca ttgctgatgt ccaacctatc 3120
   tacacttcat atcatcacag atagaggtgg caagactgac aacacagact cccttacaag 3180
   gtccccctcc gtttttgcaa aatcaaaaga gaacaagact aaggctacca ggtttgaccc 3240
   atctatggag accttagaag atatgaagta caaaccggac ctaatccgag aggatgaatt 3300
   tagagatgag atccgcaacc cggtgtacca agagagggac acagaaccca gggcctcaaa 3360
   cgcatcacgt ctcttcccct ccaaagagaa gcacacaatg cactctctca ggctcgtcat 3420
   agagagcagt cccctaagca gagctgagaa agcagcatat gtgaaatcat tatccaagtg 3480
   caagacagac caagaggtta aggcagtcat ggaactcgta gaagaggaca tagagtcact 3540
   gaccaactag atcccgggtg aggcatccca ccatcctcag tcacagagag acccaatcta 3600
   ccatcagcat cagccagtaa agattaagaa aaacttaggg tgaaagaaat ttcaccgcta 3660
   gcacctaggt ctgacatggc tgtcagcgac gcgctgctcc catctttctc cacgttcgcg 3720
   tctggcccgg cgggaaggga gaagacactg cgtcaagcag gtgccccgaa taaccgctgg 3780
   cgggaggagc tctcccacat gaagcgactt cccccagtgc ttcccggccg cccctatgac 3840
   ctggcggcgg cgaccgtggc cacagacctg gagagcggcg gagccggtgc ggcttgcggc 3900
   ggtagcaacc tggcgcccct acctcggaga gagaccgagg agttcaacga tctcctggac 3960
   ctggacttta ttctctccaa ttcgctgacc catcctccgg agtcagtggc cgccaccgtg 4020
   tcctcgtcag cgtcagcctc ctcttcgtcg tcgccgtcga gcagcggccc tgccagcgcg 4080
   ccctccacct gcagcttcac ctatccgatc cgggccggga acgacccggg cgtggcgccg 4140
   ggcggcacgg gcggaggcct cctctatggc agggagtccg ctccccctcc gacggctccc 4200
   ttcaacctgg cggacatcaa cgacgtgagc ccctcgggcg gcttcgtggc cgagctcctg 4260
   cggccagaat tggacccggt gtacattccg ccgcagcagc cgcagccgcc aggtggcggg 4320
   ctgatgggca agttcgtgct gaaggcgtcg ctgagcgccc ctggcagcga gtacggcagc 4380
   ccgtcggtca tcagcgtcag caaaggcagc cctgacggca gccacccggt ggtggtggcg 4440
   ccctacaacg gcgggccgcc gcgcacgtgc cccaagatca agcaggaggc ggtctcttcg 4500
   tgcacccact tgggcgctgg accccctctc agcaatggcc accggccggc tgcacacgac 4560
   ttccccctgg ggcggcagct ccccagcagg actaccccga ccctgggtct tgaggaagtg 4620
   ctgagcagca gggactgtca ccctgccctg ccgcttcctc ccggcttcca tccccacccg 4680
   gggcccaatt acccatcctt cctgcccgat cagatgcagc cgcaagtccc gccgctccat 4740
   taccaagagc tcatgccacc cggttcctgc atgccagagg agcccaagcc aaagagggga 4800
   agacgatcgt ggccccggaa aaggaccgcc acccacactt gtgattacgc gggctgcggc 4860
   aaaacctaca caaagagttc ccatctcaag gcacacctgc gaacccacac aggtgagaaa 4920
   ccttaccact gtgactggga cggctgtgga tggaaattcg cccgctcaga tgaactgacc 4980
   aggcactacc gtaaacacac ggggcaccgc ccgttccagt gccaaaaatg cgaccgagca 5040
   ttttccaggt cggaccacct cgccttacac atgaagaggc atttttaaag acgtcgatta 5100
   agaaaaactt agggtgaaag ttcatcgcgg ccgcttgcta gcggttcccc atggcgggac 5160
   acctggcttc ggatttcgcc ttctcgcccc ctccaggtgg tggaggtgat gggccagggg 5220
   ggccggagcc gggctgggtt gatcctcgga cctggctaag cttccaaggc cctcctggag 5280
   ggccaggaat cgggccgggg gttgggccag gctctgaggt gtgggggatt cccccatgcc 5340
   ccccgccgta tgagttctgt ggggggatgg cgtactgtgg gccccaggtt ggagtggggc 5400
   tagtgcccca aggcggcttg gagacctctc agcctgaggg cgaagcagga gtcggggtgg 5460
   agagcaactc cgatggggcc tccccggagc cctgcaccgt cacccctggt gccgtgaagc 5520
   tggagaagga gaagctggag caaaacccgg aggagtccca ggacatcaaa gctctgcaga 5580
   aagaactcga gcaatttgcc aagctcctga agcagaagag gatcaccctg ggatatacac 5640
   aggccgatgt ggggctcacc ctgggggttc tatttgggaa ggtattcagc caaacgacca 5700
   tctgccgctt tgaggctctg cagcttagct tcaagaacat gtgtaagctg cggcccttgc 5760
   tgcagaagtg ggtggaggaa gctgacaaca atgaaaatct tcaggagata tgcaaagcag 5820
   aaaccctcgt gcaggcccga aagagaaagc gaaccagtat cgagaaccga gtgagaggca 5880
   acctggagaa tttgttcctg cagtgcccga aacccacact gcagcagatc agccacatcg 5940
   cccagcagct tgggctcgag aaggatgtgg tccgagtgtg gttctgtaac cggcgccaga 6000
   agggcaagcg atcaagcagc gactatgcac aacgagagga ttttgaggct gctgggtctc 6060
   ctttctcagg gggaccagtg tcctttcctc tggccccagg gccccatttt ggtaccccag 6120
   gctatgggag ccctcacttc actgcactgt actcctcggt ccctttccct gagggggaag 6180
   cctttccccc tgtctccgtc accactctgg gctctcccat gcattcaaac tgaacgcgtc 6240
   agagacctgc aacaatgtct caagcagaca ccacctggca gtcggagcca ccgggtcact 6300
   ccttgtctta aataagaaaa acttagggat aaagtccctt agatctagcc taggcgcatg 6360
   tacaacatga tggagacgga gctgaagccg ccgggcccgc agcaaacttc ggggggcggc 6420
   ggcggcaact ccaccgcggc ggcggccggc ggcaaccaga aaaacagccc ggaccgcgtc 6480
   aagcggccca tgaatgcctt catggtgtgg tcccgcgggc agcggcgcaa gatggcccag 6540
   gagaacccca agatgcacaa ctcggagatc agcaagcgcc tgggcgccga gtggaaactt 6600
   ttgtcggaga cggagaagcg gccgttcatc gacgaggcta agcggctgcg agcgctgcac 6660
   atgaaggagc acccggatta taaataccgg ccccggcgga aaaccaagac gctcatgaag 6720
   aaggataagt acacgctgcc cggcgggctg ctggcccccg gcggcaatag catggcgagc 6780
   ggggtcgggg tgggcgccgg cctgggcgcg ggcgtgaacc agcgcatgga cagttacgcg 6840
   cacatgaacg gctggagcaa cggcagctac agcatgatgc aggaccagct gggctacccg 6900
   cagcacccgg gcctcaatgc gcacggcgca gcgcagatgc agcccatgca ccgctacgac 6960
   gtgagcgccc tgcagtacaa ctccatgacc agctcgcaga cctacatgaa cggctcgccc 7020
   acctacagca tgtcctactc gcagcagggc acccctggca tggctcttgg ctccatgggt 7080
   tcggtggtca agtccgaggc cagctccagc ccccctgtgg ttacctcttc ctcccactcc 7140
   agggcgccct gccaggccgg ggacctccgg gacatgatca gcatgtatct ccccggcgcc 7200
   gaggtgccgg aacccgccgc ccccagcaga cttcacatgt cccagcacta ccagagcggc 7260
   ccggtgcccg gcacggccat taacggcaca ctgcccctct cacacatgtg aggacgtcag 7320
   atctgtatat aataagaaaa acttagggtg aaagtgaggt tgcgcggtat tttagctagc 7380
   ttagacgctg gatttttttc gggtagtgga aaaccagcag cctcccgcga cgatgcccct 7440
   caacgttagc ttcaccaaca ggaactatga cctcgactac gactcggtgc agccgtattt 7500
   ctactgcgac gaggaggaga acttctacca gcagcagcag cagagcgagc tgcagccccc 7560
   ggcgcccagc gaggatatct ggaagaaatt cgagctgctg cccaccccgc ccctgtcccc 7620
   tagccgccgc tccgggctct gctcgccctc ctacgttgcg gtcacaccct tctcccttcg 7680
   gggagacaac gacggcggtg gcgggagctt ctccacggcc gaccagctgg agatggtgac 7740
   cgagctgctg ggaggagaca tggtgaacca gagtttcatc tgcgacccgg acgacgagac 7800
   cttcatcaaa aacatcatca tccaggactg tatgtggagc ggcttctcgg ccgccgccaa 7860
   gctcgtctca gagaagctgg cctcctacca ggctgcgcgc aaagacagcg gcagcccgaa 7920
   ccccgcccgc ggccacagcg tctgctccac ctccagcttg tacctgcagg atctgagcgc 7980
   cgccgcctca gagtgcatcg acccctcggt ggtcttcccc taccctctca acgacagcag 8040
   ctcgcccaag tcctgcgcct cgcaagactc cagcgccttc tctccgtcct cggattctct 8100
   gctctcctcg acggagtcct ccccgcaggg cagccccgag cccctggtgc tccatgagga 8160
   gacaccgccc accaccagca gcgactctga ggaggaacaa gaagatgagg aagaaatcga 8220
   tgttgtttct gtggaaaaga ggcaggctcc tggcaaaagg tcagagtctg gatcaccttc 8280
   tgctggaggc cacagcaaac ctcctcacag cccactggtc ctcaagaggt gccacgtctc 8340
   cacacatcag cacaactacg cagcgcctcc ctccactcgg aaggactatc ctgctgccaa 8400
   gagggtcaag ttggacagtg tcagagtcct gagacagatc agcaacaacc gaaaatgcac 8460
   cagccccagg tcctcggaca ccgaggagaa tgtcaagagg cgaacacaca acgtcttgga 8520
   gcgccagagg aggaacgagc taaaacggag cttttttgcc ctgcgtgacc agatcccgga 8580
   gttggaaaac aatgaaaagg cccccaaggt agttatcctt aaaaaagcca cagcatacat 8640
   cctgtccgtc caagcagagg agcaaaagct catttctgaa gaggacttgt tgcggaaacg 8700
   acgagaacag ttgaaacaca aacttgaaca gctacggaac tcttgtgcgt aagaccggtg 8760
   tcggctttgc tgacactaga gtcatctccg aacatccaca atatctctca gtctcttacg 8820
   tctctcacag tattaagaaa aacccagggt gaatgggaag cttgccatag gtcatggatg 8880
   ggcaggagtc ctcccaaaac ccttctgaca tactctatcc agaatgccac ctgaactctc 8940
   ccatagtcag ggggaagata gcacagttgc acgtcttgtt agatgtgaac cagccctaca 9000
   gactaaagga cgacagcata ataaatatta caaagcacaa aattaggaac ggaggattgt 9060
   cccctcgtca aattaagatc aggtctctgg gtaaggctct tcaacgcaca ataaaggatt 9120
   tagaccgata cacctttgaa ccgtacccaa cctactctca ggaattactt aggcttgata 9180
   taccagagat atgtgacaaa atccgatccg tcttcgcggt ctcggatcgg ctgaccaggg 9240
   agttatctag tgggttccag gatctttggt tgaatatctt caagcaacta ggcaatatag 9300
   aaggaagaga ggggtacgat ccgttgcagg atatcggcac catcccggag ataactgata 9360
   aatacagcag gaatagatgg tataggccat tcctaacttg gttcagcatc aaatatgaca 9420
   tgcggtggat gcagaagacc agaccggggg gacccctcga tacctctaat tcacataacc 9480
   tcctagaatg caaatcatac actctagtaa catacggaga tcttatcatg atactgaaca 9540
   agttgacatt gacagggtat atcctaaccc ctgagctggt cttgatgtat tgtgatgttg 9600
   tagagggaag gtggaatatg tctgctgcag ggcatctaga taagaagtcc attgggataa 9660
   caagcaaagg tgaggaatta tgggaactag tggattccct cttctcaagt cttggagagg 9720
   aaatatacaa tgtcatcgca ctattggagc ccctatcact tgctctcata caactaaatg 9780
   atccagttat acctctacgt ggggcattta tgaggcatgt gttgacagag ctacaggctg 9840
   ttttaacaag tagggacgtg tacacagatg ctgaagcaga cactattgtg gagtcgttac 9900
   tcgccatttt ccatggaacc tctattgatg agaaagcaga gatcttttcc ttctttagga 9960
   catttggcca ccccagctta gaggctgtca ctgccgccga caaggtaagg gcccatatgt 10020
   atgcacaaaa ggcaataaag cttaagaccc tatacgagtg tcatgcagtt ttttgcacta 10080
   tcatcataaa tgggtataga gagaggcatg gcggacagtg gcccccctgt gacttccctg 10140
   atcacgtgtg tctagaacta aggaacgctc aagggtccaa tacggcaatc tcttatgaat 10200
   gtgctgtaga caactataca agtttcatag gcttcaagtt tcggaagttt atagaaccac 10260
   aactagatga agatctcaca atatatatga aagacaaagc actatccccc aggaaggagg 10320
   catgggactc tgtatacccg gatagtaatc tgtactataa agccccagaa tctgaagaga 10380
   cccggcggct tattgaagtg ttcataaatg atgagaattt caacccagaa gaaattatca 10440
   attatgtgga gtcaggagat tggttgaaag acgagaagtt caacatctcg tacagtctca 10500
   aagagaaaga gatcaagcaa gagggtcgtc tattcgcaaa aatgacttat aagatgcgag 10560
   ccgtacaggt gctggcagag acactactgg ctaaaggaat aggagagctg ttcagcgaaa 10620
   atgggatggt taaaggagag atagacctac ttaaaagatt gactactctt tctgtctcag 10680
   gagtccccag gactgattca gtgtacaata actctaaatc atcagagaag agaaacgaag 10740
   gcatgaaaaa gaagaactct ggggggtact gggacgaaaa gaagaggtcc agacatgaat 10800
   tcaaggcaac agattcatca acagacggct atgaaacgtt aagttgcttc ctcacaacag 10860
   acctcaagaa atactgctta aactggagat ttgaaagtac tgcattgttt ggtcagagat 10920
   gcaacgagat atttggcttc aagaccttct ttaactggat gcatccagtc cttgaaaggt 10980
   gtacaatata tgttggggat ccttactgtc cagtcgccga ccggatgcat cgacaactcc 11040
   aggatcatgc agactctggc attttcatac ataatcctag ggggggcata gaaggttact 11100
   gccagaagct gtggacctta atctcaatca gtgcaatcca cctagcagct gtgagagtgg 11160
   gtgtcagggt ctctgcaatg gttcagggtg acaatcaagc tatagccgtg acatcaagag 11220
   tacctgtagc tcagacttac aagcagaaga aaaatcatgt ctataaggag atcaccaaat 11280
   attttggtgc tctaagacac gtcatgtttg atgtagggca cgagctaaaa ttgaacgaga 11340
   ccatcattag tagcaagatg tttgtctata gtaaaagaat atactatgat gggaagattt 11400
   taccacagtg cctgaaagcc ttgaccaggt gtgtattctg gtccgagaca ctggtagatg 11460
   aaaacagatc tgcttgttcg aacatctcaa catccatagc aaaagctatc gaaaatgggt 11520
   attctcctat actaggctac tgcattgcgt tgtataagac ctgtcagcag gtgtgcatat 11580
   cactagggat gactataaat ccaactatca gcccgaccgt aagagatcaa tactttaagg 11640
   gtaagaattg gctgagatgt gcagtgttga ttccagcaaa tgttggagga ttcaactaca 11700
   tgtctacatc tagatgcttt gttagaaata ttggagaccc cgcagtagca gccctagctg 11760
   atctcaaaag attcatcaga gcggatctgt tagacaagca ggtactatac agggtcatga 11820
   atcaagaacc cggtgactct agctttctag attgggcttc agacccttat tcatgtaacc 11880
   tcccgcattc tcagagtata actacgatta taaagaatat cactgctaga tctgtgctgc 11940
   aggaatcccc gaatcctcta ctgtctggtc tcttcaccga gactagtgga gaagaggatc 12000
   tcaacctggc ctcgttcctt atggaccgga aagtcatcct gccgagagtg gctcatgaga 12060
   tcctgggtaa ttccttaact ggagttaggg aggcgattgc agggatgctt gatacgacca 12120
   agtctctagt gagatccagc gttaagaaag gaggattatc atatgggata ttgaggaggc 12180
   ttgtcaatta tgatctattg cagtacgaga cactgactag aactctcagg aaaccggtga 12240
   aagacaacat cgaatatgag tatatgtgtt cagttgagct agctgtcggt ctaaggcaga 12300
   aaatgtggat ccacctaact tacgggagac ccatacatgg gctagaaaca ccagaccctt 12360
   tagagctctt gaggggaaca tttatcgaag gttcagaggt gtgcaagctt tgcaggtctg 12420
   agggagcaga ccccatctat acatggttct atctccctga caatatagac ctggacacgc 12480
   ttacaaacgg atgtccggct ataagaatcc cctattttgg atcagccact gatgaaaggt 12540
   cggaagccca actcgggtat gtaagaaatc taagcaaacc cgcaaaggct gccatccgga 12600
   tagctatggt gtatacgtgg gcctacggga ctgatgagat atcgtggatg gaagccgctc 12660
   ttatagccca aacaagagct aatctgagct tagagaatct aaagctgctg actcctgttt 12720
   caacctccac taatctatct cataggttga aagatacggc aacccagatg aagttctcta 12780
   gtgcaacact agtccgtgca agtcggttca taacaatatc aaatgataac atggcactca 12840
   aagaagcagg ggagtcgaag gatactaatc tcgtgtatca gcagattatg ctaactgggc 12900
   taagcttgtt cgagttcaat atgagatata agaaaggttc cttagggaag ccactgatat 12960
   tgcacttaca tcttaataac gggtgctgta taatggagtc cccacaggag gcgaatatcc 13020
   ccccaaggtc cacattagat ttagagatta cacaagagaa caataaattg atctatgatc 13080
   ctgatccact caaggatgtg gaccttgagc tatttagcaa ggtcagagat gttgtacata 13140
   cagttgacat gacttattgg tcagatgatg aagttatcag agcaaccagt atctgtactg 13200
   caatgacgat agctgataca atgtctcaat tagatagaga caacctaaaa gagatgatcg 13260
   cgctagtaaa tgacgatgat gtcaacagcc tgattactga gtttatggtg attgatgttc 13320
   ctttattttg ctcaacgttc gggggtattc tagtcaatca gtttgcatac tcactctacg 13380
   gcttaaacat cagaggaagg gaagaaatat ggggacatgt agtccggatt cttaaagata 13440
   cctcccacgc agttctaaaa gtcttatcta atgctctatc tcatcccaaa atcttcaaac 13500
   gattctggaa tgcaggtgtc gtggaacctg tgtatgggcc taacctctca aatcaggaca 13560
   agatactctt ggccctctct gtctgtgaat attctgtgga tctattcatg cacgattggc 13620
   aagggggtgt accgcttgag atctttatct gtgacaatga cccagatgtg gccgacatga 13680
   ggaggtcctc tttcttggca agacatcttg catacctatg cagcgtggca gagatatcta 13740
   gggatgggcc aagattagaa tcaatgaact ctctagagag gctcgagtca ctaaagagtt 13800
   acctggaact cacatttctt gatgacccgg tactgaggta cagtcagttg actggcctag 13860
   tcatcaaagt attcccatct actttgacct atatccggaa gtcatctata aaagtgttaa 13920
   ggacaagagg tataggagtc cctgaagtct tagaagattg ggatcccgag gcagataatg 13980
   cactgttaga tggtatcgcg gcagaaatac aacagaatat tcctttggga catcagacta 14040
   gagccccttt ttgggggttg agagtatcca agtcacaggt actgcgtctc cgggggtaca 14100
   aggagatcac aagaggtgag ataggcagat caggcgttgg tctgacgtta ccattcgatg 14160
   gaagatatct atctcaccag ctgaggctct ttggcatcaa cagtactagc tgcttgaaag 14220
   cacttgaact tacctaccta ttgagcccct tagttgacaa ggataaagat aggctatatt 14280
   taggggaagg agctggggcc atgctttcct gttatgacgc tactcttggc ccatgcatca 14340
   actattataa ctcaggggta tactcttgtg atgtcaatgg gcagagagag ttaaatatat 14400
   atcctgctga ggtggcactg gtgggaaaga aattaaacaa tgttactagt ctgggtcaaa 14460
   gagttaaagt gttattcaac gggaatcctg gctcgacatg gattggaaat gatgagtgtg 14520
   aggctttgat ttggaatgaa ttgcagaata gctcgatagg cctagtccac tgtgacatgg 14580
   agggaggaga tcataaggat gatcaagttg tactgcatga gcattacagt gtaatccgga 14640
   tcgcgtatct ggtgggggat cgagacgttg tgcttataag caagattgct cctaggctgg 14700
   gcacggattg gaccaggcag ctcagcctat atctgagata ctgggacgag gttaacctaa 14760
   tagtgcttaa aacatctaac cctgcttcca cagagatgta tctcctatcg aggcatccca 14820
   aatctgacat tatagaggac agcaagacgg tgttagctag tctcctccct ttgtcaaaag 14880
   aagatagcat caagatagaa aagtggatct taatagagaa ggcaaaggct cacgaatggg 14940
   ttactcggga attgagagaa ggaagctctt catcagggat gcttagacct taccatcaag 15000
   cactgcagac gtttggcttt gaaccaaact tgtataaatt gagcagagat ttcttgtcca 15060
   ccatgaacat agctgataca cacaactgca tgatagcttt caacagggtt ttgaaggata 15120
   caatcttcga atgggctaga ataactgagt cagataaaag gcttaaacta actggtaagt 15180
   atgacctgta tcctgtgaga gattcaggca aattgaagac agtttctaga agacttgtgc 15240
   tatcttggat atctttatct atgtccacaa gattggtaac tgggtcattc cctgaccaga 15300
   agtttgaagc aagacttcaa ttgggaatag tttcattatc atcccgtgaa atcaggaacc 15360
   tgagggttat cacaaaaact ttattagacc ggtttgagga tattatacat agtataacgt 15420
   acagattcct caccaaagaa ataaagattt tgatgaagat tttaggggca gtcaagatgt 15480
   tcggggccag gcaaaatgaa tacacgaccg tgattgatga tggatcactg ggtgatatcg 15540
   agccatatga cagctcgtaa taattagtcc ctatcgtgca gaacgatcga agctccgcgg 15600
   tacctggaag tcttggactg atccatatga caatagtaag aaaaacttac aagaagacaa 15660
   gaaaatttaa aagaatacat atctcttaaa ctcttgtctg gt 15702
<210> 39
   <211> 63
   <212> DNA
   <213> Artificial
<220>
   <223> Oligo DNA for introducing target sequence of mir-302a
<400> 39
   ccggttatca ccaaaacatg gaagcactta cgattcacca aaacatggaa gcacttaggt 60
   acc 63
<210> 40
   <211> 53
   <212> DNA
   <213> Artificial
<220>
   <223> Oligo DNA for introducing target sequence of mir-302a
<400> 40
   taagtgcttc catgttttgg tgaatcgtaa gtgcttccat gttttggtga taa 53
<210> 41
   <211> 51
   <212> DNA
   <213> Artificial
<220>
   <223> Oligo DNA for introducing target sequence of mir-302a
<400> 41
   tcaccaaaac atggaagcac ttacgattca ccaaaacatg gaagcactta a 51
<210> 42
   <211> 61
   <212> DNA
   <213> Artificial
<220>
   <223> Oligo DNA for introducing target sequence of mir-302a
<400> 42
   ccggttaagt gcttccatgt tttggtgaat cgtaagtgct tccatgtttt ggtgaggtac 60
   c 61
<210> 43
   <211> 6576
   <212> DNA
   <213> Artificial
<220>
   <223> pNK15(vector used to making Sendai virus vector having target s equence of mir-302a)
<400> 43
   atcggcacca tcccggagat aactgataaa tacagcagga atagatggta taggccattc     60
   ctaacttggt tcagcatcaa atatgacatg cggtggatgc agaagaccag accgggggga    120
   cccctcgata cctctaattc acataacctc ctagaatgca aatcatacac tctagtaaca    180
   tacggagatc ttatcatgat actgaacaag ttgacattga cagggtatat cctaacccct    240
   gagctggtct tgatgtattg tgatgttgta gagggaaggt ggaatatgtc tgctgcaggg    300
   catctagata agaagtccat tgggataaca agcaaaggtg aggaattatg ggaactagtg    360
   gattccctct tctcaagtct tggagaggaa atatacaatg tcatcgcact attggagccc    420
   ctatcacttg ctctcataca actaaatgat ccagttatac ctctacgtgg ggcatttatg    480
   aggcatgtgt tgacagagct acaggctgtt ttaacaagta gggacgtgta cacagatgct    540
   gaagcagaca ctattgtgga gtcgttactc gccattttcc atggaacctc tattgatgag    600
   aaagcagaga tcttttcctt ctttaggaca tttggccacc ccagcttaga ggctgtcact    660
   gccgccgaca aggtaagggc ccatatgtat gcacaaaagg caataaagct taagacccta    720
   tacgagtgtc atgcagtttt ttgcactatc atcataaatg ggtatagaga gaggcatggc    780
   ggacagtggc ccccctgtga cttccctgat cacgtgtgtc tagaactaag gaacgctcaa    840
   gggtccaata cggcaatctc ttatgaatgt gctgtagaca actatacaag tttcataggc    900
   ttcaagtttc ggaagtttat agaaccacaa ctagatgaag atctcacaat atatatgaaa    960
   gacaaagcac tatcccccag gaaggaggca tgggactctg tatacccgga tagtaatctg   1020
   tactataaag ccccagaatc tgaagagacc cggcggctta ttgaagtgtt cataaatgat   1080
   gagaatttca acccagaaga aattatcaat tatgtggagt caggagattg gttgaaagac   1140
   gagaagttca acatctcgta cagtctcaaa gagaaagaga tcaagcaaga gggtcgtcta   1200
   ttcgcaaaaa tgacttataa gatgcgagcc gtacaggtgc tggcagagac actactggct 1260
   aaaggaatag gagagctgtt cagcgaaaat gggatggtta aaggagagat agacctactt 1320
   aaaagattga ctactctttc tgtctcagga gtccccagga ctgattcagt gtacaataac 1380
   tctaaatcat cagagaagag aaacgaaggc atgaaaaaga agaactctgg ggggtactgg 1440
   gacgaaaaga agaggtccag acatgaattc aaggcaacag attcatcaac agacggctat 1500
   gaaacgttaa gttgcttcct cacaacagac ctcaagaaat actgcttaaa ctggagattt 1560
   gaaagtactg cattgtttgg tcagagatgc aacgagatat ttggcttcaa gaccttcttt 1620
   aactggatgc atccagtcct tgaaaggtgt acaatatatg ttggggatcc ttactgtcca 1680
   gtcgccgacc ggatgcatcg acaactccag gatcatgcag actctggcat tttcatacat 1740
   aatcctaggg ggggcataga aggttactgc cagaagctgt ggaccttaat ctcaatcagt 1800
   gcaatccacc tagcagctgt gagagtgggt gtcagggtct ctgcaatggt tcagggtgac 1860
   aatcaagcta tagccgtgac atcaagagta cctgtagctc agacttacaa gcagaagaaa 1920
   aatcatgtct ataaggagat caccaaatat tttggtgctc taagacacgt catgtttgat 1980
   gtagggcacg agctaaaatt gaacgagacc atcattagta gcaagatgtt tgtctatagt 2040
   aaaagaatat actatgatgg gaagatttta ccacagtgcc tgaaagcctt gaccaggtgt 2100
   gtattctggt ccgagacact ggtagatgaa aacagatctg cttgttcgaa catctcaaca 2160
   tccatagcaa aagctatcga aaatgggtat tctcctatac taggctactg cattgcgttg 2220
   tataagacct gtcagcaggt gtgcatatca ctagggatga ctataaatcc aactatcagc 2280
   ccgaccgtaa gagatcaata ctttaagggt aagaattggc tgagatgtgc agtgttgatt 2340
   ccagcaaatg ttggaggatt caactacatg tctacatcta gatgctttgt tagaaatatt 2400
   ggagaccccg cagtagcagc cctagctgat ctcaaaagat tcatcagagc ggatctgtta 2460
   gacaagcagg tactatacag ggtcatgaat caagaacccg gtgactctag ctttctagat 2520
   tgggcttcag acccttattc atgtaacctc ccgcattctc agagtataac tacgattata 2580
   aagaatatca ctgctagatc tgtgctgcag gaatccccga atcctctact gtctggtctc 2640
   ttcaccgaga ctagtggaga agaggatctc aacctggcct cgttccttat ggaccggaaa 2700
   gtcatcctgc cgagagtggc tcatgagatc ctgggtaatt ccttaactgg agttagggag 2760
   gcgattgcag ggatgcttga tacgaccaag tctctagtga gatccagcgt taagaaagga 2820
   ggattatcat atgggatatt gaggaggctt gtcaattatg atctattgca gtacgagaca 2880
   ctgactagaa ctctcaggaa accggtgaaa gacaacatcg aatatgagta tatgtgttca 2940
   gttgagctag ctgtcggtct aaggcagaaa atgtggatcc acctaactta cgggagaccc 3000
   atacatgggc tagaaacacc agacccttta gagctcttga ggggaacatt tatcgaaggt 3060
   tcagaggtgt gcaagctttg caggtctgag ggagcagacc ccatctatac atggttctat 3120
   ctccctgaca atatagacct ggacacgctt acaaacggat gtccggctat aagaatcccc 3180
   tattttggat cagccactga tgaaaggtcg gaagcccaac tcgggtatgt aagaaatcta 3240
   agcaaacccg caaaggctgc catccggata gctatggtgt atacgtgggc ctacgggact 3300
   gatgagatat cgtggatgga agccgctctt atagcccaaa caagagctaa tctgagctta 3360
   gagaatctaa agctgctgac tcctgtttca acctccacta atctatctca taggttgaaa 3420
   gatacggcaa cccagatgaa gttctctagt gcaacactag tccgtgcaag tcggttcata 3480
   acaatatcaa atgataacat ggcactcaaa gaagcagggg agtcgaagga tactaatctc 3540
   gtgtatcagc agattatgct aactgggcta agcttgttcg agttcaatat gagatataag 3600
   aaaggttcct tagggaagcc actgatattg cacttacatc ttaataacgg gtgctgtata 3660
   atggagtccc cacaggaggc gaatatcccc ccaaggtcca cattagattt agagattaca 3720
   caagagaaca ataaattgat ctatgatcct gatccactca aggatgtgga ccttgagcta 3780
   tttagcaagg tcagagatgt tgtacataca gttgacatga cttattggtc agatgatgaa 3840
   gttatcagag caaccagtat ctgtactgca atgacgatag ctgatacaat gtctcaatta 3900
   gatagagaca acctaaaaga gatgatcgcg ctagtaaatg acgatgatgt caacagcctg 3960
   attactgagt ttatggtgat tgatgttcct ttattttgct caacgttcgg gggtattcta 4020
   gtcaatcagt ttgcatactc actctacggc ttaaacatca gaggaaggga agaaatatgg 4080
   ggacatgtag tccggattct taaagatacc tcccacgcag ttctaaaagt cttatctaat 4140
   gctctatctc atcccaaaat cttcaaacga ttctggaatg caggtgtcgt ggaacctgtg 4200
   tatgggccta acctctcaaa tcaggacaag atactcttgg ccctctctgt ctgtgaatat 4260
   tctgtggatc tattcatgca cgattggcaa gggggtgtac cgcttgagat ctttatctgt 4320
   gacaatgacc cagatgtggc cgacatgagg aggtcctctt tcttggcaag acatcttgca 4380
   tacctatgca gcgtggcaga gatatctagg gatgggccaa gattagaatc aatgaactct 4440
   ctagagaggc tcgagtcact aaagagttac ctggaactca catttcttga tgacccggta 4500
   ctgaggtaca gtcagttgac tggcctagtc atcaaagtat tcccatctac tttgacctat 4560
   atccggaagt catctataaa agtgttaagg acaagaggta taggagtccc tgaagtctta 4620
   gaagattggg atcccgaggc agataatgca ctgttagatg gtatcgcggc agaaatacaa 4680
   cagaatattc ctttgggaca tcagactaga gccccttttt gggggttgag agtatccaag 4740
   tcacaggtac tgcgtctccg gggtacaagg gagatcacaa gaggtgagat aggcagatca 4800
   ggcgttggtc tgacgttacc attcgatgga agatatctat ctcaccagct gaggctcttt 4860
   ggcatcaaca gtactagctg cttgaaagca cttgaactta cctacctatt gagcccctta 4920
   gttgacaagg ataaagatag gctatattta ggggaaggag ctggggccat gctttcctgt 4980
   tatgacgcta ctcttggccc atgcatcaac tattataact caggggtata ctcttgtgat 5040
   gtcaatgggc agagagagtt aaatatatat cctgctgagg tggcactggt gggaaagaaa 5100
   ttaaacaatg ttactagtct gggtcaaaga gttaaagtgt tattcaacgg gaatcctggc   5160
   tcgacatgga ttggaaatga tgagtgtgag gctttgattt ggaatgaatt gcagaatagc   5220
   tcgataggcc tagtccactg tgacatggag ggaggagatc ataaggatga tcaagttgta   5280
   ctgcatgagc attacagtgt aatccggatc gcgtatctgg tgggggatcg agacgttgtg   5340
   cttataagca agattgctcc taggctgggc acggattgga ccaggcagct cagcctatat   5400
   ctgagatact gggacgaggt taacctaata gtgcttaaaa catctaaccc tgcttccaca   5460
   gagatgtatc tcctatcgag gcatcccaaa tctgacatta tagaggacag caagacggtg   5520
   ttagctagtc tcctcccttt gtcaaaagaa gatagcatca agatagaaaa gtggatctta   5580
   atagagaagg caaaggctca cgaatgggtt actcgggaat tgagagaagg aagctcttca   5640
   tcagggatgc ttagacctta ccatcaagca ctgcagacgt ttggctttga accaaacttg   5700
   tataaattga gcagagattt cttgtccacc atgaacatag ctgatacaca caactgcatg   5760
   atagctttca acagggtttt gaaggataca atcttcgaat gggctagaat aactgagtca   5820
   gataaaaggc ttaaactaac tggtaagtat gacctgtatc ctgtgagaga ttcaggcaaa   5880
   ttgaagacag tttctagaag acttgtgcta tcttggatat ctttatctat gtccacaaga   5940
   ttggtaactg ggtcattccc tgaccagaag tttgaagcaa gacttcaatt gggaatagtt   6000
   tcattatcat cccgtgaaat caggaacctg agggttatca caaaaacttt attagaccgg   6060
   tttgaggata ttatacatag tataacgtac agattcctca ccaaagaaat aaagattttg   6120
   atgaagattt taggggcagt caagatgttc ggggccaggc aaaatgaata cacgaccgtg   6180
   attgatgatg gatcactggg tgatatcgag ccatatgaca gctcgtaata attagtccct   6240
   atcgtgcaga acgatcgaag ctccgcggta cctggaagtc ttggactgat ccatatgaca   6300
   atagtaagaa aaacttacaa gaagacaaga aaatttaaaa gaatacatat ctcttaaact   6360
   cttgtctggt gtcctgtttc gaaaacgaaa cagagaacca gtaccagaga aacacacgtt   6420
   gtggtatatt acctggttct cgagcaccac caccaccacc actgagatcc ggctgctaac   6480
   aaagcccgaa aggaagctga gttggctgct gccaccgctg agcaataact agcataaccc   6540
   cttggggcct ctaaacgggt cttgaggggt tttttg                             6576
<210> 44
   <211> 31
   <212> DNA
   <213> Artificial
<220>
   <223> Primer
<400> 44
   gacagctcgt aatcccgggt ccctatcgtg c 31
<210> 45
   <211> 31
   <212> DNA
   <213> Artificial
<220>
   <223> Primer
<400> 45
   gcacgatagg gacccgggat tacgagctgt c 31
<210> 46
   <211> 15810
   <212> DNA
   <213> Artificial
<220>
   <223> Full length genome cDNA of Sendai virus vector-version3
<400> 46
   accaaacaag agaagaaaca tgtatggaat atataatgaa gtttaagaaa aacttagggt      60
   caaagtatcc accctgagga gcaggttcca gatccttttc tttgctgcca aagttcacga     120
   tggccgggtt gttgagcacc ttcgatacat ttagctctag gaggagcgaa agtattaata     180
   agtcgggagg aggtgctgtt atccccggcc agaggagcac agtctcagtg ttcatactag     240
   gcccaagtgt gactgatgat gcagacaagt tattcattgc aacaaccttc ctagctcact     300
   cattggacac agataagcag cactctcaga gaggagggtt cctcgtctct ctgcttgcca     360
   tggcttacag tagtccagaa ttgtacttga caacaaacgg agtaaacgcc gatgtcaaat     420
   atgtgatcta caacatagag aaagacccta agaggacgaa gacagacgga ttcattgtga     480
   agacgagaga tatggaatat gagaggacca cagaatggct gtttggacct atggtcaaca     540
   agagcccact cttccagggt caacgggatg ctgcagaccc tgacacactc cttcaaatct     600
   atgggtatcc tgcatgccta ggagcaataa ttgtccaagt ctggattgtg ctggtgaagg     660
   ccatcacaag cagcgccggc ttaaggaaag ggttcttcaa caggttagag gcgttcagac     720
   aagacggcac cgtgaaaggt gccttagttt tcactgggga gacagttgag gggataggct     780
   cggttatgag atctcagcaa agccttgtat ctctcatggt tgagaccctt gtgactatga     840
   atactgcaag atctgatctc accacattag agaagaacat ccagatcgtt gggaactaca     900
   tccgagatgc agggctggct tccttcatga acactattaa atatggggtg gagacaaaga     960
   tggcagctct aacgttgtca aacctgaggc ccgatattaa taagcttaga agcctcatag    1020
   acacctacct gtcaaaaggc cccagagctc cctttatctg tatcctcaag gaccctgttc    1080
   atggtgaatt tgctccaggc aattatcctg cactatggag ttacgccatg ggagtcgccg    1140
   tcgtacagaa caagtcaatg cagcagtacg tcacagggag gacatacctt gatatggaaa    1200
   tgttcttact aggacaagcc gtggcaaagg atgctgaatc gaagatcagc agtgccttgg    1260
   aagatgagtt aggagtgacg gatacagcca aggagaggct cagacatcat ctggcaaact    1320
   tgtccggtgg ggatggtgct taccacaaac caacaggcgg tggtgcaatt gaggtagctc    1380
   tagacaatgc cgatatcgac ctggaaacag aagctcatgc ggaccaggac gctaggggtt 1440
   ggggtggaga tagtggtgaa agatgggcac gtcaggtgag tggtggccac tttgtcacac 1500
   tacatggggc tgaacggtta gaggaggaaa ccaatgatga ggatgtatca gacatagaga 1560
   gaagaatagc catgagactc gcagagagac ggcaagagga ttctgcaacc catggagatg 1620
   aaggccgcaa taacggtgtt gatcacgaag aagatgacga tgccgcagca gcagctggga 1680
   taggaggaat ctaggatcat acgaggcctc aaggtacttg atccgcagta agaaaaactt 1740
   agggtgaaag ttcatccacc gatcggctca ggcaaggcca cacccaaccc caccgaccac 1800
   acccagcagt cgagacagcc acggcttcgg ctacacttac cgcatggatc aagatgcctt 1860
   cattcttaaa gaagattctg aagttgagag gaaggcgcca ggaggacgag agtcgctctc 1920
   ggatgttatc ggattcctcg atgctgtcct gtcgaatgaa ccaactgaca tcggagggga 1980
   cagaagctgg ctccacaaca ccatcaacac tccccaagga ccaggctctg ctcatagagc 2040
   caaaagtgag ggcgaaggag aagtctcaac accgtcgacc caagataatc gatcaggtga 2100
   ggagagtaga gtctctggga gaacaagcaa gccagaggca gaagcacatg ctggaaacct 2160
   tgataaacaa aatatacact gggcctttag gggaagaact ggtacaaact ctgtatctca 2220
   ggatctggac gatggaggag actccggaat ccttgaaaat cctccaaatg agagaggata 2280
   tccgagatca ggtattgaag atgaaaacag agagatggct gcgcaccctg ataagagggg 2340
   agaagaccaa gctgaaggac ttccagaaga ggtacgagga ggtacatccc tacctgatga 2400
   aggagaaggt ggagcaagta ataatggaag aagcatggag cctggcagct cacatagtgc 2460
   aagagtaact ggggtcctgg tgattcctag ccccgaactt gaagaggctg tgctacggag 2520
   gaacaaaaga agacctacca acagtgggtc caaacctctt actccagcaa ccgtgcctgg 2580
   cacccggtcc ccaccgctga atcgttacaa cagcacaggg tcaccaccag gaaaaccccc 2640
   atctacacag gatgagcaca tcaactctgg ggacaccccc gccgtcaggg tcaaagaccg 2700
   gaaaccatca atagggactc gctctgtctc agattgtcca gccaacggcc gcccaatcca 2760
   tccgggtata gagaccgact caacaaaaaa gggcatagga gagaacacat catctatgaa 2820
   agatatggct acattgttga cgagtcttgg tgtaatccag tctgctcaag aattcgagtc 2880
   atcccgagac gcgagttatg tgtttgcaag acgtgcccta aagtctgcaa actatgcaga 2940
   gatgacattc aatgtatgcg gcctgatcct ttctgccgag aaatcttccg ctcgtaaggt 3000
   agatgagaac aaacaactgc tcaaacagat ccaagagagc gtggaatcat tccgggatat 3060
   ttacaagaga ttctctgagt atcagaaaga acagaactca ttgctgatgt ccaacctatc 3120
   tacacttcat atcatcacag atagaggtgg caagactgac aacacagact cccttacaag 3180
   gtccccctcc gtttttgcaa aatcaaaaga gaacaagact aaggctacca ggtttgaccc 3240
   atctatggag accttagaag atatgaagta caaaccggac ctaatccgag aggatgaatt 3300
   tagagatgag atccgcaacc cggtgtacca agagagggac acagaaccca gggcctcaaa 3360
   cgcatcacgt ctcttcccct ccaaagagaa gcccacaatg cactctctca ggctcgtcat 3420
   agagagcagt cccctaagca gagctgagaa agcagcatat gtgaaatcat tatccaagtg 3480
   caagacagac caagaggtta aggcagtcat ggaactcgta gaagaggaca tagagtcact 3540
   gaccaactag atcccgggtg aggcatccca ccatcctcag tcacagagag acccaatcta 3600
   ccatcagcat cagccagtaa agattaagaa aaacttaggg tgaaagaaat ttcaccgcta 3660
   gcttagacga tggatttttt tcgggtagtg gaaaaccagc agcctcccgc gacgatgccc 3720
   ctcaacgtta gcttcaccaa caggaactat gacctcgact acgactcggt gcagccgtat 3780
   ttctactgcg acgaggagga gaacttctac cagcagcagc agcagagcga gctgcagccc 3840
   ccggcgccca gcgaggatat ctggaagaaa ttcgagctgc tgcccacccc gcccctgtcc 3900
   cctagccgcc gctccgggct ctgctcgccc tcctacgttg cggtcacacc cttctccctt 3960
   cggggagaca acgacggcgg tggcgggagc ttctccacgg ccgaccagct ggagatggtg 4020
   accgagctgc tgggaggaga catggtgaac cagagtttca tctgcgaccc ggacgacgag 4080
   accttcatca aaaacatcat catccaggac tgtatgtgga gcggcttctc ggccgccgcc 4140
   aagctcgtct cagagaagct ggcctcctac caggctgcgc gcaaagacag cggcagcccg 4200
   aaccccgccc gcggccacag cgtctgctcc acctccagct tgtacctgca ggatctgagc 4260
   gccgccgcct cagagtgcat cgacccctcg gtggtcttcc cctaccctct caacgacagc 4320
   agctcgccca agtcctgcgc ctcgcaagac tccagcgcct tctctccgtc ctcggattct 4380
   ctgctctcct cgacggagtc ctccccgcag ggcagccccg agcccctggt gctccatgag 4440
   gagacaccgc ccaccaccag cagcgactct gaggaggaac aagaagatga ggaagaaatc 4500
   gatgttgttt ctgtggaaaa gaggcaggct cctggcaaaa ggtcagagtc tggatcacct 4560
   tctgctggag gccacagcaa acctcctcac agcccactgg tcctcaagag gtgccacgtc 4620
   tccacacatc agcacaacta cgcagcgcct ccctccactc ggaaggacta tcctgctgcc 4680
   aagagggtca agttggacag tgtcagagtc ctgagacaga tcagcaacaa ccgaaaatgc 4740
   accagcccca ggtcctcgga caccgaggag aatgtcaaga ggcgaacaca caacgtcttg 4800
   gagcgccaga ggaggaacga gctaaaacgg agcttttttg ccctgcgtga ccagatcccg 4860
   gagttggaaa acaatgaaaa ggcccccaag gtagttatcc ttaaaaaagc cacagcatac 4920
   atcctgtccg tccaagcaga ggagcaaaag ctcatttctg aagaggactt gttgcggaaa 4980
   cgacgagaac agttgaaaca caaacttgaa cagctacgga actcttgtgc gtaaggacgt 5040
   cgattaagaa aaacttaggg tgaaagttca tcgcggccgc ttgctagcag tctgacatgg 5100
   ctgtcagcga cgcgctgctc ccatctttct ccacgttcgc gtctggcccg gcgggaaggg 5160
   agaagacact gcgtcaagca ggtgccccga ataaccgctg gcgggaggag ctctcccaca 5220
   tgaagcgact tcccccagtg cttcccggcc gcccctatga cctggcggcg gcgaccgtgg 5280
   ccacagacct ggagagcggc ggagccggtg cggcttgcgg cggtagcaac ctggcgcccc 5340
   tacctcggag agagaccgag gagttcaacg atctcctgga cctggacttt attctctcca 5400
   attcgctgac ccatcctccg gagtcagtgg ccgccaccgt gtcctcgtca gcgtcagcct 5460
   cctcttcgtc gtcgccgtcg agcagcggcc ctgccagcgc gccctccacc tgcagcttca 5520
   cctatccgat ccgggccggg aacgacccgg gcgtggcgcc gggcggcacg ggcggaggcc 5580
   tcctctatgg cagggagtcc gctccccctc cgacggctcc cttcaacctg gcggacatca 5640
   acgacgtgag cccctcgggc ggcttcgtgg ccgagctcct gcggccagaa ttggacccgg 5700
   tgtacattcc gccgcagcag ccgcagccgc caggtggcgg gctgatgggc aagttcgtgc 5760
   tgaaggcgtc gctgagcgcc cctggcagcg agtacggcag cccgtcggtc atcagcgtca 5820
   gcaaaggcag ccctgacggc agccacccgg tggtggtggc gccctacaac ggcgggccgc 5880
   cgcgcacgtg ccccaagatc aagcaggagg cggtctcttc gtgcacccac ttgggcgctg 5940
   gaccccctct cagcaatggc caccggccgg ctgcacacga cttccccctg gggcggcagc 6000
   tccccagcag gactaccccg accctgggtc ttgaggaagt gctgagcagc agggactgtc 6060
   accctgccct gccgcttcct cccggcttcc atccccaccc ggggcccaat tacccatcct 6120
   tcctgcccga tcagatgcag ccgcaagtcc cgccgctcca ttaccaagag ctcatgccac 6180
   ccggttcctg catgccagag gagcccaagc caaagagggg aagacgatcg tggccccgga 6240
   aaaggaccgc cacccacact tgtgattacg cgggctgcgg caaaacctac acaaagagtt 6300
   cccatctcaa ggcacacctg cgaacccaca caggtgagaa accttaccac tgtgactggg 6360
   acggctgtgg atggaaattc gcccgctcag atgaactgac caggcactac cgtaaacaca 6420
   cggggcaccg cccgttccag tgccaaaaat gcgaccgagc attttccagg tcggaccacc 6480
   tcgccttaca catgaagagg catttttaaa cgcgtcagag acctgcaaca atgtctcaag 6540
   cagacaccac ctggcagtcg gagccaccgg gtcactcctt gtcttaaata agaaaaactt 6600
   agggataaag tcccttagat ctagcctagg ttccccatgg cgggacacct ggcttcggat 6660
   ttcgccttct cgccccctcc aggtggtgga ggtgatgggc caggggggcc ggagccgggc 6720
   tgggttgatc ctcggacctg gctaagcttc caaggccctc ctggagggcc aggaatcggg 6780
   ccgggggttg ggccaggctc tgaggtgtgg gggattcccc catgcccccc gccgtatgag 6840
   ttctgtgggg ggatggcgta ctgtgggccc caggttggag tggggctagt gccccaaggc 6900
   ggcttggaga cctctcagcc tgagggcgaa gcaggagtcg ggtggagagg caactccgat 6960
   ggggcctccc cggagccctg caccgtcacc cctggtgccg tgaagctgga gaaggagaag 7020
   ctggagcaaa acccggagga gtcccaggac atcaaagctc tgcagaaaga actcgagcaa 7080
   tttgccaagc tcctgaagca gaagaggatc accctgggat atacacaggc cgatgtgggg 7140
   ctcaccctgg gggttctatt tgggaaggta ttcagccaaa cgaccatctg ccgctttgag 7200
   gctctgcagc ttagcttcaa gaacatgtgt aagctgcggc ccttgctgca gaagtgggtg 7260
   gaggaagctg acaacaatga aaatcttcag gagatatgca aagcagaaac cctcgtgcag 7320
   gcccgaaaga gaaagcgaac cagtatcgag aaccgagtga gaggcaacct ggagaatttg 7380
   ttcctgcagt gcccgaaacc cacactgcag cagatcagcc acatcgccca gcagcttggg 7440
   ctcgagaagg atgtggtccg agtgtggttc tgtaaccggc gccagaaggg caagcgatca 7500
   agcagcgact atgcacaacg agaggatttt gaggctgctg ggtctccttt ctcaggggga 7560
   ccagtgtcct ttcctctggc cccagggccc cattttggta ccccaggcta tgggagccct 7620
   cacttcactg cactgtactc ctcggtccct ttccctgagg gggaagcctt tccccctgtc 7680
   tccgtcacca ctctgggctc tcccatgcat tcaaactgag gacgtcagat ctgtatataa 7740
   taagaaaaac ttagggtgaa agtgaggttg cgcggtattt tagctagccc gcatgtacaa 7800
   catgatggag acggagctga agccgccggg cccgcagcaa acttcggggg gcggcggcgg 7860
   caactccacc gcggcggcgg ccggcggcaa ccagaaaaac agcccggacc gcgtcaagcg 7920
   gcccatgaat gccttcatgg tgtggtcccg cgggcagcgg cgcaagatgg cccaggagaa 7980
   ccccaagatg cacaactcgg agatcagcaa gcgcctgggc gccgagtgga aacttttgtc 8040
   ggagacggag aagcggccgt tcatcgacga ggctaagcgg ctgcgagcgc tgcacatgaa 8100
   ggagcacccg gattataaat accggccccg gcggaaaacc aagacgctca tgaagaagga 8160
   taagtacacg ctgcccggcg ggctgctggc ccccggcggc aatagcatgg cgagcggggt 8220
   cggggtgggc gccggcctgg gcgcgggcgt gaaccagcgc atggacagtt acgcgcacat 8280
   gaacggctgg agcaacggca gctacagcat gatgcaggac cagctgggct acccgcagca 8340
   cccgggcctc aatgcgcacg gcgcagcgca gatgcagccc atgcaccgct acgacgtgag 8400
   cgccctgcag tacaactcca tgaccagctc gcagacctac atgaacggct cgcccaccta 8460
   cagcatgtcc tactcgcagc agggcacccc tggcatggct cttggctcca tgggttcggt 8520
   ggtcaagtcc gaggccagct ccagcccccc tgtggttacc tcttcctccc actccagggc 8580
   gccctgccag gccggggacc tccgggacat gatcagcatg tatctccccg gcgccgaggt 8640
   gccggaaccc gccgccccca gcagacttca catgtcccag cactaccaga gcggcccggt 8700
   gcccggcacg gccattaacg gcacactgcc cctctcacac atgtgagacc ggtgtcggct 8760
   ttgctgacac tagagtcatc tccgaacatc cacaatatct ctcagtctct tacgtctctc 8820
   acagtattaa gaaaaaccca gggtgaatgg gaagcttgcc ataggtcatg gatgggcagg 8880
   agtcctccca aaacccttct gacatactct atccagaatg ccacctgaac tctcccatag 8940
   tcagggggaa gatagcacag ttgcacgtct tgttagatgt gaaccagccc tacagactaa 9000
   aggacgacag cataataaat attacaaagc acaaaattag gaacggagga ttgtcccctc 9060
   gtcaaattaa gatcaggtct ctgggtaagg ctcttcaacg cacaataaag gatttagacc 9120
   gatacacctt tgaaccgtac ccaacctact ctcaggaatt acttaggctt gatataccag 9180
   agatatgtga caaaatccga tccgtcttcg cggtctcgga tcggctgacc agggagttat 9240
   ctagtgggtt ccaggatctt tggttgaata tcttcaagca actaggcaat atagaaggaa 9300
   gagaggggta cgatccgttg caggatatcg gcaccatccc ggagataact gataaataca 9360
   gcaggaatag atggtatagg ccattcctaa cttggttcag catcaaatat gacatgcggt 9420
   ggatgcagaa gaccagaccg gggggacccc tcgatacctc taattcacat aacctcctag 9480
   aatgcaaatc atacactcta gtaacatacg gagatcttat catgatactg aacaagttga 9540
   cattgacagg gtatatccta acccctgagc tggtcttgat gtattgtgat gttgtagagg 9600
   gaaggtggaa tatgtctgct gcagggcatc tagataagaa gtccattggg ataacaagca 9660
   aaggtgagga attatgggaa ctagtggatt ccctcttctc aagtcttgga gaggaaatat 9720
   acaatgtcat cgcactattg gagcccctat cacttgctct catacaacta aatgatccag 9780
   ttatacctct acgtggggca tttatgaggc atgtgttgac agagctacag gctgttttaa 9840
   caagtaggga cgtgtacaca gatgctgaag cagacactat tgtggagtcg ttactcgcca 9900
   ttttccatgg aacctctatt gatgagaaag cagagatctt ttccttcttt aggacatttg 9960
   gccaccccag cttagaggct gtcactgccg ccgacaaggt aagggcccat atgtatgcac 10020
   aaaaggcaat aaagcttaag accctatacg agtgtcatgc agttttttgc actatcatca 10080
   taaatgggta tagagagagg catggcggac agtggccccc ctgtgacttc cctgatcacg 10140
   tgtgtctaga actaaggaac gctcaagggt ccaatacggc aatctcttat gaatgtgctg 10200
   tagacaacta tacaagtttc ataggcttca agtttcggaa gtttatagaa ccacaactag 10260
   atgaagatct cacaatatat atgaaagaca aagcactatc ccccaggaag gaggcatggg 10320
   actctgtata cccggatagt aatctgtact ataaagcccc agaatctgaa gagacccggc 10380
   ggcttattga agtgttcata aatgatgaga atttcaaccc agaagaaatt atcaattatg 10440
   tggagtcagg agattggttg aaagacgaga agttcaacat ctcgtacagt ctcaaagaga 10500
   aagagatcaa gcaagagggt cgtctattcg caaaaatgac ttataagatg cgagccgtac 10560
   aggtgctggc agagacacta ctggctaaag gaataggaga gctgttcagc gaaaatggga 10620
   tggttaaagg agagatagac ctacttaaaa gattgactac tctttctgtc tcaggagtcc 10680
   ccaggactga ttcagtgtac aataactcta aatcatcaga gaagagaaac gaaggcatga 10740
   aaaagaagaa ctctgggggg tactgggacg aaaagaagag gtccagacat gaattcaagg 10800
   caacagattc atcaacagac ggctatgaaa cgttaagttg cttcctcaca acagacctca 10860
   agaaatactg cttaaactgg agatttgaaa gtactgcatt gtttggtcag agatgcaacg 10920
   agatatttgg cttcaagacc ttctttaact ggatgcatcc agtccttgaa aggtgtacaa 10980
   tatatgttgg ggatccttac tgtccagtcg ccgaccggat gcatcgacaa ctccaggatc 11040
   atgcagactc tggcattttc atacataatc ctaggggggg catagaaggt tactgccaga 11100
   agctgtggac cttaatctca atcagtgcaa tccacctagc agctgtgaga gtgggtgtca 11160
   gggtctctgc aatggttcag ggtgacaatc aagctatagc cgtgacatca agagtacctg 11220
   tagctcagac ttacaagcag aagaaaaatc atgtctataa ggagatcacc aaatattttg 11280
   gtgctctaag acacgtcatg tttgatgtag ggcacgagct aaaattgaac gagaccatca 11340
   ttagtagcaa gatgtttgtc tatagtaaaa gaatatacta tgatgggaag attttaccac 11400
   agtgcctgaa agccttgacc aggtgtgtat tctggtccga gacactggta gatgaaaaca 11460
   gatctgcttg ttcgaacatc tcaacatcca tagcaaaagc tatcgaaaat gggtattctc 11520
   ctatactagg ctactgcatt gcgttgtata agacctgtca gcaggtgtgc atatcactag 11580
   ggatgactat aaatccaact atcagcccga ccgtaagaga tcaatacttt aagggtaaga 11640
   attggctgag atgtgcagtg ttgattccag caaatgttgg aggattcaac tacatgtcta 11700
   catctagatg ctttgttaga aatattggag accccgcagt agcagcccta gctgatctca 11760
   aaagattcat cagagcggat ctgttagaca agcaggtact atacagggtc atgaatcaag 11820
   aacccggtga ctctagcttt ctagattggg cttcagaccc ttattcatgt aacctcccgc 11880
   attctcagag tataactacg attataaaga atatcactgc tagatctgtg ctgcaggaat 11940
   ccccgaatcc tctactgtct ggtctcttca ccgagactag tggagaagag gatctcaacc 12000
   tggcctcgtt ccttatggac cggaaagtca tcctgccgag agtggctcat gagatcctgg 12060
   gtaattcctt aactggagtt agggaggcga ttgcagggat gcttgatacg accaagtctc 12120
   tagtgagatc cagcgttaag aaaggaggat tatcatatgg gatattgagg aggcttgtca 12180
   attatgatct attgcagtac gagacactga ctagaactct caggaaaccg gtgaaagaca 12240
   acatcgaata tgagtatatg tgttcagttg agctagctgt cggtctaagg cagaaaatgt 12300
   ggatccacct aacttacggg agacccatac atgggctaga aacaccagac cctttagagc 12360
   tcttgagggg aacatttatc gaaggttcag aggtgtgcaa gctttgcagg tctgagggag 12420
   cagaccccat ctatacatgg ttctatctcc ctgacaatat agacctggac acgcttacaa 12480
   acggatgtcc ggctataaga atcccctatt ttggatcagc cactgatgaa aggtcggaag 12540
   cccaactcgg gtatgtaaga aatctaagca aacccgcaaa ggctgccatc cggatagcta 12600
   tggtgtatac gtgggcctac gggactgatg agatatcgtg gatggaagcc gctcttatag 12660
   cccaaacaag agctaatctg agcttagaga atctaaagct gctgactcct gtttcaacct 12720
   ccactaatct atctcatagg ttgaaagata cggcaaccca gatgaagttc tctagtgcaa 12780
   cactagtccg tgcaagtcgg ttcataacaa tatcaaatga taacatggca ctcaaagaag 12840
   caggggagtc gaaggatact aatctcgtgt atcagcagat tatgctaact gggctaagct 12900
   tgttcgagtt caatatgaga tataagaaag gttccttagg gaagccactg atattgcact 12960
   tacatcttaa taacgggtgc tgtataatgg agtccccaca ggaggcgaat atccccccaa 13020
   ggtccacatt agatttagag attacacaag agaacaataa attgatctat gatcctgatc 13080
   cactcaagga tgtggacctt gagctattta gcaaggtcag agatgttgta catacagttg 13140
   acatgactta ttggtcagat gatgaagtta tcagagcaac cagtatctgt actgcaatga 13200
   cgatagctga tacaatgtct caattagata gagacaacct aaaagagatg atcgcgctag 13260
   taaatgacga tgatgtcaac agcctgatta ctgagtttat ggtgattgat gttcctttat 13320
   tttgctcaac gttcgggggt attctagtca atcagtttgc atactcactc tacggcttaa 13380
   acatcagagg aagggaagaa atatggggac atgtagtccg gattcttaaa gatacctccc 13440
   acgcagttct aaaagtctta tctaatgctc tatctcatcc caaaatcttc aaacgattct 13500
   ggaatgcagg tgtcgtggaa cctgtgtatg ggcctaacct ctcaaatcag gacaagatac 13560
   tcttggccct ctctgtctgt gaatattctg tggatctatt catgcacgat tggcaagggg 13620
   gtgtaccgct tgagatcttt atctgtgaca atgacccaga tgtggccgac atgaggaggt 13680
   cctctttctt ggcaagacat cttgcatacc tatgcagcgt ggcagagata tctagggatg 13740
   ggccaagatt agaatcaatg aactctctag agaggctcga gtcactaaag agttacctgg 13800
   aactcacatt tcttgatgac ccggtactga ggtacagtca gttgactggc ctagtcatca 13860
   aagtattccc atctactttg acctatatcc ggaagtcatc tataaaagtg ttaaggacaa 13920
   gaggtatagg agtccctgaa gtcttagaag attgggatcc cgaggcagat aatgcactgt 13980
   tagatggtat cgcggcagaa atacaacaga atattccttt gggacatcag actagagccc 14040
   ctttttgggg gttgagagta tccaagtcac aggtactgcg tctccggggg tacaaggaga 14100
   tcacaagagg tgagataggc agatcaggcg ttggtctgac gttaccattc gatggaagat 14160
   atctatctca ccagctgagg ctctttggca tcaacagtac tagctgcttg aaagcacttg 14220
   aacttaccta cctattgagc cccttagttg acaaggataa agataggcta tatttagggg 14280
   aaggagctgg ggccatgctt tcctgttatg acgctactct tggcccatgc atcaactatt 14340
   ataactcagg ggtatactct tgtgatgtca atgggcagag agagttaaat atatatcctg 14400
   ctgaggtggc actggtggga aagaaattaa acaatgttac tagtctgggt caaagagtta 14460
   aagtgttatt caacgggaat cctggctcga catggattgg aaatgatgag tgtgaggctt 14520
   tgatttggaa tgaattgcag aatagctcga taggcctagt ccactgtgac atggagggag 14580
   gagatcataa ggatgatcaa gttgtactgc atgagcatta cagtgtaatc cggatcgcgt 14640
   atctggtggg ggatcgagac gttgtgctta taagcaagat tgctcctagg ctgggcacgg 14700
   attggaccag gcagctcagc ctatatctga gatactggga cgaggttaac ctaatagtgc 14760
   ttaaaacatc taaccctgct tccacagaga tgtatctcct atcgaggcat cccaaatctg 14820
   acattataga ggacagcaag acggtgttag ctagtctcct ccctttgtca aaagaagata 14880
   gcatcaagat agaaaagtgg atcttaatag agaaggcaaa ggctcacgaa tgggttactc 14940
   gggaattgag agaaggaagc tcttcatcag ggatgcttag accttaccat caagcactgc 15000
   agacgtttgg ctttgaacca aacttgtata aattgagcag agatttcttg tccaccatga  15060
   acatagctga tacacacaac tgcatgatag ctttcaacag ggttttgaag gatacaatct  15120
   tcgaatgggc tagaataact gagtcagata aaaggcttaa actaactggt aagtatgacc  15180
   tgtatcctgt gagagattca ggcaaattga agacagtttc tagaagactt gtgctatctt  15240
   ggatatcttt atctatgtcc acaagattgg taactgggtc attccctgac cagaagtttg  15300
   aagcaagact tcaattggga atagtttcat tatcatcccg tgaaatcagg aacctgaggg  15360
   ttatcacaaa aactttatta gaccggtttg aggatattat acatagtata acgtacagat  15420
   tcctcaccaa agaaataaag attttgatga agattttagg ggcagtcaag atgttcgggg  15480
   ccaggcaaaa tgaatacacg accgtgattg atgatggatc actgggtgat atcgagccat  15540
   atgacagctc gtaataccgg ttatcaccaa aacatggaag cacttacgat tcaccaaaac  15600
   atggaagcac ttaggtacct caccaaaaca tggaagcact tacgattcac caaaacatgg  15660
   aagcacttaa ccggttccct atcgtgcaga acgatcgaag ctccgcggta cctggaagtc  15720
   ttggactgat ccatatgaca atagtaagaa aaacttacaa gaagacaaga aaatttaaaa  15780
   gaatacatat ctcttaaact cttgtctggt                                  15810

## Description

### TECHNICAL FIELD

The present invention relates to a Sendai virus vector for generating pluripotent stem cells and a method of producing pluripotent stem cells using the Sendai virus vector.

### BACKGROUND ART

Along with the progression toward an aging society, diseases caused by tissue degeneration and damage are increasing rapidly. For example, the diseases include cerebral infarction, cardiac infarction and renal failure that increase in frequency with age due to metabolic syndromes, as well as Alzheimer's disease, Parkinson's disease and osteoporosis due to age-related tissue degeneration. In addition, type I diabetes, multiple sclerosis, chronic rheumatoid arthritis, thermal burn, spinal damage from injury, and genetic diseases caused by congenital abnormalities in the genetic code, are all diseases caused by tissue degeneration and damage. A number of regeneration therapies are being developed as a means for treating these diseases.

Regeneration therapies can be roughly classified into two groups: guided regeneration therapies designed to activate tissue stem cells residing in a patient's tissue in various ways; and cell replacement therapies designed to the transplant stem cells or stem cell-derived somatic cells or tissues. The regeneration potential of tissue stem cells is however often limited. Development of cell replacement therapies is therefore essential to the practical application of regeneration therapies. In particular, with regard to genetic diseases, it is conceivable to perform cell replacement therapies using cells which have been genetically repaired or replaced ex vivo.

A regeneration therapy-based treatment for diseases caused by tissue degeneration/damage requires the preparation of large amounts of stem cells or stem cell-induced somatic tissues. Thus, pluripotent stem cells capable of self-renewal over long periods of time while maintaining their differentiation potential into various tissue types are essential for the development of cell replacement therapies. To date only a few pluripotent stem cells have been reported that meet the requirement and include embryo-stem cells (ES cells) derived from early embryos, and EG cells derived from primordial germ cells. These cells cannot be directly used in cell replacement therapies however because a cell having genomic information non-identical to that of a patient's cell causes transplantation rejection.

Therefore, as a prerequisite to performing cell replacement therapies in a safe and efficient manner, pluripotent stem cells are required to have genomic information identical to that of a patient's cell so as to avoid immunological rejection after transplantation. It is conceivable to generate such pluripotent stem cells from a patient's tissue cell while changing a property thereof in some way. In this case, it is desirable to allow the tissue cell to be collected without employing a procedure causing strong stress on a human body, such as surgery. It is also desirable to allow the pluripotent stem cells to be quickly generated using a minimum number of tissue cells collectable at a time. As a cell material collectable without causing strong stress and capable of maintaining gene information identical to that of a patient's cell, it is conceivable to use skin fibroblast cells, oral mucosal cells or hair follicle epithelial cells. The number of cells collectable without strong stress on a human body is about 10⁴. Thus, it is desirable to allow the pluripotent stem cells to be established from 10⁴ normal tissue cells.

It is known from research on human ES cells that as pluripotent stem cells are continuously cultured over long periods of time, chromosomal abnormalities, such as chromosomal deletions, amplifications and translocations, will be observed more frequently. If established pluripotent stem cells are uniform in terms of a cell property, it is necessary to take time for selecting pluripotent stem cells having a required function from a large number of cell lines, so that chromosomal abnormalities are significantly more likely to occur in the course of the selection. Thus, a method for producing pluripotent stem cells is required to be capable of constantly produce pluripotent stem cells each having a uniform property, with excellent reproducibility. For example, an index representing uniformity in property of pluripotent stem cells includes a correlation coefficient between respective gene-expression patterns of two cell lines, wherein the correlation coefficient may be set to a value close to 1, preferably 0.98 or more.

Whether or not a new cell line established from patient's tissue cells is a pluripotent stem cell has to be verified based on an ability to differentiate into various tissues (pluripotency). As a method for verification of pluripotency, an in-vitro differentiation method and a differentiation method based on transplantation into an immunodeficient animal have been known. However, such phenomena are also observed in malignant teratocarcinomas cells. Thus, in view of a need for verifying that the established cell is a high-safety pluripotent stem cell usable as a material for regeneration therapies, the above methods are insufficient in themselves (Non-Patent Document 18).

As a technique for, in a laboratory animal such as a mouse, distinguishing between a high-safety pluripotent stem cell which is less likely to become a malignant tumor, and a teratocarcinoma which has differentiation capability but becomes a malignant tumor, there has been known a method of checking germ-line transmission, i.e., transmission of genetic information derived from the pluripotent stem cell from a chimeric animal having a tissue derived from the pluripotent stem cell, to a descendant thereof. In this method, the germ-line transmission is observed in the high-safety pluripotent stem cell which is less likely to become a malignant tumor but not in the teratocarcinoma which becomes a malignant tumor. However, this verification cannot be performed in a human. In a method for establishing high-safety pluripotent stem cells in a human, it is necessary to establish pluripotent stem cells from a laboratory animal using this method, and then verify the germ-line transmission using the established cells.

Therefore, the method for establishing pluripotent stem cells is required to be capable of establishing pluripotent stem cells from not only a human but also a nonhuman animal (preferably, a mouse for which reproductive technologies are well established), and verifying the germ-line transmission using the animal.

From the above discussion, a method for producing human pluripotent stem cells applicable to regeneration therapies, etc., needs to meet the following requirements: 1) an established human pluripotent stem cell must have genomic information identical to that of a patient's cell; 2) a human pluripotent stem cell must be established from 10⁴ or less cells; 3) established human pluripotent stem cells must have a sufficiently uniform property; and 4) the germ-line transmission must be ascertainable using a chimeric animal derived from pluripotent stem cells established by applying the method to a nonhuman animal.

As means for generating a pluripotent stem cell having genomic information identical to that of a patient's cell, there has been known a technique of introducing or transfecting genes into a human normal tissue cell using a retroviral vector so as to forcibly induce expression of the genes to generate a human induced pluripotent stem cell (human iPS cell) closely resembling a human ES cell. For example, there have been reported a method of transfecting four types of genes consisting of Oct3/4, Sox2, Klf4 and c-Myc into a human skin-derived normal fibroblast cell using a retroviral or lentiviral vector so as to induce expression of the genes to generate a human iPS cell (see Non-Patent Document 1), and a method of transfecting four types of genes consisting of Oct3/4, Sox2, Nanog and LIN28 into a human skin-derived normal fibroblast cell using a lentiviral vector so as to induce expression of the genes to generate a human iPS cell (see Non-Patent Document 3).

A human iPS cell can also be generated by a modified technique in which one or two of the above four types of genes are replaced by a low-molecular-weight compound. For example, there has been reported a method of transfecting two types of genes consisting of Oct3/4 and Sox2 into a human skin-derived normal fibroblast cell so as to induce expression of the genes under treatment with a histone deacetylase inhibitor to generate a human iPS cell (see Non-Patent Document 4).

However, in each of the above methods, the genes transfected into a tissue cell are inserted and left at non-specific positions on a chromosome of the generated iPS cell, and thereby genomic information of the iPS cell is not exactly identical to that of the original tissue cell. This means that an iPS cell generated by the above techniques fails to meet the requirement: "a pluripotent stem cell having genomic information identical to that of a patient's cell", necessary in cell replacement therapies.

In terms of ensuring the safety of cell replacement therapies, the above gene insertion phenomenon causes the following problem. If externally transfected genes are inserted at non-specific positions on the chromosome, they are likely to abnormally activate genes adjacent to the insertion sites to cause side-effects, such as tumorigenic transformation of cells. For example, it is known that, if genes are inserted at non-specific positions on a chromosome of a human bone marrow stem cell capable of maintaining a self-renewal ability over a long period of time, using a retroviral vector, oncogenes which are inhibited from transcription in normal cells are abnormally activated due to effects of the inserted exogenous genes to cause transformation of the cells at high frequencies (see Non-Patent Document 5).

This gene insertion phenomenon further causes the following problem in terms of ensuring the safety of cell replacement therapies. In an iPS cell produced by inserting exogenous genes into a chromosome, although expression of the exogenous genes are inhibited during a period where a cell is kept in an undifferentiated state, the expression of the exogenous genes may be induced when the cell has differentiated into a tissue cell, and the resulting cell is likely to become cancerous. For example, it is known that, in a mouse iPS cell-derived mouse individual generated by transfecting four types of genes consisting of Oct3/4, Sox2, Klf4 and c-Myc into a skin-derived normal fibroblast cell using a retroviral vector, cancer develops at high frequencies due to reactivation of the externally introduced c-Myc gene (see Non-Patent Document 6). Further, it is noted that expression of the Klf4 or Oct3/4 gene is also likely to cause transformation of cells (Non-Patent Document 7).

With a view to solving the above problems caused by gene insertion into the chromosome, there has been reported a method of transfecting plasmid DNA capable of inducing transient expression of an exogenous gene, into a cell, to generate an iPS cell from a somatic cell. For example, it has been reported that, when plasmid DNA loaded with four types of genes consisting of Oct3/4, Sox2, Klf4 and c-Myc into a mouse skin-derived normal fibroblast cell by a lipofection-based method, a mouse iPS cell in which the exogenous genes are not inserted into a chromosome thereof can be generated (see Non-Patent Document 8). However, the insertion of the transfected genes into the chromosome is actually observed in 75% of mouse iPS cells generated by this method. Thus, this method does not exactly guarantee to prevent insertion of exogenous genes into a generated iPS cell. Moreover, there is no report describing successful generation of a human iPS cell without insertion of exogenous genes into a chromosome thereof through the use of this method.

In order to solve the problems caused by gene insertion into a chromosome, there has also been reported a method of generating an iPS cell from a somatic cell using an adenoviral vector capable of inducing transient expression of an exogenous gene without insertion of the exogenous gene into a chromosome. For example, it has been reported that a mouse iPS cell can be generated by loading four types of genes consisting of Oct3/4, Sox2, Klf4 and c-Myc into respective adenoviral vectors, individually, and simultaneously transfecting the adenoviral vectors into a mouse liver-derived normal liver cell (see Non-Patent Document 9). However, it has been reported that, in the gene transfection using adenoviral vectors, the introduced genes are inserted at non-specific positions on a chromosome at significant frequencies (see Non-Patent Document 10). Thus, this method does not fully guarantee to prevent insertion of exogenous genes into a generated iPS cell. Moreover, there is no report describing successful generation of an iPS cell without insertion of exogenous genes into a chromosome thereof, from a mouse-derived normal fibroblast cell or a human-derived cell through the use of this method.

It has also been reported that, after generating an iPS cell by randomly inserting four types of genes consisting of Oct3/4, Sox2, Klf4 and c-Myc into a chromosome, the inserted genes can be removed (see Non-Patent Document 11). In cases where Cre recombinase is externally transfected to allow the four types of genes consisting of Oct3/4, Sox2, Klf4 and c-Myc used in generating an iPS cell to be removed from the chromosome (see Non-Patent Document 11), promoter regions necessary for inducing expression of the genes remain on the chromosome. Thus, genomic information of an iPS cell as a final product is not exactly identical to that of a normal cell, and the possibility of occurrence of insertion mutation cannot be denied. It has also been reported that, in cases where a mouse iPS cell is generated by a method of inserting the genes into the chromosome while loading the genes on transposon, the transposon can be fully removed by inducing expression of an enzyme "transposase" (see Non-Patent Document 12). However, this method is inefficient, because the probability of successful removal is about 0.001% of the total number of generated iPS cells, and no example using a human cell is provided therein. The report by Woltjen, et al., includes an example in which artificial insertion of four bases remains even after the removal of transposon. In this case, the possibility of insertional mutagenesis cannot be denied. Moreover, the transposase used for removing the transposon is an enzyme having both an activity capable of excising (removing) the transposon from a chromosome and an activity capable of inserting the transposon into the chromosome. Thus, it is anticipated that transposon excised from an insertion position can be re-inserted at a different position on the chromosome, in theory. Therefore, it is necessary to check each generated iPS cell to verify that no re-insertion occurs therein.

Yu, et al., have reported that simultaneous expression of seven types of genes consisting of Oct3/4, Sox2, Klf4, c-Myc, Nanog, LIN28 and SV40 · T antigen is induced in a human normal fibroblast cell, using an extrachromosomally-replicable circular DNA vector (EBV vector) having a replication origin of Epstein-Barr virus (EBV) and EBNA1 gene, and then a human iPS cell including no exogenous gene can be generated by means of free fall of the vector (see Non-Patent Document 13). As of now, this is the only report describing the generation of pluripotent stem cells that have genetic information identical to that of a somatic cell. However, generation efficiency is in the range of about 0.0003 to 0.0006%, i.e., at least 3 × 10⁵ cells are required to establish a single iPS cell. Moreover, it is known that genome DNA of EBV is not only extrachromosomally replicated but also inserted into the chromosome at high frequencies (see Non-Patent Document 14). Thus, this method also does not fully guarantee to prevent insertion of exogenous genes into a generated iPS cell, and it is necessary to check each generated human iPS cell to verify that no exogenous gene is included therein.

As other means for generating a pluripotent stem cell having genomic information identical to that of an adult tissue cell, there have been reported a method of transplanting a tissue cell nucleus to an enucleated unfertilized ovum (see Non-Patent Document 15), and a method of adding a peptide capable of crossing cell membranes, to four types of proteins consisting of Oct3/4, Sox2, Klf4 and c-Myc, and transfecting the proteins from outside a cell (see Non-Patent Document 16). There is no report describing successful generation of a human pluripotent stem cell by these methods.

Recently, Fusaki, et al., have reported a method of inducing expression of Oct3/4, Sox2, Klf4 and c-Myc genes in a human skin-derived fibroblast cell, using as a vector a Sendai virus capable of inducing expression of an exogenous gene without inserting the exogenous gene into a chromosome of the cell to produce pluripotent stem cells (see Non-Patent Document 17) and Patent Document 1). This report says that, based on this method, pluripotent stem cells were established with a maximum efficiency rate of 1%, by: loading four types of reprogramming genes on respective vectors, individually; mixing the vectors together; and infecting a cell therewith. However, this report makes no mention of uniformity of the established cells. In view of an analysis result of a gene expression represented by semi-qualitative RT-PCR (Reverse Transcription-Polymerase Chain Reaction), it is apparent that properties of the established cells are non-uniform. Moreover, only a human cell is described in the examples. In other words, it is not demonstrated that this method is broadly applicable to different animal species, and that germ-line transmission can be ascertained using a chimeric animal derived from a pluripotent stem cell established by applying this method to a nonhuman animal.

Thus, as for means to establish human pluripotent stem cells necessary for regeneration therapies, there has not been reported any method which meets the following four requirements: 1) the established human pluripotent stem cells have genetic information identical to that of the patient's cell; 2) the human pluripotent stem cell can be established from 10⁴ or less cells; 3) properties of the established human pluripotent stem cells are sufficiently uniform (specifically, a correlation coefficient of gene-expression pattern is 0.98 or more); and 4) the germ-line transmission can be ascertained using a chimeric animal derived from a pluripotent stem cell established by applying the method to a nonhuman animal.

### LIST OF PRIOR ART DOCUMENTS

### [PATENT DOCUMENTS]

Patent Document 1: PCT/JP 2009/062911

### [NON-PATENT DOCUMENTS]

Non-Patent Document 1: Takahashi, et al., Cell, 131, 861-872, 2007
Non-Patent Document 2: Carey, et al., Proc. Natl. Acad. Sci. USA, 106, 157-162, 2009
Non-Patent Document 3: Yu, et al., Science, 318, 1917-1920, 2007
Non-Patent Document 4: Huangfu, et al., Nature Biotechnology, 26, 1269-1275, 2008
Non-Patent Document 5: Hacein-Bey-Abina, et al., Science, 302, 415-419, 2003
Non-Patent Document 6: Takahashi and Yamanaka, Cell, 126, 663-676, 2006
Non-Patent Document 7: Jaenishi and Young, Cell, 132, 562-582, 2008
Non-Patent Document 8: Okita, et al., Science, 322, 949-953, 2008
Non-Patent Document 9: Stadfeld, et al., Science, 322, 945-949, 2008
Non-Patent Document 10: Ohbayashi, et al., Proc. Natl. Acad. Sci. USA, 102, 13528-13533,2005
Non-Patent Document 11: Kaji, et al., Nature, 458, 771-775, 2009
Non-Patent Document 12: Woltjen, et al., Nature, 458, 766-770, 2009
Non-Patent Document 13: Yu, et al., Science, 324, 797-801, 2009
Non-Patent Document 14: Hurley, et al., J. Virol, 65, 1245-1254, 1991
Non-Patent Document 15: Wakayama, et al., Science, 292, 740-743, 2002
Non-Patent Document 16: Zhou, et al., Cell Stem Cell, 4, 381-384, 2009
Non-Patent Document 17: Fusaki, et al., Proc. Jpn. Acad. Ser. B85, 348-362, 2009
Non-Patent Document 18: Nakanishi, Regenerative Medicine, under printing, 2010

### SUMMARY OF THE INVENTION

The invention in its broadest sense is as defined in the independent claims.

### [PROBLEM TO BE SOLVED BY THE INVENTION]

It is therefore an object of the present invention to establish an induced pluripotent stem cell (hereinafter referred to occasionally as "iPS cell") from a normal human tissue cell at an efficiency rate of 0.01% or more, in such a manner as to have genomic information identical to that of a patient's cell and properties similar to those of an ES cell, so as to avoid the possibility of immunological rejection of a transplanted cell and tumorigenic transformation due to insertion of exogenous genes into a chromosome.

### [MEANS FOR SOLVING THE PROBLEM]

This object can be achieved by using a gene expression system free of an activity capable of altering human genome by interactions of recombination, insertion, repair, etc. The inventors found that a differentiated animal cell can be efficiently reprogrammed by loading genes encoding respective human gene products consisting of Oct3/4, Sox2, Klf4 and c-Myc serving as reprogramming genes, on a sustained expression-type Sendai virus vector (JP 4478788B and PCT/JP 2008/057212), and transfecting the genes into the differentiated animal cell. The inventors further found that the reprogramming gene-loaded vector is free of causing incorporation of the exogenous genes into a chromosome, and capable of being removed easily and quickly using an siRNA after the reprogramming, which makes it possible to establish induced pluripotent stem cells (iPS cells) having genomic information identical to that of a donor individual of the differentiated cell. Based on the findings, the present invention has been accomplished.

The present invention provides the reprogramming gene-loaded Sendai virus of claims 1-4, the reprogramming gene-loaded Sendai virus vector of claims 5 and 6, the template vector of claims 7-12, a cell according to any one of claims 13-16 and methods of claims 17, 18, 19 and 20. Disclosed herein is:
(1) A reprogramming gene-loaded Sendai virus characterized in that it comprises, as its genome, Sendai virus genes and reprogramming genes, wherein the Sendai virus genes include an NP gene, P/C gene, M gene, F gene, HN gene and L gene, and wherein each of the M gene, the F gene and the HN gene is from a Sendai virus strain Cl.151-derived gene, and wherein at least one of the M gene, the F gene and the HN gene is functionally deleted, and the L gene encodes the amino-acid sequence of the L protein in which the amino-acid residue at position 1618 is valine.
(2) The Sendai virus set forth in (1), characterized in that all of the M gene, the F gene and the HN gene are functionally deleted.
(3) The Sendai virus set forth in (1) or (2), characterized in that it is a virus particle.
(4) The Sendai virus set forth in any one of (1) to (3), characterized in that the functional deletion of the M, F or HN gene is a result of subjecting the gene to insertion or replacement by a reprogramming gene and/or a marker gene.
(5) The Sendai virus set forth in any one of (1) to (4), characterized in that the reprogramming genes comprise a combination of Oct3/4, Sox2 and Klf4, or a combination of Oct3/4, Sox2, Klf4 and c-Myc.
(6) A reprogramming gene-loaded Sendai virus vector for generating an induced pluripotent stem cell, characterized in that it comprises the Sendai virus set forth in any one of (1) to (5).
(7) A reprogramming gene-loaded Sendai virus vector for generating an induced pluripotent stem cell, characterized in that it comprises the Sendai virus set forth in any one of (1) to (5), wherein the Sendai virus vector comprises, on its genome, a target sequence for an expressed microRNA of a differentiated cell for use in producing an induced pluripotent stem cell.
(8) A template vector for preparing a reprogramming gene-loaded Sendai virus, characterized in that it comprises a cloning vector with Sendai virus genes and reprogramming genes inserted therein, wherein the Sendai virus genes include an NP gene, P/C gene, M gene, F gene, HN gene and L gene, and wherein each of the M gene, the F gene and the HN gene is from the Sendai virus strain Cl.151-derived gene, and wherein at least one of the M gene, the F gene and the HN gene is functionally deleted, and the L gene encodes the amino-acid sequence of the L protein in which the amino-acid residue at position 1618 is valine.
(9) The template vector set forth in (8), characterized in that all of the M gene, the F gene and the HN gene are functionally deleted.
(10) The template vector set forth in (8) or (9), characterized in that the functional deletion of the M, F or HN gene is a result of subjecting the gene to insertion or replacement by a reprogramming gene and/or a marker gene.
(11) The template vector set forth in any one of (8) to (10), characterized in that the cloning vector is a phage vector.
(12) The template vector set forth in (11), characterized in that the phage vector is a λ phage vector.
(13) The template vector set forth in any one of (8) to (12), characterized in that the reprogramming genes comprise a combination of Oct3/4, Sox2 and Klf4, or a combination of Oct3/4, Sox2, Klf4 and c-Myc.
(14) The template vector set forth in any one of (8) to (13), characterized in that it comprises DNA.
(15) The template vector set forth in (14), characterized in that it comprises a sequence complementary to a target sequence for an expressed microRNA of a differentiated cell for use in producing an induced pluripotent stem cell.
(16) A cell characterized in that it is transfected with the template vector set forth in any one of (8) to (15).
(17) The cell set forth in (16), characterized in that it is further transfected with at least one of an M gene, F gene and HN gene which are functionally deleted, or further transfected with an NP gene, P gene and L gene.
(18) The cell set forth in (17), characterized in that T7 RNA polymerase is expressed therein.
(19) A method of producing a reprogramming gene-loaded Sendai virus vector, characterized in that it comprises cultivating the cell set forth in any one of (16) to (18), in culture medium, to collect a Sendai virus particle which comprises, as its genome, Sendai virus genes and reprogramming genes, wherein the Sendai virus genes include NP gene, P/C gene, M gene, F gene, HN gene and L gene, and wherein each of the M gene, the F gene and the HN gene is from a Sendai virus strain Cl.151-derived gene, and wherein at least one of the M gene, the F gene and the HN gene is functionally deleted, and the L gene encodes the amino-acid sequence of the L protein in which the amino-acid residue at position 1618 is valine.
(20) A method for producing an induced pluripotent stem cell, characterized in that it comprises the steps of: infecting a differentiated cell with the reprogramming gene-loaded Sendai virus vector set forth in (6) to reprogram the differentiated cell; and then allowing siRNA to act on the vector to remove the reprogramming gene-loaded Sendai virus vector from the cell.
(21) The method set forth in (20), characterized in that the siRNA is adapted to target an L protein of a Sendai virus.
(22) An siRNA which is adapted to target an L protein of a Sendai virus.
(23) A reagent for removing a reprogramming gene-loaded Sendai virus vector after reprogramming a differentiated cell, comprising the siRNA set forth in (22).
(24) A method of producing an induced pluripotent stem cell, characterized in that it comprises: infecting a differentiated cell with the reprogramming gene-loaded Sendai virus vector set forth in (7) to reprogram the differentiated cell; and then automatically removing the reprogramming gene-loaded Sendai virus vector, after forming an induced pluripotent stem cell, wherein the differentiated cell is a microRNA-expressing cell.
(25) A method of producing an induced pluripotent stem cell, characterized in that it comprises: infecting a differentiated cell with the reprogramming gene-loaded Sendai virus vector set forth in (6) or (7) to reprogram the differentiated cell; and then culturing the cell under high-temperature conditions to promote removal of the reprogramming gene-loaded Sendai virus vector from the cell.

### [EFFECT OF THE INVENTION]

In the Sendai virus vector of the present invention, mammalian reprogramming genes comprising a combination of Oct3/4, Sox2 and Klf4 or a combination of Oct3/4, Sox2, Klf4 and c-Myc can be loaded on a single common vector, and expressed simultaneously in the same cell, so that a manipulation for reprogramming a differentiated cell is significantly simple and efficient. In addition, the reprogramming gene-loaded Sendai virus vector of the present invention can express reprogramming genes while being retained in a cytoplasm of the cell in a sustained and stable manner, so that it is significantly safe because there is no risk of causing insertion of the exogenous genes into a chromosome of the cell and therefore no risk of causing the cell to become cancerous. Furthermore, based on the use of the vector of the present invention, an induced pluripotent stem cell (hereinafter referred to occasionally as "iPS cell") having genomic information identical to that of a patient's cell and pluripotency similar to that of an ES cell can be established at a pluripotent stem cell-establishment efficiency rate of at least 0.01% to over 1%, even from 10⁴ or less human normal tissue cells, and properties of the established induced pluripotent stem cells are significantly uniform as evidenced by the fact that a correlation coefficient between respective gene expression patterns of the cells is 0.98 or more, so that it becomes possible to avoid tumorigenic transformation which would otherwise occur due to prolonged culture of induced pluripotent stem cells. As for the pluripotent stem cell obtained using the Sendai virus vector of the present invention, germ-line transmission has been ascertained in a laboratory animal such as a mouse, so that it can be said that it is less likely to become a malignant tumor, are highly safe. In view of this, application thereof to human can be highly expected.

After inducing expression of the reprogramming genes in the cytoplasm to transform the cytoplasm into a pluripotent stem cell, the reprogramming gene-loaded Sendai virus vector can be significantly easily removed from the cell using siRNA. Consequently, the safety is further enhanced, and an iPS cell having genomic information identical to that of a donor individual of the differentiated cell is obtained. Further, a Sendai virus vector incorporating a target sequence for a microRNA expressed in the pluripotent stem cell may be used. In this case, after the cell reprogramming, the vector can be automatically removed. In other embodiment, the removal of the vector can be facilitated by culturing the cell at high-temperatures.

By utilizing wide species-specificity/cellular-specificity of a Sendai virus, a pluripotent stem cell can be established from nonhuman animal-derived cells, or human tissue cells other fibroblast cells (particularly, blood cells). This makes it possible to verify the function of the pluripotent stem cell using a nonhuman animal.

Thus, compared with conventional vectors for use in generating iPS cells, such as adenoviral vectors, EBV vectors, other DNA vectors, and conventional Sendai virus vectors, the present invention makes it possible to generate iPS cells significantly efficiently and simply with excellent reproducibility, and drastically improve the safety of the generated iPS cell. This contributes greatly to technical development in a wide range of technical fields, such as regeneration therapies (particularly, cell replacement therapy and gene therapy), researches on drug discovery using patient-derived iPS cells having various genetic backgrounds, production of biotechnology-based medicines using human cells, and investigations for causes of and developments of treatment methods for cancers and intractable diseases.

### BRIEF DESCRIPTION OF THE DRAWINGS

FIG. 1 is a diagram of the preparation of a template cDNA for producing a hOct4/hSox2/hKlf4 sustained expression-inducing Sendai virus vector.
FIG. 2 is a diagram of the preparation of a template cDNA for producing a hOct4/hSox2/hKlf4/hc-Myc sustained expression-inducing Sendai virus vector.
FIG. 3 is a photograph depicting an observation result of removal of a sustained expression-type Sendai virus vector using siRNA.
FIG. 4 is a photograph depicting a time-series observation result of expression of alkaline phosphatase in a mouse embryonic fibroblast cell infected with the hOct4/hSox2/hKlf4/hc-Myc sustained expression-inducing Sendai virus vector.
FIG. 5 is a photograph depicting a time-series observation result of expression of EGFP in a Nanog-EGFP knock-in mouse embryonic fibroblast cell infected with the hOct4/hSox2/hKlf4/ hc-Myc sustained expression-inducing Sendai virus vector.
FIG. 6 is a photograph depicting a detection result of each expression of Sendai virus NP gene, endogenous mouse Oct4 gene and endogenous mouse Nanog gene in a mouse embryonic fibroblast cell on the 14th day after infection with the hOct4/hSox2/hKlf4/hc-Myc sustained expression-inducing Sendai virus vector.
FIG. 7 is a photograph depicting a detection result of expression of SSEA-1 protein in a mouse embryonic fibroblast cell on the 14th day after infection with the hOct4/hSox2/hKlf4/ hc-Myc sustained expression-inducing Sendai virus vector.
FIG. 8 is an electrophoresis photograph depicting a genome PCR-based gene-type analysis result of a mouse iPS marker-expressing cell prepared using a sustained expression-type Sendai virus vector.
FIG. 9 is an electrophoresis photograph depicting a detection result of each expression of Sendai virus NP gene and endogenous mouse Nanog gene in a mouse iPS marker-expressing cell after removal of a sustained expression-inducing Sendai virus vector using siRNA.

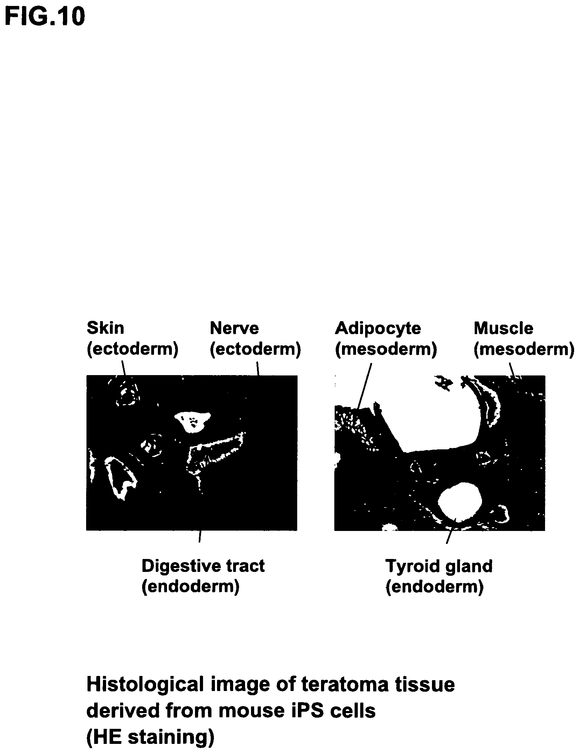

FIG. 10 is a photograph depicting an observation result of a tissue slice of teratoma derived from a mouse iPS marker-expressing cell after removal of a sustained expression-inducing Sendai virus vector using siRNA.
FIG. 11 is a photograph depicting a time-series observation result of expression of alkaline phosphatase in a human embryonic fibroblast cell infected with the hOct4/hSox2/hKlf4/hc-Myc sustained expression-inducing Sendai virus vector.
FIG. 12 is a photograph depicting an observation result of expression of endogenous human Nanog gene in a human embryonic fibroblast cell on the 14th day after infection with the hOct4/hSox2/hKlf4/hc-Myc sustained expression-inducing Sendai virus vector.
FIG. 13 is a photograph depicting an observation result of expression of SSEA-4 protein in a human embryonic fibroblast cell on the 25th day after infection with the hOct4/hSox2/hKlf4/hc-Myc sustained expression-inducing Sendai virus vector.
FIG. 14 is a photograph depicting a detection result of expression of Sendai virus NP gene and endogenous human Nanog gene in a human iPS marker-expressing cell after removal of a sustained expression-inducing Sendai virus vector using siRNA.
FIG. 15 is a photograph depicting an observation result of expression of SSEA-4 protein and endogenous human Oct4 protein in a human iPS marker-expressing cell after removal of a sustained expression-inducing Sendai virus vector using siRNA.
FIG. 16 is a photograph depicting respective emergence efficiencies of a human iPS marker (alkaline phosphatase)-expressing cell colony, under normal culture conditions (37°C, 5% CO₂) and under high-temperature culture conditions (40°C, 2% CO₂).
FIG. 17 is a photograph depicting respective efficiencies of removal of a Sendai virus vector from a human iPS marker-expressing cell, under normal culture conditions (37°C, 5% CO₂) and under high-temperature culture conditions (40°C, 2% CO₂).
FIG. 18 is a photograph depicting a mouse iPS cell-derived chimeric mouse prepared using a hOct4/hSox2/hKlf4/hc-Myc sustained expression-inducing Sendai virus vector Version 2, and germ-line transmission from the mouse.
FIG. 19 is a photograph depicting an observation result of a tissue slice of a teratoma derived from a human iPS marker-expressing cell after removal of a hOct4/hSox2/hKlf4/hc-Myc sustained expression-inducing Sendai virus vector.
FIG. 20 is a diagram schematically illustrating the preparation of a template cDNA for producing the hOct4/hSox2/hKlf4/hc-Myc sustained expression-inducing Sendai virus vector Version 2.
FIG. 21 is a diagram schematically illustrating the preparation of a template cDNA for producing a hOct4/hSox2/hKlf4/hc-Myc sustained expression-inducing Sendai virus vector Version 3.
FIG. 22 is a graph obtained by quantitatively measuring a temporal change in removal of a sustained expression-type Sendai virus vector from a cell, using siRNA.
FIG. 23 is a photograph and a graph illustrating a comparison between gene expression patterns in two cases: one case where two types of exogenous genes are loaded on a single common sustained expression-type Sendai virus vector; and the other case where the two types of exogenous genes are loaded on respective sustained expression-type Sendai virus vectors, individually.
FIG. 24 is a graph illustrating emergence efficiency of a mouse iPS marker-expressing cell in two cases: one case where four types of exogenous genes are loaded on a single common sustained expression-type Sendai virus vector; and the other case where the four types of exogenous genes are loaded on respective sustained expression-type Sendai virus vectors, individually.
FIG. 25 is a photograph depicting the establishment of a human iPS marker-expressing cell using the hOct4/hSox2/hKlf4/hc-Myc sustained expression-inducing Sendai virus vector Version 3.
FIG. 26 is a photograph of a human iPS marker-expressing cell established from adult human peripheral blood cells using a hOct4/hSox2/hKlf4/hc-Myc sustained expression-inducing Sendai virus vector.
FIG. 27 is a diagram illustrating a comparison between gene expression patterns of a plurality of types of human iPS marker-expressing cells established using a hOct4/hSox2/hKlf4/ hc-Myc sustained expression-inducing Sendai virus vector.

### DESCRIPTION OF EMBODIMENTS

The invention in its broadest sense is as defined in the independent claims. A vector loaded with reprogramming genes for use in producing an induced pluripotent stem cell in the present invention is a Sendai virus particle which comprises, as its genome Sendai virus genes including NP gene, P/C gene and L gene each derived from a Sendai virus, and at least one gene from F, M and HN genes of the Sendai virus is replaced by mammalian reprogramming genes which comprise a combination of Oct3/4, Sox2 and Klf4, or a combination of Oct3/4, Sox2, Klf4 and c-Myc, wherein the L gene is a mutated gene and encodes an amino-acid sequence of the L-protein in which the amino-acid residue at position 1618 is valine and each of the M gene, the F gene and the HN gene is a gene derived from a Sendai virus strain Cl.151 (this vector will hereinafter be referred to as "Sendai virus vector").

As used in this specification, the term "gene" or "gene material" encompasses negative-strand RNA or cDNA and positive-strand RNA or cDNA complementary thereto. In other words, any vector capable of synthesizing either one of such genes or gene materials by means of transcription or reverse transcription should be construed as being included in the present invention.

As used in this specification, the term "induced pluripotent stem cell (iPS cell)" means a cell which expresses a morphology similar to an embryonic stem cell (ES cell), and an embryonic stem cell-specific marker, and has a self-renewal ability in vitro. It is known that the iPS cell has the potential to differentiate into any of the three germ layers in vivo or in vitro. The marker includes a well-known type, such as Oct4, alkaline phosphatase, SSEA-1 and SSEA-4.

### [CONSTITUENT MATERIALS OF SENDAI VIRUS VECTOR]

The Sendai virus vector is a gene transfection/expression vector in which any exogenous gene is inserted into a genome of a Sendai virus or used to replace a gene of a Sendai virus thus enabling the expression of the exogenous gene. A Sendai virus has an NP gene, a P/C gene, an F gene, an M gene, an HN gene and an L gene, wherein each of the NP, P/C and L genes is involved in the transcription and replication of the Sendai virus, and each of the F, M and HN genes is involved in formation of a virus particle. Therefore, a Sendai virus vector subjected to replacement of the F, M and HN genes is incapable of forming new virus particles by itself, i.e., is non-transmissible, after transfection into a cell.

The Sendai virus vector of the present invention comprises an L gene which encodes an L protein having a valine residue at position 1618. This mutation was found in the amino-acid sequence of an L protein derived from the Sendai virus strain Cl.151. It is known that the Sendai virus strain Cl.151 exhibits temperature-sensitive growth, where almost no virus particle is produced at 38°C, but at 32°C, the replication cycle becomes active and permits the production of virus particles. The Sendai virus strain Cl.151 was first reported by Tetsuya Yoshida, PhD, in 1979 (Yoshida, et al., (1979), Virology, 92, 139-154).

In the Sendai virus strain Cl.151 having a gene which encodes an L protein having a valine residue at position 1618, an ability to induce interferon activity is lowered, and therefore a sustained infectious ability is exhibited without cytotoxicity, so that, when an exogenous gene is borne thereby, expression of the gene will be maintained in the cell for a long period of time. For example, as the L gene, it is possible to use a mutated L gene obtained by mutating the L gene of the Sendai virus strain Nagoya in such a manner that a leucine residue at position 1618 of the L gene is replaced by valine. As described herein, the L protein having a valine residue at position 1618 will be referred to occasionally as a "mutated-L protein".

Thus, as long as the L gene has the above mutation, each of the NP, C/P and L genes as constituent genes of the Sendai virus vector of the present invention may have a base sequences of a Sendai virus strain with cytotoxicity and without a sustained infectious ability, such as a Sendai virus strain Nagoya or Z, as well as a base sequence of the Sendai virus strain Cl.151.

A transcriptional termination sequence of a Sendai virus may be artificially inserted into the 3'-terminal end of the leader RNA, which makes it possible to reduce the copy number of anti-genomic RNAs and further lower the interferon-inducing ability.

As a prerequisite to sustained infection of a Sendai virus in an animal cell, it is essential that the Sendai virus has the mutated-L gene, and all of the F, M and HN genes are derived from the Sendai virus strain Cl.151. Thus, the Sendai virus vector having the mutated-L gene and the Sendai virus strain Cl.151-derived F, M and HN genes together can have a sustained infectious ability without cytotoxicity, so that, when an exogenous gene is borne by the Sendai virus vector, the expression of the gene will be maintained in the cell over a long period of time. In Sendai virus vectors based on the strain Cl.151, one or more (including "all") of the strain Cl.151-derived F, M and HN genes may be replaced by mammalian reprogramming genes, without interfering with the ability to drive the expression of the gene loaded thereon. In this case, although the replacement of only one of the three genes allows the transmissibility of the vector to be significantly suppressed, it is preferable to replace all of the F, M and HN genes with mammalian reprogramming genes in order to fully suppress the transmissibility. The replacement of one or more of the F, M and HN genes may be achieved by simply deleting a part or all of the three genes, or by insertion or replacement by a given exogenous gene.

A full-length cDNA of the Sendai virus strain Cl.151 has already been registered in the GenBank (Accession Number AB275416).

### [REPROGRAMMING GENE]

Mammalian reprogramming genes are inserted into the Sendai virus vector of the present invention. The mammalian reprogramming genes comprise a combination of Oct3/4, Sox2 and Klf4 genes or a combination of Oct3/4, Sox2, Klf4 and c-Myc genes of human, mouse or any other mammal; and optionally the combination plus one or more of Nanog, Lin28, Esrrb, UTFI, TERT (telomerase catalytic subunit) and SV40 T antigen genes.

### [TEMPLATE VECTOR FOR PREPARING REPROGRAMMING GENE-LOADED SENDAI VIRUS VECTOR]

In the present invention, the NP, P/C and mutated-L genes as constituent materials of the Sendai virus vector are inserted into a cloning vector such as phage, together with the reprogramming genes. In this process, all of the required reprogramming genes can be inserted together. This allows an aftermentioned reprogramming gene-loaded Sendai virus vector to contain all of the genes required for reprogramming, so that the reprogramming can be quite efficiently performed without the need for transfecting each reprogramming gene into a different vector as in the conventional techniques.

The recombinant vector obtained in the above manner can serve as a template for preparing a reprogramming gene-loaded Sendai virus vector of the present invention, i.e., a Sendai virus particle bearing the reprogramming genes. This recombinant vector will hereinafter be referred to as "template vector".

In regard to the template vector, the NP, P/C and mutated-L genes and the reprogramming genes are incorporated into a vector such as phage, in the following order: NP → P/C → reprogramming genes (a marker gene may further be transfected thereinto) → mutated L.

The reprogramming genes are used to replace at least one of the F, M and HN genes of the Sendai virus vector, by inserting it into the at least one gene or by replacing the sequence of the at least one gene therewith.

A selectable marker gene, such as a drug-resistance gene, may also be inserted into the template vector. This makes it possible to facilitate screening of a target cell into which the template vector or the Sendai virus vector is inserted.

More specifically, the template vector is prepared by combining the constituent materials of the Sendai virus vector comprising the above genes, and the reprogramming gene cDNA and/or the marker gene cDNA, together in the above order, in such a manner as to allow a (+) strand genomic RNA to be formed in a cell. For example, the constituent material cDNA is incorporated into a cloning vector, such as λ DASH II. A T7 promoter sequence and three guanidine residues are then arranged on the upstream side of the incorporated full-length cDNA (i.e.. at the 3'-terminal end of the genomic RNA) in this order, and a hairpin ribozyme sequence of a tobacco ringspot virus and a termination sequence of T7 RNA polymerase are then arranged on the downstream side of the full-length cDNA (i.e. at the 5'-terminal end of the genomic RNA) in this order.

The T7 promoter sequence is added to allow a (+) strand genomic RNA to be biosynthesized from the 3'-terminal end of the genomic RNA by T7 RNA polymerase, and three guanidine residues are added to enhance the efficiency of RNA transcription by the T7 RNA polymerase (S. Leyrer, et al., (1998) J. Virol. Methods, 75; 47-58). The hairpin ribozyme sequence of the tobacco ringspot virus is added to allow the transcript (+) strand genomic RNA to be accurately cut at one end, and the termination sequence of T7 RNA polymerase is added to allow the RNA transcription by the T7 RNA polymerase to accurately terminate.

### [PREPARATION OF REPROGRAMMING GENE-LOADED SENDAI VIRUS VECTOR]

The template vector with the reprogramming genes prepared in the above manner can then be transfected into a viral vector-producing cell in order to prepare a reprogramming gene-loaded Sendai virus vector.

In order to transcribe (+) strand anti-genomic RNA from the template vector in a virus-producing cell, it is necessary to supply T7 RNA polymerase. For example, the viral vector-producing cell can be infected with T7 RNA polymerase-expression vaccinia virus, or may be a cell strain cloned to constantly express T7 RNA polymerase.

In the cell strain (BHK/T7 cell) capable of constantly expressing humanized T7 RNA polymerase, the expression level of T7 RNA polymerase is significantly increased as compared with a cell strain (BSR-T7-5 cell) capable of expressing conventional bacterial T7 RNA polymerase. As a result of production of recombinant viruses using the BHK/T7 cell, recombinant viruses can be efficiently collected. In other words, the use of a cell strain having an enhanced expression level of T7 RNA polymerase is effective for efficiently producing recombinant viruses.

In the virus vector-producing cell transfected with the template vector, T7 RNA polymerase drives transcription of the template vector DNA to RNA, initially from the T7 promoter. However, in the produced RNA molecule, sequences downstream of the hairpin ribozyme sequence are cleaved off and removed by the hairpin ribozyme sequence, so that a (+) strand anti-genomic RNA molecule is generated corresponding to a DNA portion which includes the NP gene, the P/C gene, the reprogramming genes and the mutated-L gene in the template vector, and may further include a marker gene.

An expression vector for producing NP, P and L gene products may be additionally transfected into the viral vector-producing cell having the (+) strand anti-genomic RNA transcribed from the template vector by the T7 RNA polymerase. In this case, the NP, P and L gene products are bound to the (+) strand anti-genomic RNA to form an RNP complex (nucleocapsid). Then, using the RNP complex as a template, a (-) strand genomic RNA is transcribed from the (+) strand anti-genomic RNA by the RNA polymerase in the viral vector-producing cell. The (-) strand genomic RNA is bound to NP, P and mutated-L gene products in the viral vector-producing cell to form a RNP complex including the (-) strand genomic RNA.

In the template vector used in the above manner, one or more of the strain Cl.151-derived M, F and HN genes are replaced with mammalian reprogramming genes to allow the vector to become non-transmissible. Thus, the RNP complex formed based on this template vector is suppressed from formation of virus particles required for infecting a tissue cell. Therefore, an expression vector capable of expressing a missing one or more of the F, M and HN gene products is transfected into the virus vector-producing cell comprising the RNP complex (nucleocapsid) with the (-) strain genomic RNA formed in the above manner. The transfected cell is then incubated at a virus-particle production temperature of 32°C.

Consequently, the RNP complex (nucleocapsid) including the (-) strand genomic RNA is incorporated into viral vector particles to reconstruct reprogramming gene-loaded Sendai virus particles. As described above, in the present invention, an expression vector loaded with a missing one or more of the F, M and HN genes is separately transfected into a virus-producing cell to form virus particles. This makes it possible to harvest the virus particles from a culture supernatant of the viral vector-producing cell. In cases where two or more of the F, M and HN genes are missing, an expression system may comprise a plurality of vectors each transfected with a respective one of the missing genes, or may comprise a vector transfected with all of the missing genes.

In the above virus-particle production process, with a view to assisting the formation of virus protein to efficiently produce virus particles, it is preferable to transfect an expression vector having an NP gene, a P/C gene and an L gene into the cell, in addition to the at least one replaced gene(s).

In addition, a drug-resistance gene may be inserted into a target viral vector-producing cell as discussed above. In this case, it becomes possible to select the viral vector-producing cell through incubation in culture medium containing an appropriate drug. Alternatively, a target viral vector-producing cell may be isolated using a marker gene, such as the EGFP gene.

The reprogramming gene-loaded Sendai virus vector obtained in the above manner is in the form of a virus particle that is capable of stably infecting a differentiated cell. However, as one or more of the F, M and HN genes of the vector are replaced, the formation of virus particles required for the infection or transmission is suppressed after the transfection. Thus, the vector is non-transmissible. In addition, the L gene of the vector is mutated such that leucine of the L protein at position 1618 is replaced with valine. Thus, the vector has sustained infectious ability without interferon-inducing ability, so that it can stably express the reprogramming genes while being retained in the cell over a long period of time.

### [REPROGRAMMING OF DIFFERENTIATED CELL]

The reprogramming gene-loaded Sendai virus vector obtained in the form of a virus particle in the above manner is then transfected into a target differentiated cell to be subjected to reprogramming. For example, the target differentiated cell may include: cells derived from a normal human or a patient with a disease, such as fibroblast cells, oral mucosal cells, blood cells, and hair follicle epithelial cells; and cells obtained by surgical intervention or tissue biopsies, such as liver cells, large intestinal mucosa cells, small intestinal mucosa cells and lung epithelial cells. The target differentiated cell is not limited to human cells, but may include differentiated cells of an animal, such as mouse, rat, hamster, guinea pig, rabbit, dog, cat, monkey, bovine, pig, sheep, goat or chicken, which is known to be capable of being infected with a Sendai virus. This is because the Sendai virus is capable of infecting various cells derived from an extremely wide variety of animal species. A known cell incapable of being infected with the Sendai virus is only equine-derived cells and B-lymphocytes of various animal species.

This feature is an advantage significantly different from other viral vectors that exhibit a narrow host range, such as a retroviral vector, a lentiviral vector and an adenoviral vector; or other gene expression systems usable only in human cells, such as an EBV vector; or plasmid vectors, transposon and EBV vectors that can be transfected into only limited cell species due to a need for being used in combination with a physical gene introduction process. For example, although the adenoviral vector can be used just to reprogram a mature mouse liver cell at a reprogramming efficiency rate of 0.0005% or less. Moreover, the adenoviral vectors are incapable of reprogramming a mouse or human fibroblast cell. On the other hand, the reprogramming gene-loaded Sendai virus vector of the present invention can reprogram a mouse fibroblast cell (Example 5), a human blood mononuclear cell (Example 18), as well as a human fibroblast cell. In addition, a chimeric mouse derived from a mouse pluripotent stem cell produced using the reprogramming gene-loaded Sendai virus vector of the present invention has an ability to achieve the germ-line transmission, which verifies that cell lines produced by the present invention are high-safety normal pluripotent stem cells that are less likely to become malignant tumor (Example 10).

In the reprogramming gene-loaded Sendai virus vector of the present invention, an L gene is mutated to have no interferon induction activity, and at least one gene from M, F and HN genes in a wild-type Sendai virus are replaced. Thus, the vector exhibits a sustained infectious ability without cytotoxicity, and, after infection of a differentiated cell, stably exists in the cytoplasm of the infected cell independently with respect to the chromosome. Even after cell division, this state is maintained. This feature is not observed in other types of Sendai virus vectors without a mutated-L gene or with at least one of the M, F and HN genes of the wild-type Sendai virus. Thus, the use of the Sendai virus vector of the present invention makes it possible to maintain expression of the reprogramming genes for 10 to 20 days, which is required for the completion of the reprogramming process. Reprogramming can therefore be completed by a single gene transfection. This advantage is not available with adenoviral vectors or plasmid vectors that are only capable of inducing transient gene expression. For example, in cases where Oct3/4, Sox2, Klf4 and c-Myc are loaded on the reprogramming gene-loaded Sendai virus vector of the present invention, even if the vector is transfected into a cell only once, the cell can maintain endogenous (i.e., non-vector-derived or cell-derived) expression of the reprogramming genes and alkaline phosphatase as a marker of an embryo-stem cell (ES cell), after the 7th to 14th days.

As long as the Sendai virus vector of the present invention is used, there is no possibility that a part or all of the introduced genes are inserted at non-specific positions on a chromosome. Thus, resulting iPS cells are significantly safe because there is no risk of causing transformation. In this regard, the Sendai virus vector of the present invention is significantly advantageous, compared to a system causing genes to be inserted at non-specific positions on a chromosome, such as a retroviral vector or a lentiviral vector/transposon. The Sendai virus vector of the present invention is also significantly advantageous, compared to a system where a possibility of gene insertion into chromosomes is hardly denied, such the an adenovirus vector or a plasmid vector (including the EBV vector). For example, it is known that c-Myc, Oct4 or Lin28 in the reprogramming genes causes cell transformation or heteromorphism by itself. Even these genes can be safely used in combination with the Sendai virus vector of the present invention.

A vector system for use in reprogramming a cell requires a property capable of establishing pluripotent stem cells having uniform properties with excellent reproducibility. In this connection, it is necessary that a plurality of (e.g., four) types of reprogramming genes can be simultaneously transfected into a single common cell, and the transfected reprogramming genes are always expressed at a constant rate. In cases where a plurality of types of genes are transfected into a cell by a Sendai virus vector, the following two processes are contemplatable: one process of loading all of the genes on a single common vector as illustrated in inventive examples; and the other process of loading the genes on respective ones of a plurality of vectors and infecting a cell with the vectors after mixing them together, as illustrated in the Non-Patent Document 17 and the Patent Document 1. A difference in gene expression between the two processes was verified by comparing the following two cases: one case where the Enhanced Green Fluorescent Protein (EGFP) gene and the Kusabira-Orange (KO) gene are loaded on a single common vector; and the other case where the two genes are loaded on respective viral vectors, individually. The result shows that it is necessary to load a plurality of types of genes on a single common vector in order to allow exogenous genes to be expressed at a constant ratio with excellent reproducibility (Example 15). It was also ascertained that all of the reprogramming genes have to be loaded on a single common vector in order to induce iPS marker-expressing cell with higher efficiency (Example 16).

In the reprogramming gene-loaded Sendai virus vector of the present invention, all of the reprogramming genes are loaded on a single common vector, so that, when the vector of the present invention is used, emergence efficiency of an iPS cell (reprogramming efficiency) is extremely high. For example, in cases where Oct3/4, Sox2, Klf4 and c-Myc are loaded thereon, the efficiency rate reaches up to 16.8% (Example 5, Example 8). In contrast, in cases where an adenoviral vector is used, a mature mouse liver cell can be reprogrammed just at a rate of 0.0005% or less. Even using an EBV vector which has heretofore been reported as the only vector capable of establishing human iPS cells including no exogenous gene, the efficiency rate is just in the range of about 0.0003 to 0.0006%.

When the plurality of types of reprogramming genes are loaded on a single common vector and always expressed at a constant expression ratio, resulting established pluripotent stem cells exhibit quite uniform properties. For example, in an analysis of human pluripotent stem cells established according to the present invention by a DNA chip method, a correlation coefficient between respective ones of four different cell lines was determined to be 0.98 or more in any case (Example 19). This contrasts with the fact that the gene expression pattern in pluripotent stem cells established using a retroviral vector is generally quite non-uniform, and a correlation coefficient between cell lines is 0.95 or less in many cases (Reference: Chin, et al., Cell Stem Cells, 5, 111-123,2009).

As above, it became evident that, based on the use of the reprogramming gene-loaded Sendai virus vector of the present invention where four types of reprogramming genes are loaded on a single common vector, pluripotent stem cells having uniform properties can be established with significantly high efficiency while constantly ensuring excellent reproducibility.

### [REMOVAL OF REPROGRAMMING GENES]

The reprogramming gene-loaded Sendai virus vector of the present invention is transfected into a differentiated cell in an infectious manner, and the reprogramming genes are stably expressed in the cytoplasm of the cell to reprogram the cell. In order to make the genetic information of the reprogrammed cell identical to that of the original or pre-programming cell, the reprogramming genes which have become unneeded have to be removed from the cell. In the present invention, the entire reprogramming gene-loaded Sendai virus vector is removed using a siRNA. The siRNA is designed to target the L gene of the Sendai virus vector. According to experimental results by the inventors, the reprogramming gene-loaded Sendai virus vector can be completely removed by targeting the L gene, although the reprogramming gene-loaded Sendai virus vector can also be removed to some extent by targeting the NP gene or the P gene. For example, a target region of the L gene may be a region including a portion encoding a nucleotide residue of the L protein at position 527 or 1913. The target region is not particularly limited to such a region, buy may be any other suitable region. The siRNA is transfected into the cell 5 to 20 days after infecting the differentiated cell with the reprogramming gene-loaded Sendai virus vector.

It is contemplatable that microRNA (miRNA) existing in a cell may be used instead of siRNA. miRNA is a small RNA transcribed from the genome of an animal cell, and capable of interacting with DNA, mRNA and protein to adjust the functions thereof. In then interaction with mRNA, there exists a mechanism where the miRNA binds to a target sequence on the mRNA to induce decomposition of the mRNA or suppress translation of the mRNA to thereby suppress gene expression. It is known that a target sequence for a specific miRNA is artificially inserted into a protein-noncoding region of mRNA transcribed from certain gene, to allow expression of the gene to be suppressed in a cell where the miRNA is expressed, based on the above mechanism. Thus, a target sequence for miRNA appearing only in a reprogrammed cell may be added to an L, NP or P gene-noncoding region of the Sendai virus vector. In this case, expression of the L, NP or P gene can be suppressed based on the above mechanism, in the same manner as that based on the siRNA, so as to remove the reprogramming gene-loaded Sendai virus vector.

The miRNA to be used for the above purpose may include, but is not limited to, mir-302a that is specifically expressed, for example, in human or mouse ES cells. For example, the technique of removing the reprogramming gene-loaded Sendai virus vector using miRNA has an advantage of being able to automatically remove the Sendai virus vector without the need for externally transfecting siRNA. Further, in a human cell, the removal of the vector can also be promoted by means of culture at a high temperature (40 °C).

As above, the reprogramming gene-loaded Sendai virus vector can be removed by using siRNA that targets the L gene, or by culture at high temperatures, or through the use of a Sendai virus vector in which a target sequence for miRNA is added to the noncoding region of the L, NP or P genes. Thus, a resulting iPS cell does not have the exogenous genes inserted into the chromosome, so that it has genomic information identical to that of a donor individual of the differentiated cell. Further, it has a long-term self-renewal capability in vitro.

Various examples of the present invention will be described below. It is understood that the present invention is not limited to the following examples.

### [EXAMPLES]

### EXAMPLE 1: Preparation of Cells for reconstructing Sustained Expression-Type Sendai virus vectors

A cDNA (SEQ ID NO: 1 in the following Sequence Table) encoding T7 RNA polymerase where codons are optimized to improve expression efficiency in an animal cell, was cloned into a plasmid pCX4SRalpha-neo vector for preparing a retroviral vector. A cDNA encoding Sendai virus strain Cl.151 M protein was first cloned into a plasmid pCX4SRalpha-puro vector for preparing a retroviral vector. The plasmid DNAs were then introduced into respective PLAT-E packaging cells using Lipofectamine 2000, and retroviruses (T7 RNA polymerase recombinant retrovirus and 151M recombinant retrovirus) were obtained from a culture supernatant. The T7 RNA polymerase recombinant retrovirus was transfected into BHK-21 cells. The infected BHK-21 cells were then transferred to Dulbecco's Modified Minimal Essential Medium (DMEM) containing 800 µg/ml of G418 and 10% of fetal bovine serum (FCS), and G418-resistant cells (BHK/T7 (SE)) which stably express T7 RNA polymerase were isolated. Subsequently, the 151M recombinant retrovirus was transfected into BHK/T7(SE) cells and the infected BHK/T7 (SE) cells were transferred to a DMEM containing 800 µg/ml of G418, 15 µg/ml of puromycin and 10% of FCS, and G418 + puromycin-resistant cells (BHK/T7/151M (SE)) which stably express T7 RNA polymerase and an M protein were isolated.

### EXAMPLE 2: Preparation of hOct4/hSox2/hKlf4 Sustained Expression-Inducing Sendai virus vector

### (1) Construction of Template cDNA for Preparing Vector

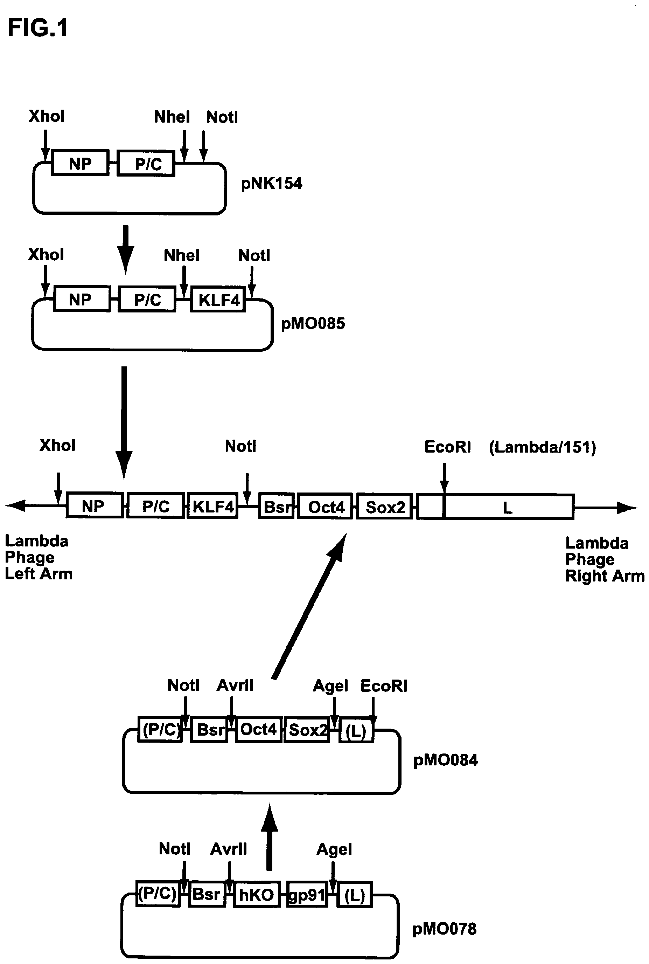

A double-stranded DNA (SEQ ID NO: 2 in the Sequence Table) including Avr II recognition sequence, human Oct4 ORF, Sendai virus (SeV) genome cDNA (bases 6617 to 6666), human Sox2 ORF and Age I recognition sequence in this order was synthesized, and then cloned into the plasmid vector pUC57 (the cloning was entrusted to GenScript Inc.) (pUC57-OctSox). A DNA sequence cut from the pUC57-OctSox at Avr II and Age I sites was inserted between Arv II and Age I sites of a plasmid vector pMO078 (SEQ ID NO: 3 in the Sequence Table) where Cla I recognition sequence, SeV strain Cl.151 genome cDNA (bases 2871 to 3650), Not I recognition sequence, blasticidin S-resistance gene, Mlu I recognition sequence, SeV strain Cl.151 genome cDNA (bases 4728 to 4828), Avr II recognition sequence, humanized Kusabira-Orange gene, SeV strain Cl.151 genome cDNA (bases 6617 to 6666), gp91phox gene, Age I recognition sequence and SeV strain Cl.151 genome cDNA (bases 8442 to 10479) were inserted into a plasmid pBluescript II SK(+) (Agilent Technologies Inc.) in this order. In this manner, a plasmid pMO084 was obtained (FIG. 1).

A double-stranded DNA (SEQ ID NO: 4 in the Sequence Table) including Nhe I recognition sequence, human Klf4 ORF, Sendai virus transcription termination sequence, Sendai virus transcription initiation sequence and Not I recognition sequence in this order was synthesized, and then cloned into a plasmid vector pUC57 (the cloning was entrusted to GenScript Inc.) (pUC57-KLF4). A DNA sequence cut from the pUC57-KLF4 at Nhe I and Not I sites was inserted between Nhe I and Not I sites of a plasmid vector pNK154 (SEQ ID NO: 5 in the Sequence Table) where SeV strain Nagoya genome cDNA (bases 1 to 2871), SeV strain Cl.151 genome cDNA (bases 2872 to 3656), Nhe I recognition sequence and Not I recognition sequence are inserted into pBluescript II SK(+) (Agilent Technologies Inc.) in this order. In this manner, a plasmid pMO085 was obtained (FIG. 1).

A DNA fragment (including a T7 promoter sequence, a SeV genome cDNA (bases 1 to 3655) and a human Klf4 cDNA) cut from the pMO085 at restriction endonucleases Xho I and Not I, a DNA fragment (including human Oct4 and human Sox2 cDNAs) cut from the pMO084 at restriction endonucleases Not I and EcoR I, and a DNA fragment (including a cDNA complementary to bases 10480 to 15384 in the SeV genome, and a right arm in a λDASH II) cut from a phage genome DNA of λ/151 (lambda phage vector cloned with a full-length SeV strain Cl.151 genome cDNA: Nishimura, et al., J. Biol. Chem., 282, 27383-27391, 2007) at EcoR I site, were then combined together, and cloned into a lambda phage vector λDASH II to prepare λ/SeVp (Mp + Klf4, +M:: Bsr, +F:: Oct4, +HN:: Sox2) (FIG. 1) (a cDNA complementary to a full-length genome of a hOct4/hSox2/hKlf4 sustained expression-inducing Sendai virus vector is described as SEQ ID NO: 6 in the Sequence Table).

### (2) Preparation of hOct4/hSox2/hKlf4 Sustained Expression-Inducing Sendai virus vector

BHK/T7/151M (SE) cells were seeded on a 6-well plate at a density of 5 × 10⁵ cells/well, and washed after cultivation for 24 hours. A λ/SeVp (Mp + Klf4, +M:: Bsr, +F:: Oct4, +HN:: Sox2) phage DNA, an NP protein-expression plasmid pGEM/NP, a P protein-expression plasmid pGEM/P, an L protein-expression plasmid pGEM/L, an F protein-expression plasmid pSRD-FZmut and an HN protein-expression plasmid pMKIT-NaHN were suspended in 300 µL of Opti-MEM, respectively, at quantitative ratios of 2 µg, 1 µg, 1 µg, 1 µg, 1 µg and 1 µg, and the obtained suspension was mixed with 300 µL of Opti-MEM containing 10 µL of Lipofectamine 2000. After leaving the mixture at room temperatures for 20 minutes, it was added to the cells, and the cells were cultured for 4 hours. Then, the cells were washed, and after adding DMEM containing 10% of FCS, further cultured at 32°C for 3 days. Then, the cells were transferred to DMEM containing 10% of FCS and 10 µg of blastcidin S. Blastcidin-resistant cells were then isolated as hOct4/hSox2/hKlf4 sustained expression-inducing Sendai virus vector-producing cells (BHK/T7/151M/KBOS). The occurrence of reconstruction of a vector genome was ascertained by a fluorescent antibody method using antibody against Sendai virus NP protein and antibodies to hOct4/hSox2/hKlf4 gene products.

2 µg each of pMKIT-151M, pSRD-ZFmut and pMKIT/NaHN as defective gene-expression plasmids were transfected into 5.0 × 10⁵ BHK/T7/151M/KBOS cells using Lipofectamine 2000. After 4 hours, the cells were washed, and, after adding DMEM containing 10% of FCS thereto, further cultured at 32°C for 4 days. Culture supernatant containing a hOct4/hSox2/hKlf4 sustained expression-inducing Sendai virus vector was then harvested. The culture supernatant was filtered through a 0.45 µm filter, and ultracentrifuged as needed to concentrate the vector. The vector suspension was quickly frozen using liquid nitrogen, and cryopreserved at - 80°C.

### EXAMPLE 3: Preparation of hOct4/hSox2/hKlf4/hc-Myc Sustained Expression-Inducing Sendai virus vector

### (1) Preparation of Vector cDNA

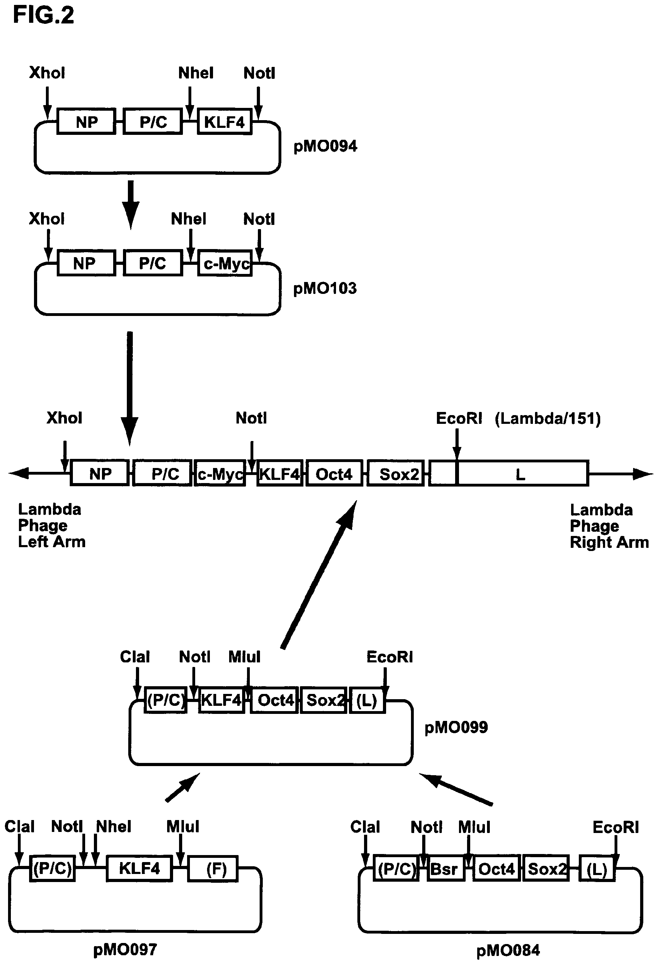

A human Klf4 gene was amplified from the pUC57-KLF4 by a PCR method using two primers consisting of 5'-ACTAGCTAGCAGTCTGACATGGCTGTCAGCGACGCGCT-3' (SEQ ID NO: 7 in the Sequence Table (N-terminal side)) and 5'-GGTCCACGCGTTTAAAAA TGCCTCTTCATGTG-3' (SEQ ID NO: 8 in the Sequence Table (C-terminal side)) as hKlf4 gene-amplifying primers. The termini of the obtained double-stranded DNA were cut at Nhe I and Mlu I sites, and inserted between Nhe I and Mlu I sites of pMO026 (SEQ ID NO: 9 in the Sequence Table) (a plasmid vector where Cla I recognition sequence, SeV strain Cl.151 genome cDNA (bases 2871 to 3650), Not I recognition sequence, Nhe I recognition sequence, blasticidin S-resistance gene, Mlu I recognition sequence and SeV strain Cl.151 genome cDNA (bases 4728 to 5335) were inserted into pBluescript II SK(+)). In this manner, pMO097 was obtained. Furthermore, a fragment between Cla I and Mlu I sites of the pMO097 was combined with a fragment between Cla I and Mlu I sites of the pMO084 to obtain pMO099 (FIG. 2).

A human c-Myc gene was amplified from a plasmid pJL1 including a full-length human c-Myc cDNA by a PCR method using two primers consisting of 5'-ACTAGCTAGCTTAGA CGCTGGATTTTTTTCGGGTAGTGG-3' (SEQ ID NO: 10 in the Sequence Table (N-terminal side)) and 5'-GTCCGACGTCCTTACGCACAAGAGTTCCGT-3' (SEQ ID NO: 11 in the Sequence Table (C-terminal side)) as hc-Myc gene-amplifying primers. The termini of the double-stranded DNA were cut at Nhe I and Aat II sites, and inserted between Nhe I and Aat II sites of pMO094 (SEQ ID NO: 12 in the Sequence Table) (a plasmid vector where an SeV strain Nagoya genome cDNA (bases 1 to 43), Sendai virus transcription termination sequence, SeV strain Nagoya genome cDNA (bases 56 to 2870), SeV strain Cl.151 genome cDNA (bases 2871 to 3656), Nhe I recognition sequence, human Klf4 ORF, Aat II recognition sequence, Sendai virus transcription termination sequence, Sendai virus transcription initiation sequence and Not I recognition sequence were inserted into pBluescript II SK(+) (Agilent Technologies Inc.)). In this manner, pMO103 was obtained (FIG. 2).

Based on the plasmids obtained as described above, the T7 promoter sequence and DNA fragment SeV (1 to 3655), and DNA fragment SeV (3655 to 10480), were cut out from pMO103 and pMO099, respectively, and combined with a DNA fragment of SeV (10480 to 15384) + the right arm of the λDASH II obtained by cutting the λ/151 at the EcoR I site. Then, the combination was cloned to prepare λ/SeVp (Mp + myc, +M:: Klf4, +F:: Oct4, +HN:: Sox2) (FIG. 2) (a cDNA complementary to a full-length genome of a hc-Myc/hOct4/hSox2/hKlf4 sustained expression-inducing Sendai virus vector is described as SEQ ID NO: 13 in the Sequence Table).

### (2) Preparation of hOct4/hSox2/hKlf4/hc-Myc Sustained Expression-Inducing Sendai virus vector

The BHK/T7/151M (SE) cells were seeded on a 6-well plate at a density of 5 × 10⁵ cells/well, and, after culture for 24 hours, the cells were washed. A λ/SeVp (Mp + myc, +M:: Klf4, +F:: Oct4, +HN:: Sox2) phage DNA, an NP protein-expression plasmid pGEM/NP, a P protein-expression plasmid pGEM/P, an L protein-expression plasmid pGEM/L, an F protein-expression plasmid pSRD-FZmut and an HN protein-expression plasmid pMKIT-NaHN were suspended in 300 µL of Opti-MEM, respectively, at quantitative ratios of 2 µg, 1 µg, 1 µg, 1 µg, 1 µg and 1 µg, and the obtained suspension was mixed with 300 µL of Opti-MEM containing 10 µL of Lipofectamine 2000. After leaving the culture medium at room temperatures for 20 minutes, the culture medium was added to the cells, and the cells were cultured for 4 hours. The cells were washed and, after adding DMEM containing 10% of FCS, further cultured at 32°C for 3 days and 37°C for another 3 days. Cells were then stained by the fluorescent antibody method using antibody against Sendai virus NP protein and antibodies to the hOct4/hSox2/hKlf4 gene products, to detect the reconstruction of the vector genome in the cells. The cell population was used as hOct4/hSox2/hKlf4/hc-Myc sustained expression-inducing Sendai virus vector-producing cells without further cloning.

2 µg each of pMKIT-151M, pSRD-ZFmut and pMKIT/NaHN as defective gene-expression plasmids were introduced into 5.0 × 10⁵ hOct4/hSox2/hKlf4/hc-Myc sustained expression-inducing Sendai virus vector-producing cells using Lipofectamine 2000. After 4 hours, the cells were washed, and, after adding DMEM containing 10% of FCS, further cultured at 32°C for 4 to 9 days. The culture supernatant containing hOct4/hSox2/hKlf4/hc-Myc sustained expression- inducing Sendai virus vector was then harvested. The culture supernatant was filtered through a 0.45 µm filter, and ultracentrifuged as needed to concentrate the vector. The vector suspension was quickly frozen using liquid nitrogen, and cryopreserved at - 80°C.

### EXAMPLE 4: Removal of the Sustained Expression-Type Sendai Virus Vector from Cells Using siRNA

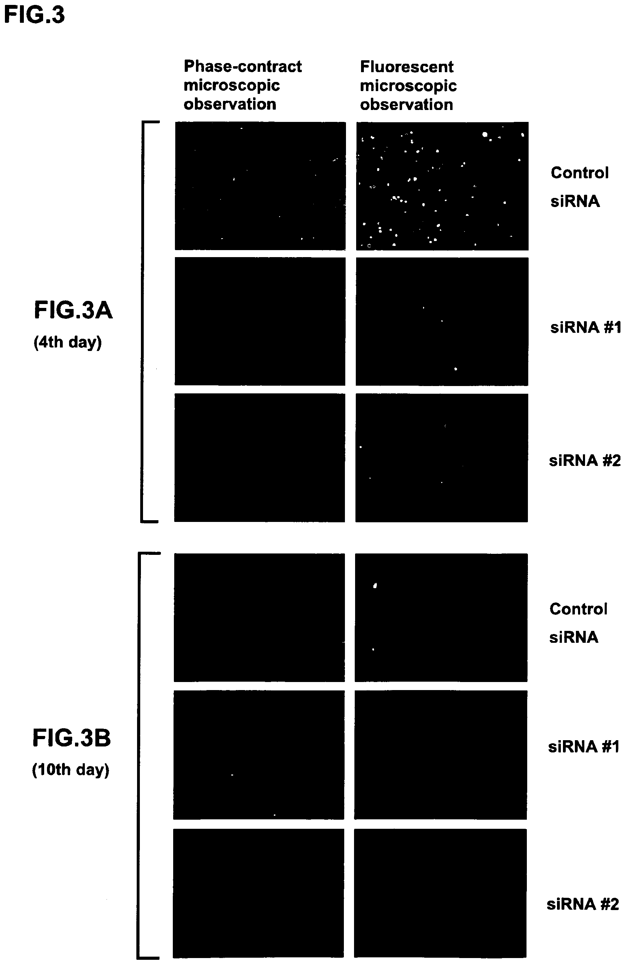

In order to remove the RNA genome of the sustained expression-type Sendai virus vector from the cells stably retaining the RNA genome, two types of short interfering RNAs (siRNAs) were prepared to suppress expression of the L gene encoding a subunit of RNA-dependent RNA polymerase that is necessary for sustained infection of the vector (#1: sense strand 5'-GGUUCAGCAUCAAAUAUGAAG-3' (SEQ ID NO: 14 in the Sequence Table), antisense strand 5'-UCAUAUUUGAUGCUGAACCAU-3' (SEQ ID NO: 15 in the Sequence Table), #2: sense strand 5'-GGUCCAGACAUGAAUUCAAAG-3' (SEQ ID NO: 16 in the Sequence Table), antisense strand 5'-UUGAAUUCAUGUCUGGACCAU-3' (SEQ ID NO: 17 in the Sequence Table)). In order to check a vector genome removing effect by the siRNA, BHK/T7 cells stably retaining the sustained expression-type Sendai virus vector loaded with an aequorea victoria-derived EGFP gene (Enhanced Green Fluorescent Protein gene: Clontech Laboratories Inc.) were seeded onto a 48-well plate at a density of 1.0 × 10⁴ cells/well. The next day, the siRNA targeting the L gene was transfected into the cells to have a final concentration of 100 nM. After 4 days post transfection, EGFP fluorescence was observed by fluorescence microscopy. As a result, it was proven that the intensity of EGFP fluorescence in the cell transfected with the siRNA targeting the L gene was greatly reduced, as compared to a cell transfected with siRNA targeting a firefly luciferase gene used as a negative control (FIG. 3A).

Moreover, some of the cells treated with the siRNA were re-seeded on a 12-well plate, and cultured for another 6 days. As a result, no EGFP fluorescence was detected in most all of the cells. This shows that the reduction in intensity of EGFP fluorescence is not caused by transient suppression of gene expression, but by removal of the RNA genome of the vector from the cells (FIG. 3B).

### EXAMPLE 5: Induction of Cells Expressing Mouse iPS Marker from Mouse Embryo-Derived Fibroblast Cells

### (1) Preparation of Mouse Embryo-Derived Fibroblast Cells

An embryo was removed from a mouse (C57BL/6J or Nanog-EGFP (Enhanced Green Fluorescent Protein) knock-in mouse (STOCK Tg (Nanog-GFP, Puro) 1Yam) at the 14th day of pregnancy. After removing the head, four limbs and internal organs, the remaining body parts were chopped up, and treated with trypLE Express (Invitrogen Corp.) at 37°C for 30 minutes. After precipitating non-cellular components, the cells in the supernatant were cultured in Dulbecco's Modified Minimal Essential Medium (DMEM) containing 10% of fetal bovine serum (FCS) to obtain mouse embryo-derived fibroblast (MEF) cells.

### (2) Induction of the Cells Expressing Mouse iPS Markers

The MEF cells were cultured in a 12-well plate at a density of 1.0 × 10⁵ cells/well. The next day, each of the hOct4/hSox2/hKlf4 sustained expression-inducing Sendai virus vector prepared in Example 2, the hOct4/hSox2/hKlf4/hc-Myc sustained expression-inducing Sendai virus vector prepared in Example 3 and the hOct4/hSox2/hKlf4/hc-Myc sustained expression-inducing Sendai virus vector Version 2 prepared in Example 12 were added to culture medium to infect the MEF cells at room temperature for 2 hours, and then the infected MEF cells were cultured at 37°C overnight. Mitomycin C-treated MEF cells were plated on a gelatin-coated dish, as feeder cells. The vector-infected cells were seeded thereon, and then cultured in mouse ES medium (DMEM, 15% FCS, 0.1 mM nonessential amino acids, 0.55 mM 2-ME, 1000 U/ml Leukemia Inhibitory Factor (LIF)) or KSR medium (Knockout DMEM, 15% Knockout Serum Replacement (KSR), 2 mM Glutamine, 0.1 mM nonessential amino acids, 0.05 mM 2-ME, 1000 U/ml Leukemia Inhibitory Factor (LIF)).

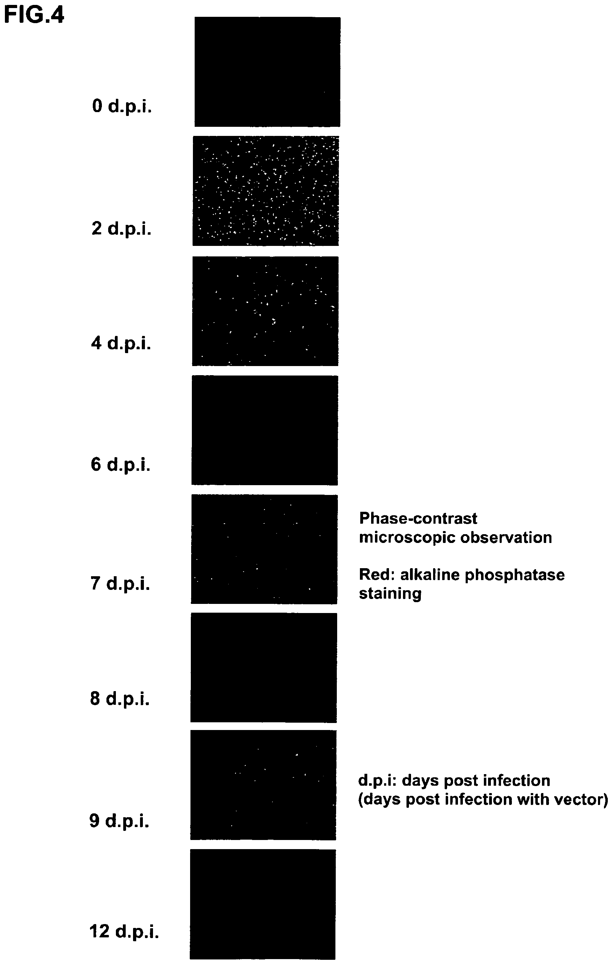

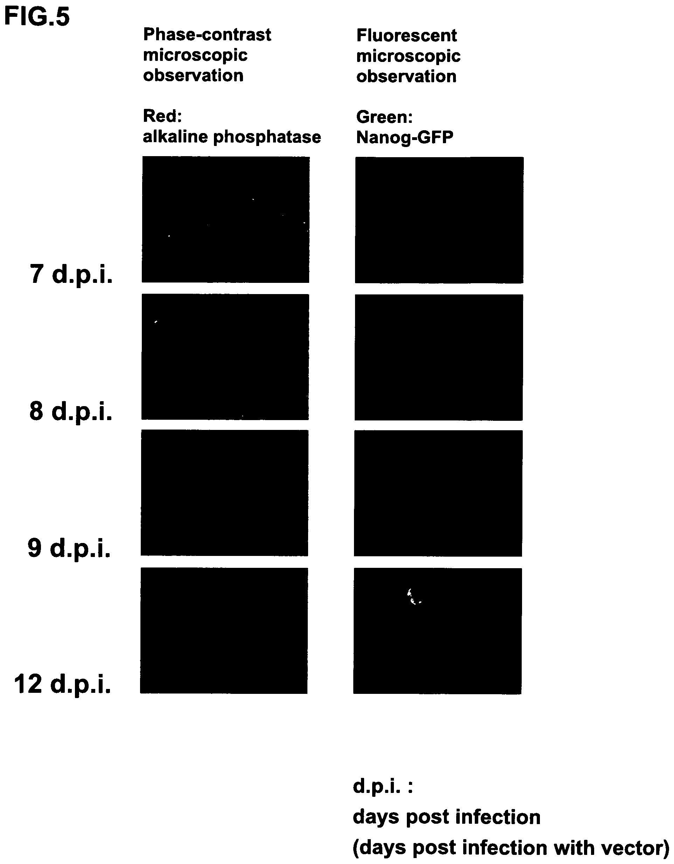

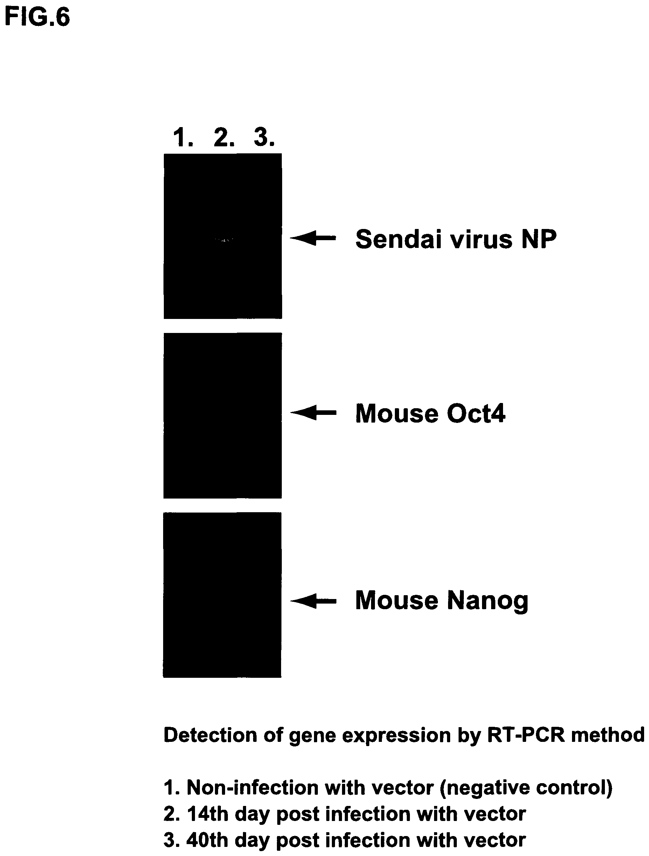

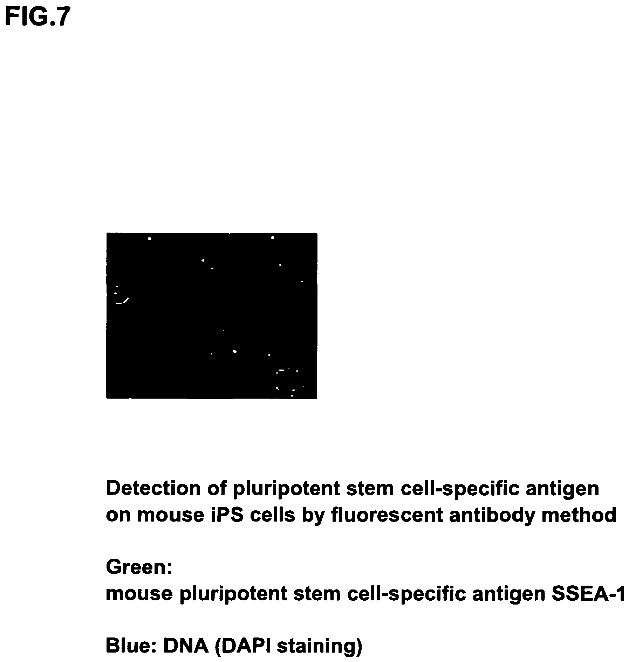

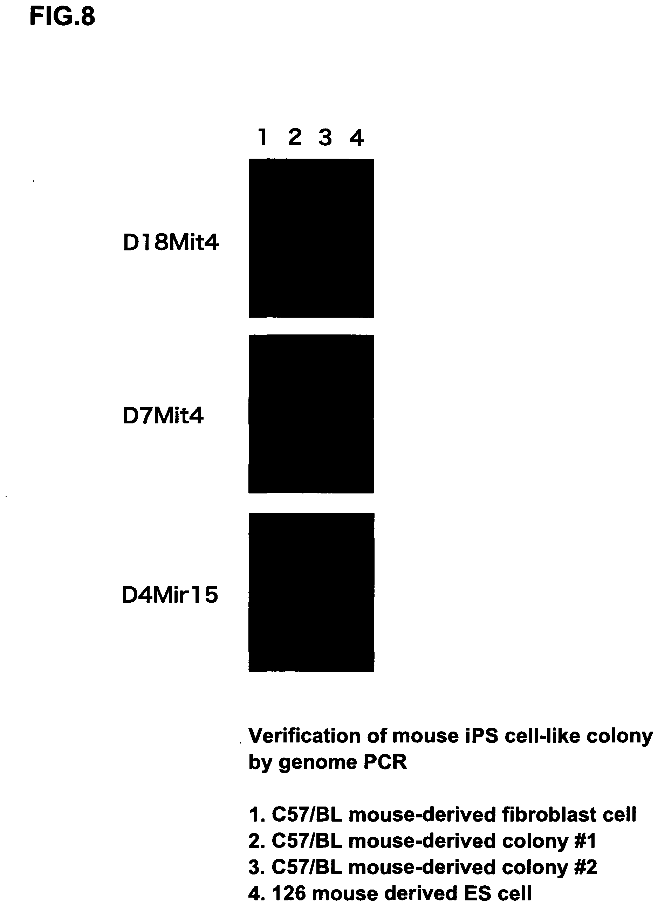

6 days after infection with the hOct4/hSox2/hKlf4/hc-Myc sustained expression-inducing Sendai virus vector, the formation of mouse ES-like cell colonies stained positively for alkaline phosphatase activity ((b) described below) was detected (FIG. 4). In those MEF cells derived from Nanog-EGFP knock-in mice, GFP-positive colonies were detected 8 days post-infection indicating induction of expression from the endogenous Nanog gene (FIG. 5). RT-PCR analysis ((c) described below) further showed that mouse Nanog and Oct4 (markers of a mouse iPS cell) are induced in cells forming iPS colonies (FIG. 6). Using the fluorescent antibody method ((a) described below), mouse SSEA-1 (maker of a mouse iPS cell) was detected in cells forming the iPS colonies (FIG. 7). Genotyping ((d) described below) demonstrated that a genotype of the induced mouse iPS marker-expressing cell is identical to that of the MEF cell used in the initial gene transfection but different from that of the mouse ES cell used as a positive control. Hence, these results demonstrate that the mouse iPS marker-expressing cells were generated by introducing the four genes consisting of hOct4, hSox2, hKlf4 and hc-Myc into the MEF cells (FIG. 8). Substantially the same result was obtained with hOct4/hSox2/ hKlf4/hc-Myc sustained expression-inducing Sendai virus vector Version 2.

### (3) Test for Induction Efficiency of Mouse iPS Marker-Expressing Cells

The proportion of cells infected with the sustained expression-type Sendai virus vector was quantitatively measured using the fluorescent antibody method against the NP (the (a)), and the number of alkaline phosphatase activity-positive colonies (the (b)) was corrected for infection efficiency to calculate the induction efficiency of mouse iPS marker-expressing cells. The result is illustrated in Table 1.

**TABLE 1: Time-series observation of the emergence frequency of alkaline phosphatase -expressing cell in mouse embryo fibroblast cells infected with hOct4, hSox2, hKlf4, and hc-Myc sustained expression-inducing Sendai virus vector**

| Time (days) after infection | Frequency with respect to all cells (%) | Frequency with respect to infected cells (%) |
|---|---|---|
| 6 | 5.3 | 9.1 |
| 7 | 8.6 | 14.9 |
| 9 | 13.1 | 22.5 |
| 12 | 7.1 | 12.3 |

As seen in the result of Table 1, it became evident that the mouse iPS marker-expressing cells can be induced with significantly higher efficiency than previous reports on iPS cell production in which four genes consisting of hOct4, hSox2, hKlf4 and hc-Myc were transfected using a retroviral vector or the like. Substantially the same result was obtained with the hOct4/hSox2/hKlf4/hc-Myc sustained expression-inducing Sendai virus vector Version 2.

### EXAMPLE 6: Production of Mouse iPS Cells by Removal of RNA Genome of Sustained Expression-Type Sendai Virus Vector

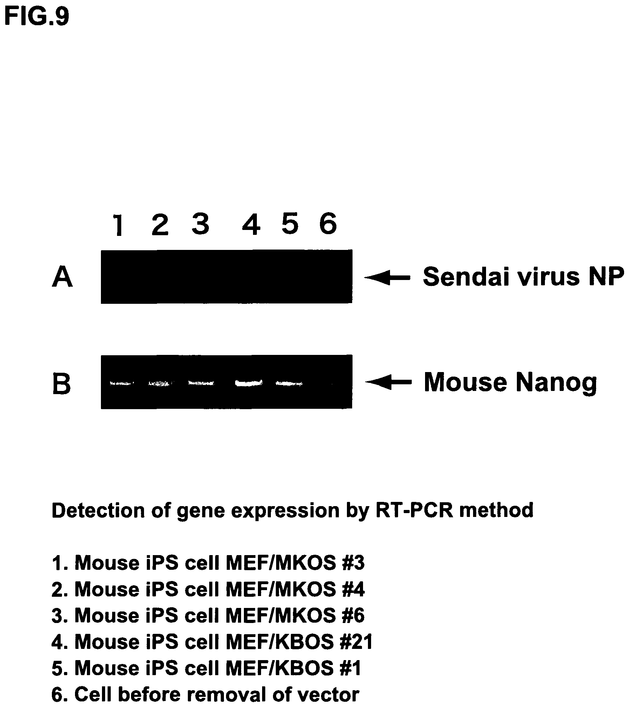

In order to remove the genomic RNA of the vector from the mouse iPS marker-expressing cells obtained in Example 5, the siRNA targeting the L gene was transfected into the cells as described in Example 4. In each of the first and subsequent subcultures, the siRNA was added to culture medium in the form of a mixture with lipofectamin 2000. After about one week from the transfection, the presence of a colony in which no vector RNA genome remains in the cells was detected by fluorescent antibody staining (the (a)) to Sendai virus NP protein, as described in Example 5. Furthermore, the colony was cloned, and a mouse iPS cell clone devoid of any vector genome was obtained while checking the cloned colony by RT-PCR analysis (the (c)) for detecting NP gene-derived messenger RNA (mRNA) (FIG. 9A). Moreover, the expression of mouse Nanog and Oct4 genes was evaluated by RT-PCR analysis (the (c)). As a result, it was ascertained that the expression of the iPS cell markers is stably maintained even after the removal of the vector RNA (FIG. 9B).

### EXAMPLE 7: Formation of Teratomas from Mouse iPS Cells

The mouse iPS cells obtained in Example 6 were adjusted to a concentration of 1.0 × 10⁶ cells/100 µL PBS/mouse, and transplanted under a skin at the root of a leg of a mouse (C. B17/Icr-scidJcl) sedated using isoflurane anesthesia. 2 weeks after the inoculation, a visually identifiable teratoma was formed. 30 days after the implantation, the teratoma was excised and fixed in Bouin's fixative solution (75% of saturated picric acid, 12% of formalin, 3% of acetic acid), and dehydrated by treatment with 70% ethanol solution (1 hour), 90% ethanol solution (1 hour), 100% ethanol solution (1 hour, twice), 50% ethanol solution, 50% 2-butanol solution (1 hour) and 100% 2-butanol solution (30 minutes, twice). Samples were then fixed in paraffin, and a slice having a thickness of 6 µm was prepared using a microtome. After deparaffinization, the slice was subjected to HE staining observation. As a result, differentiation into tissues of all three germ layers was observed (FIG. 9).

### EXAMPLE 8: Induction of Cells Expressing Human iPS Marker from Human Embryo-Derived Fibroblast Cells

### (1) Induction of Human iPS Marker-Expressing Cells

TIG3 cells, i.e. human embryo-derived fibroblast cells, were cultured in a 12-well plate at a density of 10 × 10⁵ cells/well. After one day, each of the hOct4/hSox2/hKlf4/hc-Myc sustained expression-inducing Sendai virus vector (prepared in Example 4) and the hOct4/hSox2/hKlf4 /hc-Myc sustained expression-inducing Sendai virus vector Version 2 (prepared in Example 12) were added to culture medium, and left at room temperature for 2 hours. The cells were then cultured overnight at 37°C, and infected. Mitomycin C-treated MEF cells were plated on a gelatin-coated dish, as feeder cells. The vector-infected cells were seeded thereon, and then cultured in hES medium (DMEM/F12, 20% of Knockout Serum Replacement (KSR), 0.1 mM nonessential amino acids, 0.55 mM 2-ME, 10 ng/ml bFGF) or primate ES cell culture medium (ReproCELL).

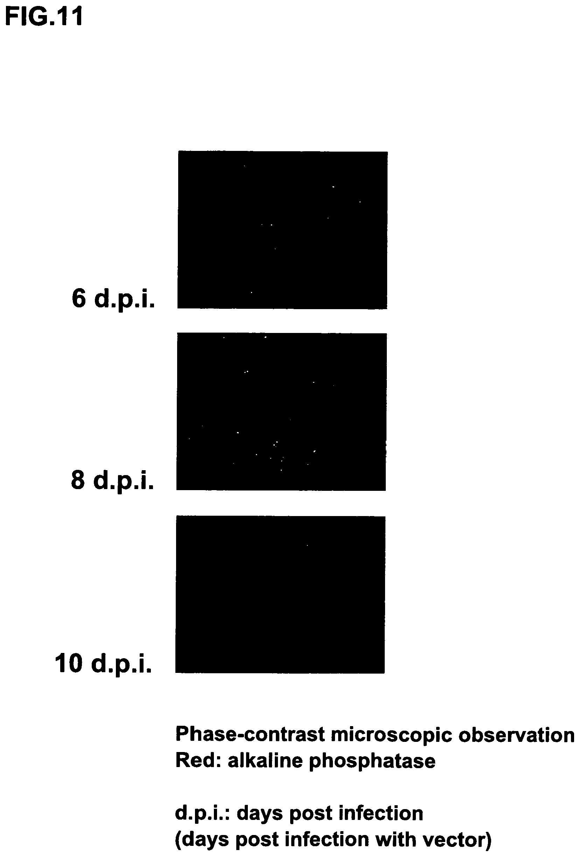

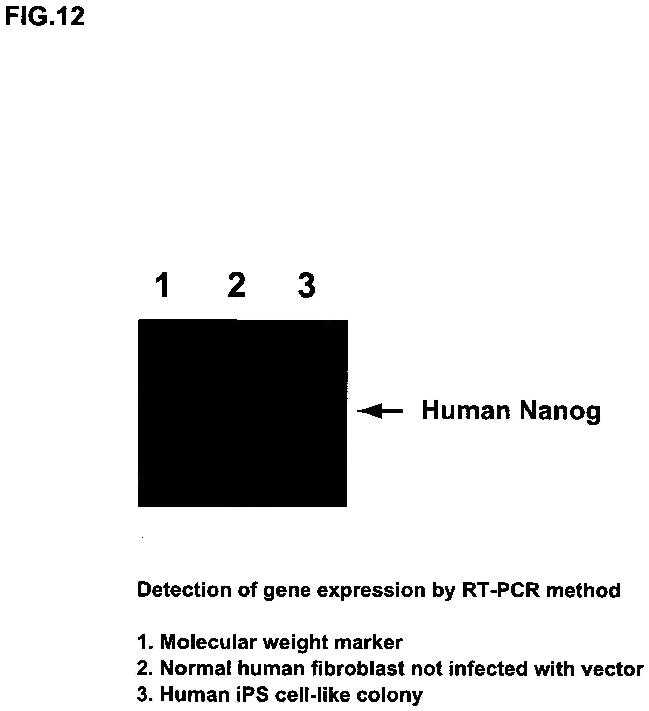

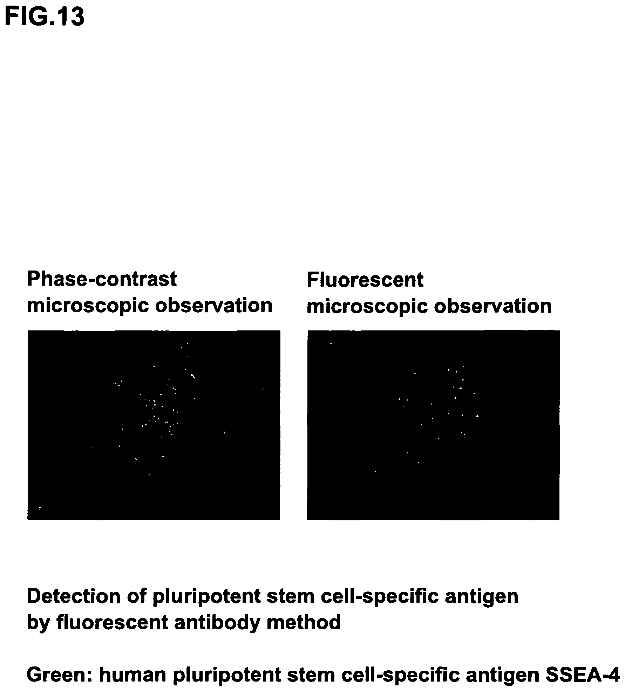

10 days after the infection with the hOct4/hSox2/hKlf4/hc-Myc sustained expression-inducing Sendai virus vector, the formation of human ES-like cell colonies was detected, and alkaline phosphatase activity (the (b)) was detected (FIG. 11). RT-PCR analysis (the (c)) further showed that human Nanog is induced in cells forming the colonies (FIG. 12). Using the fluorescent antibody method (the (a)), it was ascertained that SSEA-4 as a marker of a human iPS cell is induced in cells forming the colonies (FIG. 13). Substantially the same results were obtained using the hOct4/hSox2/ hKlf4/hc-Myc sustained expression-inducing Sendai virus vector Version 2.

### (2) Test for Induction Efficiency of Human iPS Marker-Expressing Cells

A vector genomic RNA retention rate in a cell transfected with the sustained expression-type Sendai virus vector was quantitatively determined by the fluorescent antibody method, and the number of cells in an alkaline phosphatase activity-positive colony was corrected by the vector retention rate, so as to calculate an induction efficiency rate of human iPS marker-expressing cells. The results are illustrated in Table 2.

**TABLE 2: Time-series observation of emergence frequency of alkaline phosphatase-expressing cells in human embryo fibroblast cells infected with hOct4/hSox2/hKlf4/hc-Myc sustained expression-inducing Sendai virus vector**

| Time (days) after infection | Frequency with respect to all cells (%) | Frequency with respect to infected cells (%) |
|---|---|---|
| 6 | 2.7 | 10.2 |
| 8 | 3.7 | 13.9 |
| 10 | 4.4 | 16.8 |

From the result in Table 2, it became evident that the human iPS marker-expressing cells can be induced with significantly higher efficiency than previous reports on iPS cell production in which four genes consisting of hOct4, hSox2, hKlf4 and hc-Myc were transfected using a retroviral vector. Substantially the same result was obtained with the hOct4/hSox2/hKlf4/hc-Myc sustained expression-inducing Sendai virus vector Version 2.

### (3) Change in Human iPS Marker-Expressing Cell Induction Efficiency due to Difference in Culture Conditions

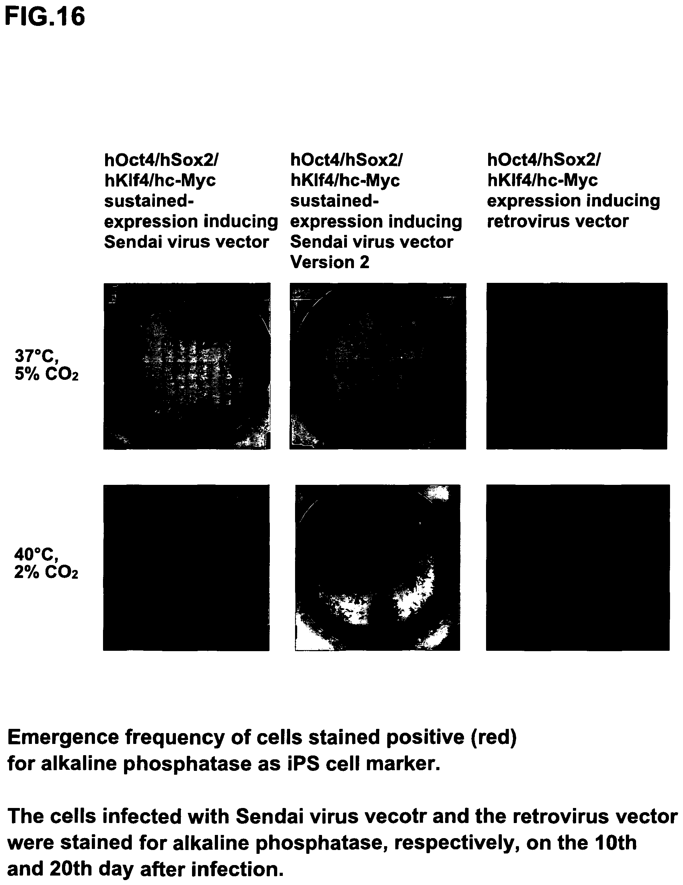

The inventors found that, when an iPS cell is established from human embryo-derived fibroblast cells, an efficiency rate of the establishment is increased about ten times by performing the culturing at 40°C and in 2% CO₂, instead of conventional cell culture conditions of 37°C in 5% CO₂. This phenomenon was commonly observed in the hOct4/hSox2/hKlf4/hc-Myc sustained expression-inducing Sendai virus vector and the hOct4/hSox2/hKlf4/hc-Myc sustained expression-inducing Sendai virus vector Version 2, whereas it was not observed in a retrovirus vector (FIG. 16).

### EXAMPLE 9: Production of Human iPS Cells by Removing RNA Genome of Sustained Expression-Type Sendai Virus Vector

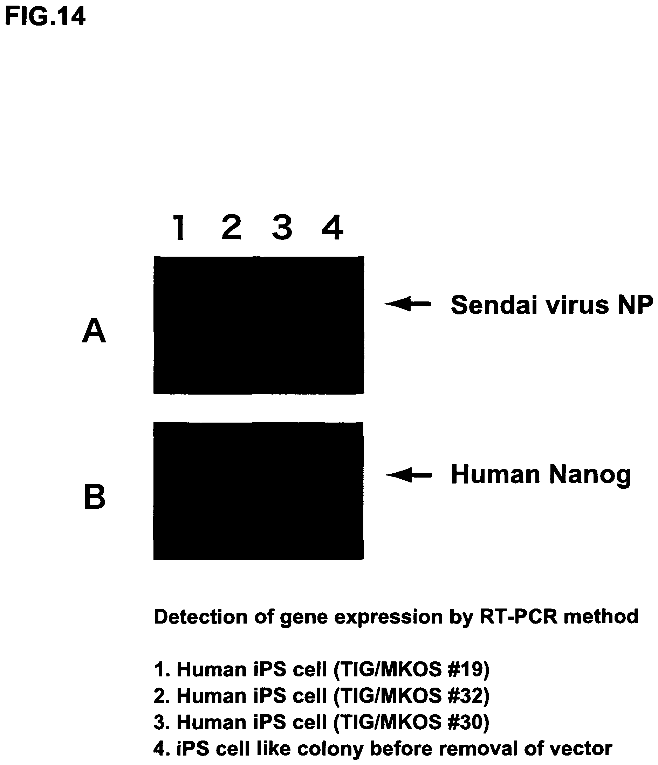

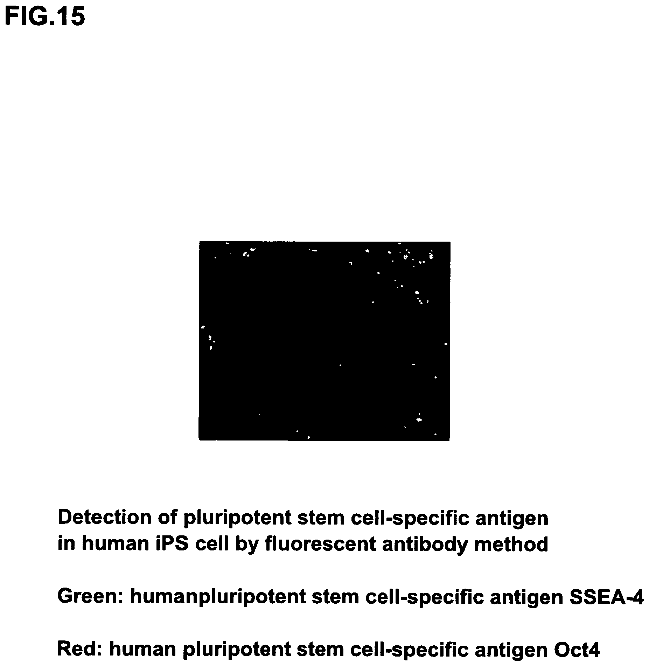

The human iPS marker-expressing cells obtained in Example 8 were successively cultured. One month after the infection with the vector, the presence of a colony in which no vector RNA genome remains in the cells was detected by fluorescent antibody staining to Sendai virus NP protein, as described in Example 5. Furthermore, the colony was cloned, and a human iPS cell clone devoid of any vector genome was obtained while checking the cloned colony by the RT-PCR analysis for detecting NP gene-derived messenger RNA (mRNA) as described in Example 7 (FIG. 14A). Based on the RT-PCR analysis (the (c)), it was also ascertained that the expression of human Nanog as a marker of an iPS cell is stably maintained even after the removal of the vector RNA (FIG. 14B). Additionally, based on the fluorescent antibody method (the (a)), it was ascertained that the expression of human SSEA and Oct4 as markers of an iPS cell is stably maintained even after the removal of the vector RNA (FIG. 15).

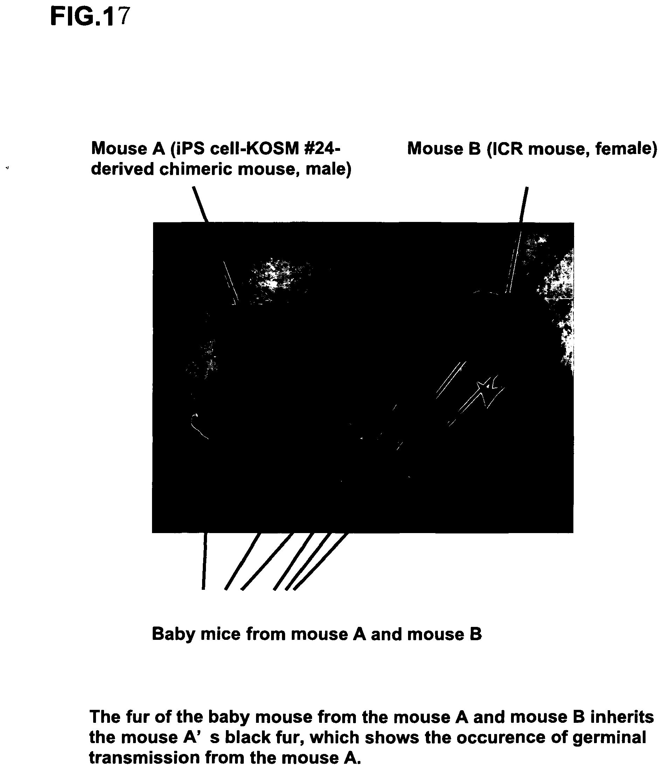

The inventors found a phenomenon that, when the human iPS cell maker-expressing cells are subcultured at 40°C in 2% CO₂, instead of conventional cell culture conditions of 37°C in 5% CO₂, the removal of the vector is facilitated (FIG. 17). The sustained expression-type Sendai virus vector used in this test has the property that gene expression becomes deteriorated at a high temperature (40°C). Thus, it is considered that the deterioration in expression of the Sendai virus including the L gene is likely to lead to acceleration of the removal of the vector.

### EXAMPLE 10: Preparation of Chimeric Mouse from Mouse iPS Cells

The iPS cell line KOSM #24 was established from MEF cells derived from a Nanog-EGFP (Enhanced Green Fluorescent Protein) knock-in mouse (STOCK Tg (Nanog-GFP, Puro) 1Yam), by a process described in Examples 5 and 6, using the hOct4/hSox2/hKlf4/hc-Myc sustained expression-inducing Sendai virus vector Version 2, and used as mouse iPS cells. A chimeric mouse was prepared according to a method described in the following Reference (Manipulating the Mouse Embryo, A Laboratory Manual, Third Edition (Nagy, A., et al, Cold Spring Harbor Laboratory Press, 2003)).

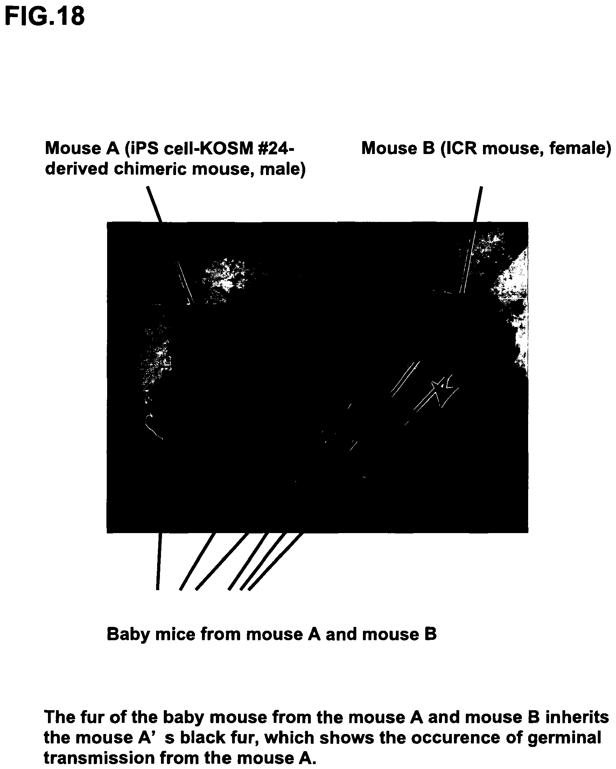

An eight-cell embryo was collected from the uterus of an ICR mouse (female, 6 to 8 week-old) on the 2.5th day of pregnancy and washed in M2 Medium. The embryo was then cultured in KSOM (Potasium Simplex Optimized Medium) for 1 to 2 hours, and then subjected to a microinjection. Mouse iPS cells were first dispersed with trypsin, and 10 to 15 iPS cells were introduced into the embryo from a small hole formed in a zona pellucida by using a micromanipulator. Subsequently, the embryo was cultured in KSOM for additional 24 hours, and then transplanted into the uterus of a female ICR mouse (surrogate parent mouse) crossed with a male mouse with bound ductus deferens. The chimaerism of the mouse after childbirth and the germ-line transmission to progeny were determined by detecting hair color and a gene unique to the iPS cells (EGFP). As a result, high levels of chimaerism, and germ-line transmission were observed (FIG. 18).

### EXAMPLE 11: Formation of Teratoma from Human iPS Cells

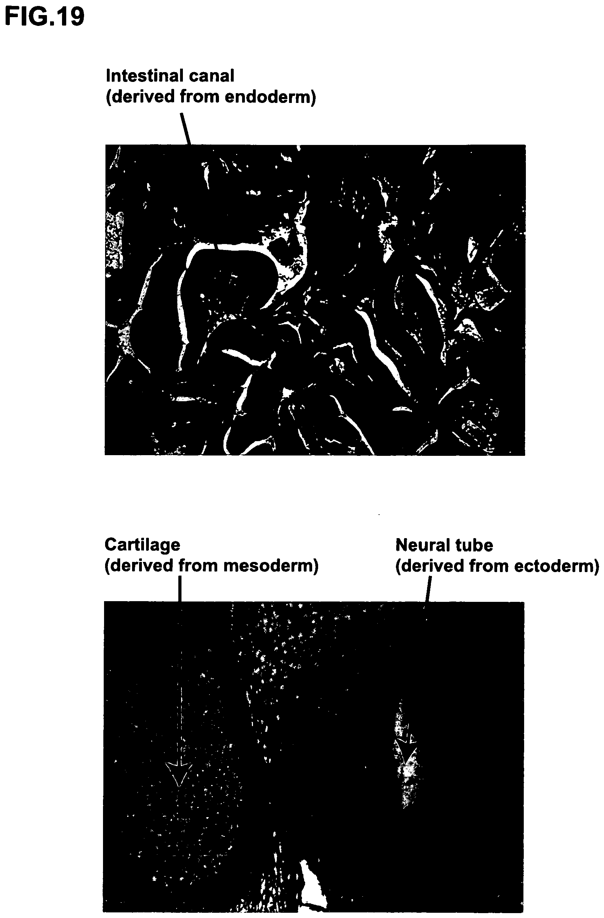

The human iPS cells obtained in Example 9 were adjusted to a concentration of 1.0 × 10⁶ cells/40 µL Hepes Buffered Saline Solution (HBSS)/mouse. A testis of a mouse (C.B17/ Icr-scidJcl) anesthesized with Nembutal and isoflurane was exposed and inoculated with the adjusted iPS cells . After about 8 weeks post-inoculation, a visually identifiable teratoma was formed. After 60 days post-inoculation, the teratoma was excised and fixed in Bouin's fixative solution (75% of saturated picric acid, 12% of formalin, 3% of acetic acid), and dehydrated by a treatment with 70% ethanol solution (1 hour), 90% ethanol solution (1 hour), 100% ethanol solution (1 hour, twice), 50% ethanol solution, 50% 2-butanol solution (1 hour) and 100% 2-butanol solution (30 minutes, twice). A sample thereof was then fixed in paraffin, and a slice having a thickness of 6 µm was prepared using a microtome. After deparaffinization, the slice was subjected to HE staining observation. As a result, differentiation into tissues of all three germ layers was observed (FIG. 19).

Ascertaining means used in Examples 5 to 11 is as follows:

### (a) Ascertainment of Gene Expression by Indirect Fluorescent Antibody Method

Expression of each of human Oct4, human Sox2, human Klf4, human c-Myc, mouse SSEA-1, human SSEA-4 and Sendai virus NP gene in each cell was ascertained by using a fluorescent antibody method using an antibody against each gene product. A primary antibody and a dilution rate used herein are as follows. The human Oct4: rabbit anti-Oct4 polyclonal antibody (Abcam Inc.) [× 100]; the human Sox2: rabbit anti-Sox2 polyclonal antibody (Abcam Inc.) [× 100]; the human Klf4: rabbit anti-Klf4 polyclonal antibody (CeMines Inc.) [× 100]; the human c-Myc: rabbit anti-c-myc polyclonal antibody (Santa Cruz Biotechnology Inc.) [× 100]; the SSEA-1: mouse anti-SSEA-1 monoclonal antibody (Santa Cruz Biotechnology Inc.) [× 200]; the SSEA-4: mouse anti-SSEA-4 monoclonal antibody (Santa Cruz Biotechnology Inc.) [× 200]; and the Sendai virus NP: mouse anti-NP monoclonal antibody [× 200] or rabbit anti-NP polyclonal antibody [× 1000].

### (b) Alkaline Phosphatase Staining

Culture medium was first removed, and the cells were washed with PBS. Then, Vector Red Alkaline Phosphatase Kit I (Vector Laboratories Inc.) was added to the cells, and left to react at room temperatures for 20 to 30 minutes. Cells having alkaline phosphatase activity were stained red.

### (c) Ascertainment of Expression of Mouse Nanog, Mouse Oct4, Human Nanog and Sendai Virus NP Gene by Reverse Transcription-Polymerase Chain Reaction (RT-PCR) Method.

Total RNA was extracted from iPS cells using ISOGEN (Nippon Gene Co. Ltd.). cDNA was synthesized using random primer according to instructions in the SuperScript III First strand synthesis system (Invitrogen Corporation). Target cDNA was then amplified by PCR using the following primers, to ascertain the expression. The mouse Nanog: 5'-GGAAGCATCGAATTCTGGGA-3' (SEQ ID NO: 18 in the Sequence Table (sense strand)), 5'-CGGAGCAGCATTCCAAGGCT-3' (SEQ ID NO: 19 in the Sequence Table (antisense strand)); the mouse Oct4: 5'-TGAGCCGTCTTTCCACCAGG-3' (SEQ ID NO: 20 in the Sequence Table (sense strand)); 5'-ACATGGTCTCCAGACTCCAC-3' (SEQ ID NO: 21 in the Sequence Table (antisense strand)); the human Nanog: 5'-AGCATCCGACTGTAAAGAAT-3' (SEQ ID NO: 22 in the Sequence Table (sense strand)), 5'-CCTCTCCACAGTTATAGAAG-3' (SEQ ID NO: 23 in the Sequence Table (antisense strand)); SeV NP: 5'-AGACCCTAAGAGGACGAAGA-3' (SEQ ID NO: 24 in the Sequence Table (sense strand)), 5'-ACTCCCATGGCGTAACTCCATAGTG-3' (SEQ ID NO: 25 in the Sequence Table (antisense strand)).

### (d) Genotyping of Mouse Cell

After collecting cells, genomic DNA was extracted using DNeasy Tissue Kit (QIAGEN Inc.). The extracted DNA was subjected to PCR using the following primer to determine a genotype. D18Mit4: 5'-ACTGTTGCTGGGGAATGG-3' (SEQ ID NO: 26 in the Sequence Table (sense strand)), 5'-CCAAGTTCAAAGCTGCTGG-3' (SEQ ID NO: 27 in the Sequence Table (antisense strand)); D7Mit44: 5'-TTCTGGCCTCTGTGAAGTAGTG-3' (SEQ ID NO: 28 in the Sequence Table (sense strand)), 5-GTGAAACCATGGTGCAGATG-3' (SEQ ID NO: 29 in the Sequence Table (antisense strand)); and D4Mit15: 5'-AGGAATACTGAATGTGGACTTTCC-3' (SEQ ID NO: 30 in the Sequence Table (sense strand)), 5'-TCCCTTGATTAACAGAAGACC TG-3' (SEQ ID NO: 31 in the Sequence Table (antisense strand)).

### Example 12: Preparation of hOct4/hSox2/hKlf4/hc-Myc Sustained Expression-Inducing Sendai Virus Vector Version 2

### (1) Preparation of Vector cDNA

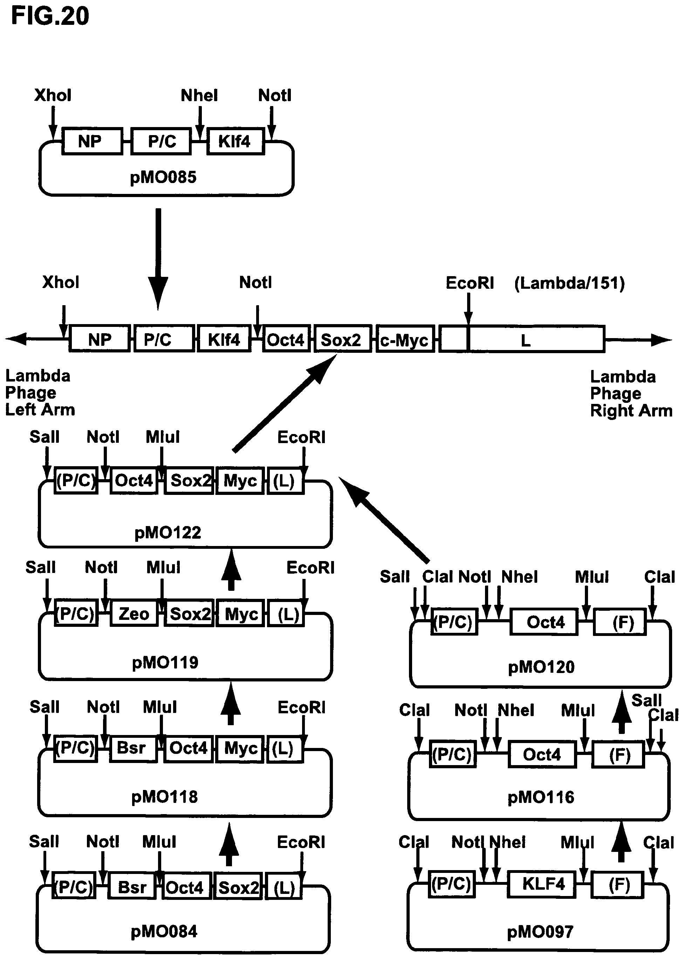

A human c-Myc gene was amplified from a plasmid pJL1 including a full-length human c-Myc cDNA by a PCR method using two primers consisting of 5'-ACTAGCTAGCTTAGA CGCTGGATTTTTTTCGGGTAGTGG-3' (SEQ ID NO: 32 in the Sequence Table (N-terminal side)) and 5'-GTCCACCGGTCTTACGCACAAGAGTTCCGT-3' (SEQ ID NO: 33 in the Sequence Table (C-terminal side)) as hc-Myc gene-amplifying primers. The termini of the double-stranded PCR DNA fragment were then cleaved at the Nhe I and Age I sites, and inserted between the Nhe I and Age I sites of the pMO084 prepared in Example 2 to obtain plasmid pMO118 (FIG. 20).

A human Sox2 gene was amplified from pUC57-Sox2 by a PCR method using two primers hSox2 gene-amplifying primers consisting of 5'-AGTACCTAGGCGCATGTACAACATGAT GGAGACGG-3' (SEQ ID NO: 34 in the Sequence Table (N-terminal side)) and 5'-GTCCGAC GTCCTCACATGTGTGAGAGGGGCAGT-3' (SEQ ID NO: 35 in the Sequence Table (C-terminal side)). The termini of the double-stranded PCR DNA fragment were cleaved at Avr II and Aat II sites, and inserted between the Avr II and Aat II sites of the pMO118 plasmid to obtain pMO119 (FIG. 20).

A human Oct4 gene was amplified from pUC57-Oct4 by a PCR method using two hOct4 gene-amplifying primers consisting of 5'-ACTAGCTAGCGGTTCCCCATGGCGGGACACCT GGCTTCGG-3' (SEQ ID NO: 36 in the Sequence Table (N-terminal side)) and 5'-GGTCCACG CGTTCAGTTTGAATGCATGGGAGAGCC-3' (SEQ ID NO: 37 in the Sequence Table (C-terminal side)). The termini of the double-stranded PCR DNA fragment was then cleaved at Nhe I and Mlu I sites, and inserted between the Nhe I and Mlu I sites of the pMO097 to obtain the plasmid pMO116. The orientation of a Cla I-Cla I fragment of the pMO116 was reversed to obtain pMO120. Furthermore, a Sal I and Mlu I fragment of pMO119 was combined with a fragment between Sal I and Mlu I sites of pMO120 to obtain pMO122 (FIG. 20).

Based on the plasmids obtained thus far, a T7 promoter sequence to SeV (1 to 3655), and SeV (3655 to 10480), were cut out from pMO085 and pMO122, respectively, and combined with a DNA fragment of SeV (10480 to 15384) + the right arm of the λDASH II obtained by cutting the λ/151 at EcoR I site. The combination was then cloned to prepare λ/SeVp (Mp + Klf4, +M:: Oct4, +F:: Sox2, +HN:: c-Myc) (FIG. 20) (a cDNA complementary to a full-length genome of a hOct4/hSox2/hKlf4/hc-Myc sustained expression-inducing Sendai virus vector Version 2 is described as SEQ ID NO: 38 in the Sequence Table).

### (2) Preparation of hOct4/hSox2/hKlf4/hc-Myc Sustained Expression-Inducing Sendai virus vector Version 2

In accordance with the protocol described in Example 3, a hOct4/hSox2/hKlf4/hc-Myc sustained expression-inducing Sendai virus vector Version 2 was prepared from the cDNA complementary to a full-length genome of the hOct4/hSox2/hKlf4/hc-Myc sustained expression-inducing Sendai virus vector Version 2.

### Example 13: Preparation of hOct4/hSox2/hKlf4/hc-Myc Sustained Expression-Inducing Sendai virus vector Version 3 capable of being Automatically Removed from iPS Cell

### (1) Preparation of Vector cDNA

In order to clone a sequence formed by connecting four target sequences for mir-302a which is ES cell-specific of miRNA, two sets of oligo DNAs consisting of a set of 5'-CCGGTTATCACCAAAACATGGAAGCACTTACGATTCACCAAAACATGGAAGCACT TAGGTACC-3' (SEQ ID NO: 39 in the Sequence Table) and 5'-TAAGTGCTTCCATGT TTTGGTGAATCGTAAGTGCTTCCATGTTTTGGTGATAA-3' (SEQ ID NO: 40 in the Sequence Table) and a set of 5'-TCACCAAAACATGGAAGCACTTACGATTCACCAAAA CATGGAAGCACTTAA-3' (SEQ ID NO: 41 in the Sequence Table) and 5'-CCGGTTAAGT GCTTCCATGTTTTGGTGAATCGTAAGTGCTTCCATGTTTTGGTGAGGTACC-3' (SEQ ID NO: 42 in the Sequence Table) were annealed, and then ligated together. Ligated DNA was cloned into pGL4.12 (Promega Corp.) cut at Age I site to obtain pNK300.

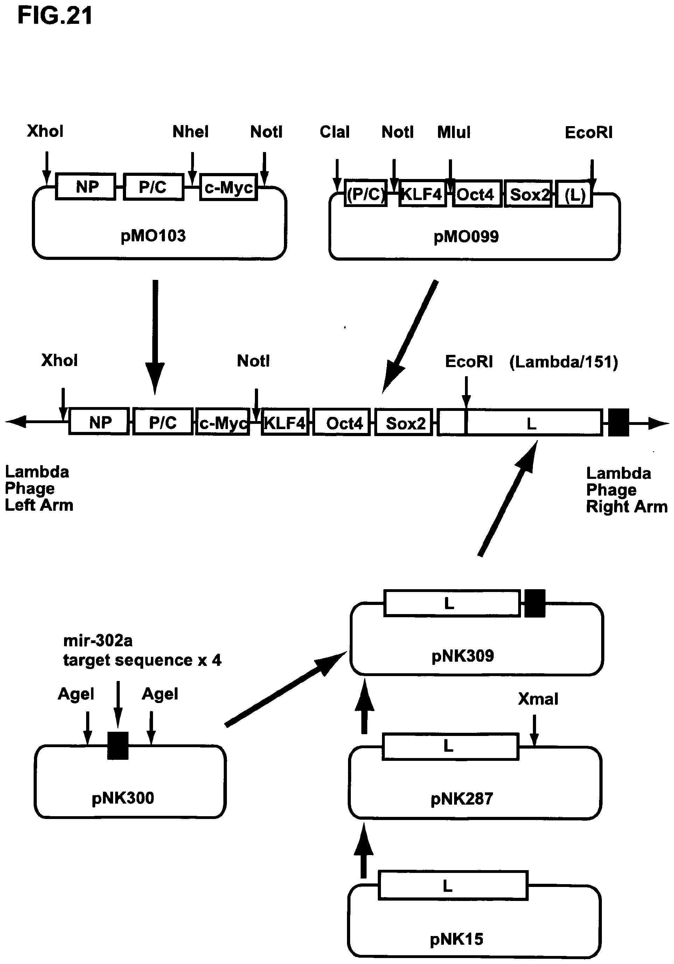

A plasmid vector pNK15 (SEQ ID NO: 43 in the Sequence Table) was prepared by inserting the SeV strain Cl.151 genome cDNA (bases 9014 to 15384), a hairpin ribozyme sequence of a tobacco ringspot virus and a termination sequence of T7 RNA polymerase into pBluescript II SK(+) (Agilent Technologies, Inc.)). Then, using 5'-GACAGCTCGTAATCCC GGGTCCCTATCGTGC-3' (SEQ ID NO: 44 in the Sequence Table (sense strand)) and 5'-GCACGATAGGGACCCGGGATTACGAGCTGTC-3' (SEQ ID NO: 45 in the Sequence Table (antisense strand)) as an Xma I-recognition sequence insertion site-forming primer, an Xma I-recognition sequence was inserted into the plasmid vector pNK15 at a site just after SeV (15244) by a Quickchange Site-directed Mutagenesis II kit (Agilent Technologies, Inc.), to obtain pNK287. A fragment obtained by cutting the pNK300 at Age I site was inserted into the Xma I site of the pNK287 to obtain pNK309 (FIG. 21).

T7 promoter sequence to SeV (1 to 3655 with c-Myc), and SeV (3655 to 10480 with Klf4/Oct4/Sox2), was cut out from pMO103 and pMO099 as described in Example 3, and the connected SeV (9014 to 15384)-hairpin ribozyme sequence-T7 RNA polymerase termination sequence was cut out from the pNK309. Then, these fragments were combined with a DNA fragment consisting of right and left arms of the λDASH II, and the obtained combination was cloned to create λ/SeVp (Mp + myc, +M:: Klf4, +F:: Oct4, +HN:: Sox2, L + mir302T4) (FIG. 21) (a cDNA complementary to a full-length genome of a hOct4/hSox2/hKlf4/hc-Myc sustained expression-inducing Sendai virus vector Version 3 is described as SEQ ID NO: 46 in the Sequence Table).

### (2) Preparation of hOct4/hSox2/hKlf4/hc-Myc Sustained Expression-Inducing Sendai virus vector Version 3

In accordance with the same process as that in Example 3-(2), a hOct4/hSox2 /hKlf4/hc-Myc sustained expression-inducing Sendai virus vector Version 3 was prepared from the cDNA complementary to a full-length genome of the hOct4/hSox2/hKlf4/hc-Myc sustained expression-inducing Sendai virus vector Version 3.

### Example 14: Time-Series Evaluation on Removal of RNA Genome of Sustained Expression-Type Sendai virus vector from Cell using siRNA

As for the technique of removing the vector genome from a cell stably retaining the RNA genome of the sustained expression-type Sendai virus vector, using siRNA, as described in the Example 4, a time-series change in the removal is quantitatively analyzed, and it is ascertained that no vector genome remained in the cell subjected to the siRNA treatment.

As a marker of gene expression by the sustained expression-type Sendai virus vector, unstable firefly luciferase gene (Luc2CP, Promega Corp.) and Escherichia coli hygromycin B-resistant gene (HygB) were used. A luciferase activity reflects the copy number of the recombinant Sendai virus RNAs, and the number of hygromycin B-resistant cells reflects the number of cells retaining the sustained expression-type Sendai virus vector.

A KO/HygB/EGFP/Luc2CP-loaded sustained expression-type Sendai virus vector loaded with a Luc2CP gene and a HygB gene was prepared by substituting the hOct4 gene, the hSox2 gene, the hKlf4 gene and the hc-Myc gene with the Kusabira Orange (KO) gene (Medical & Biological Laboratories, Co. Ltd.), HygB gene, Enhanced Green Fluorescent Protein (EGFP) gene, and Luc2CP gene, respectively, using the same methodology as that described for the cloning of hOct4/hSox2/hKlf4/hc-Myc sustained expression-inducing Sendai virus vector (see Example 3). Two types of short interfering RNAs (siRNAs) (same as Example 4) were used for suppressing expression of L gene (#1: sense strand 5'-GGUUCAGCAUCAAAUAUGAAG-3' (SEQ ID NO: 14 in the Sequence Table) and antisense strand 5'-UCAUAUUUGAUGCUGAAC CAU-3' (SEQ ID NO: 15 in the Sequence Table).

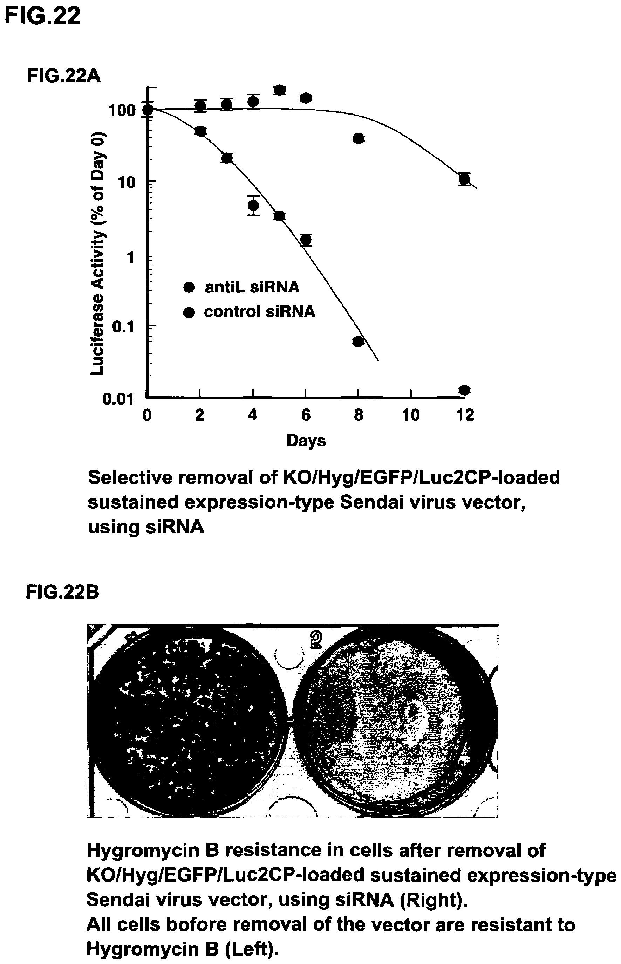

siRNA complimentary to sea-firefly luciferase gene (Rluc, Promega Corp.) served as negative control because it did not have any homologous with the Sendai virus vector genome. In order to check a vector genome removing effect by the siRNA, a HeLa cell stably retaining the genome of the sustained expression-type Sendai virus vector loaded with Luc2CP gene and HygB gene was seeded into a 24-well plate at a concentration of 3 × 10⁴ cells/0.4 mL medium (MEM, 10% fetal bovine serum)/well. The siRNA was diluted with Opti-MEM to a final concentration of 40 nM, and 1 µL of Lipofectamine RNAiMAX (Lifetechnologies, Inc.) was added to induce a reaction at room temperature for 20 minutes. Then, the siRNA was added to the above cells. Subsequently, the cells were collected in a time-series manner. On the 3rd and 6th days, the cells were subcultured under the above conditions, and the siRNA was added to the cell again under the above conditions. As a result, the luciferase activity as an index of an amount of the vector in the cells was lowered with time. On and after the 8th day, luciferase activity was lowered down to a detection limit (FIG. 22A).

Cells transfected with siRNA three times were cultured for 4 weeks in the absence of siRNA. The cells were then transferred to selective medium containing 200 µg/mL of hygromycin B, in a number of 10⁴, and further cultured for another week. As a result, no hygromycin B-resistance clone emerged, which demonstrates that there is no cell having the sustained expression-type Sendai virus vector loaded with the HygB gene (FIG. 22B).

### Example 15: Evaluation of the Gene Expression Patterns of Two Exogenous Genes Loaded on Sustained Expression-Type Sendai virus vector

In the production of iPS cells, all of the four types of reprogramming genes need to be simultaneously expressed in a common cell. If the balance of expression intensity between the reprogramming genes is changed, the reprogramming efficiency is changed (Reference: Papapetrou, et al., Proc. Natl. Acad. Sci. USA, 106, 12759-12764, 2009), and a low-quality cell line with a similar configuration to that of an iPS cell but without pluripotency is likely to emerge (Reference: Chan, et al., Nat. Biotech., 27, 1034-1037, 2009). Thus, the method of producing iPS cells with high efficiency and excellent reproducibility needs to meet the following two requirements: 1) the four types of reprogramming genes must be simultaneously transfected into a common cell; and 2) the four types of transfected reprogramming genes must be expressed in each cell in a balanced manner. In cases where four types of reprogramming genes are transfected into cells using the sustained expression-type Sendai virus vector, the following two processes are contemplatable: one process in which all of the reprogramming genes are loaded on a single vector, as described in the Examples of the present invention; and the other process in which a plurality of vectors each loaded with one to three types of reprogramming genes are prepared separately, and, after mixing them, transfected, as described in the Patent Document 1 and the Non-Patent Document 17. Using, as an index, expression patterns of two types of genes: Kusabira Orange (KO) gene and Enhanced Green Fluorescent Protein (EGFP) gene, it was evaluated whether there is a difference in expression pattern of the exogenous genes between the two processes.

A KO/HygB/EGFP/Luc2CP-loaded sustained expression-type Sendai virus vector described in Example 14 was used as a vector loaded with both of KO and EGFP genes. Further, for use as a vector loaded with only KO gene, a Zeo/KO/CLuc-loaded sustained expression-type Sendai virus vector was prepared by removing the hKlf4 gene from the hOct4/hSox2/hKlf4 sustained expression-inducing Sendai virus vector described in the Example 2, and replacing the Bsr gene, Oct4 gene and Sox2 gene with zeocin-resistant (Zeo) gene, KO gene and secreted luciferase (CLuc) gene, respectively. For use as a vector loaded with only the EGFP gene, a Bsr/EGFP/gp91phox-loaded sustained expression-type Sendai virus vector was prepared by removing the hKlf4 gene from the hOct4/hSox2/hKlf4 sustained expression-inducing Sendai virus vector described in the Example 2, and replacing the Oct4 gene and Sox2 gene with EGFP gene and chronic granulomatous disease-caused gene (gp91phox), respectively.

The KO/HygB/EGFP/Luc2CP-loaded vector was transfected into a monkey LLCMK2 cell line on condition that the number of vector particles/cell is 5, and the resulting cells were selected with hygromycin B, to establish a cell pool LLCMK2 (SeVdp/KO/HygB/EGFP/Luc2) retaining the KO/HygB/EGFP/Luc2CP-loaded vector. In the same manner, the Zeo/KO/CLuc-loaded vector and the Bsr/EGFP/gp91phox-loaded vectors were mixed at a vector particle ratio of 1 : 1, and the mixture was transfected into LLCMK2 cells on condition that the number of vector particles/cell is 5w. Then, the resulting cells were simultaneously selected with blasticidin S and Zeocin, to establish a cell pool LLCMK2 (SeVdp/Zeo/KO/CLuc + SeVdp/Bsr/EGFP/gp91phox) having both vectors in each of the cells.

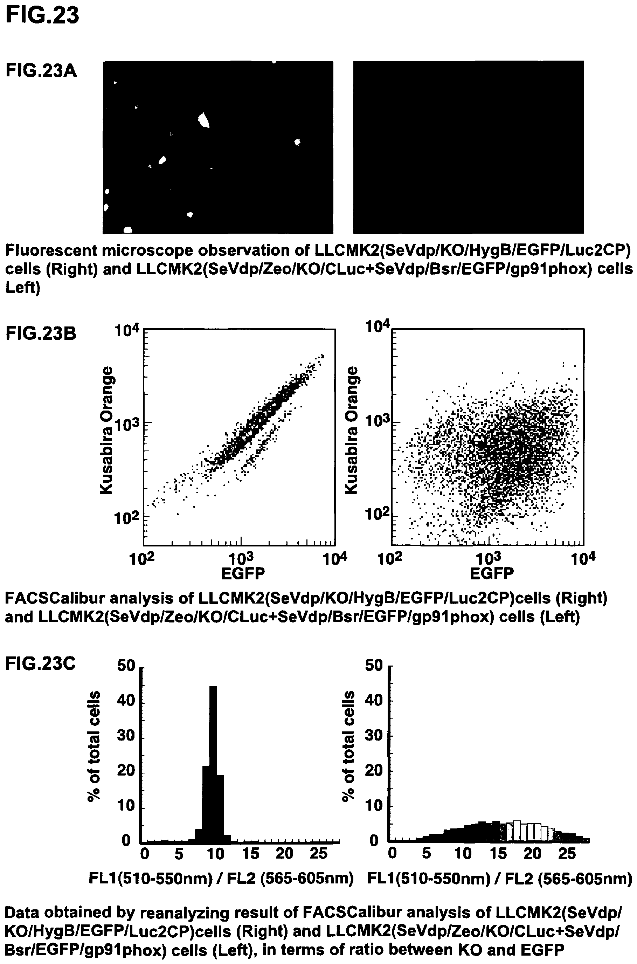

The two types of cell lines were observed by fluorescent microscopy (Zeiss), and two images thereof were superimposed on each other, while assigning a red pseudocolor and a green pseudocolor to fluorescence generated by KO and fluorescence generated by EGFP, respectively. The image of LLCMK2 (SeVdp/KO/HygB/EGFP/Luc2) cells became yellow which indicates that KO and EGPF are simultaneously expressed, whereas the image of the LLCMK2 (SeVdp/Zeo/KO/CLuc + SeVdp/Bsr/EGFP/gp91phox) cells indicated a mixture of red/yellow/ green-colored cells, which shows that a balance between the expression of KO and EGFP is significantly different in each cell (FIG. 23A).

In order to quantitatively analyze the balance between respective expressions of KO and EGFP, the above cells were analyzed by a Fluorescent-activated Cell Analyzer (BD FACSCalibur, Becton, Dickinson and Company). 10⁴ LLCMK2 (SeVdp/KO/HygB/EGFP/ Luc2) cells and 10⁴ LLCMK2 (SeVdp/Zeo/KO/CLuc + SeVdp/Bsr/ EGFP/gp91phox) cells were suspended in 2 mL of buffer to measure the fluorescence intensity (FL1) of EGFP and a fluorescence intensity (FL2) of KO. The analysis shows that a ratio between the fluorescence intensities of EGFP and KO in the LLCMK2 (SeVdp/KO/HygB/EGFP/Luc2) is kept constant, whereas the ratio in the LLCMK2 (SeVdp/Zeo/KO/CLuc + SeVdp/Bsr /EGFP/gp91phox) cells significantly fluctuates (FIG. 23B). In an analysis of a ratio between FL1 and FL2, 50% or more of the LLCMK2 (SeVdp/KO/HygB/EGFP/Luc2) cells had the same ratio, whereas the ratio in the LLCMK2 (SeVdp/Zeo/KO/CLuc + SeVdp/Bsr /EGFP/gp91phox) was widely distributed in a broad range from 0 to 100% (FIG. 23C).

The above results show that the need for simultaneously transfecting two or more types of genes into each cell and inducing gene expression at the same ratio can be satisfied by the process to be implemented by the present invention in which the four types of reprogramming genes are loaded on a single common vector, but cannot be satisfied by the process as described in the Patent Document 1 and the Non-Patent Document 17 in which each of the four types of reprogramming genes is loaded on a respective one of two or more vectors, separately.

### Example 16: Induction of iPS cells using Sustained Expression-Type Sendai virus vectors loaded with Reprogramming Genes, respectively

As for the two iPS production processes: one process to be implemented by the present invention in which the four types of reprogramming genes are loaded on a single common vector; the other process as described in the Patent Document 1 and the Non-Patent Document 17 in which each of the four types of reprogramming genes is loaded on a respective one of two or more vectors, separately, a comparative evaluation on iPS cell production efficiency was performed. The hOct4/hSox2/hKlf4/hc-Myc sustained expression-inducing Sendai virus vector was used as a vector for the process in which the four types of reprogramming genes are loaded on a single common vector. Further, the hOct4/hSox2/hKlf4 sustained expression-inducing Sendai virus vector described in the Example 2, and a Zeo/KO/hc-Myc sustained expression-inducing Sendai virus vector loaded with only the c-Myc as a reprogramming gene, are used as vectors for the process in which the four types of reprogramming genes are loaded on two or more vectors, separately. The Zeo/KO/hc-Myc sustained expression-inducing Sendai virus vector was prepared by replacing the Oct4 gene, Sox2 gene and Klf4 gene of the hOct4/hSox2/hKlf4 sustained expression-inducing Sendai virus vector with the Zeo gene, KO gene and c-Myc gene, Respectively.

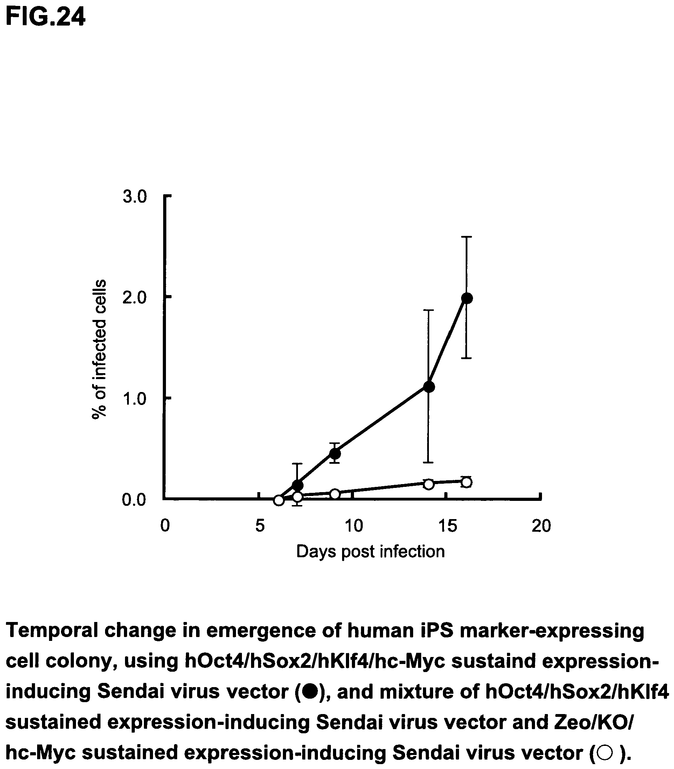

According to the Example 5, the hOct4/hSox2/hKlf4/hc-Myc sustained expression-inducing Sendai virus vector itself, or a mixture of hOct4/hSox2/hKlf4 sustained expression-inducing Sendai virus vector and the Zeo/KO/hc-Myc sustained expression-inducing Sendai virus vector formed at a vector particle ratio of 1 : 1, was transfected into mouse embryo-derived fibroblast cells. Using, as an index, emergence of an alkaline phosphatase-positive cell colony, emergency of an iPS cell colony was assayed. The result shows that, in the process to be implemented by the present invention in which the four types of reprogramming genes are loaded on a single common vector has cell production efficiency far greater than that in the process in which each of the four types of reprogramming genes is loaded on a respective one of two vectors, separately (FIG. 24).

### Example 17: Induction of iPS Cells using hOct4/hSox2/hKlf4/hc-Myc Sustained Expression-Inducing Sendai virus vector Version 3

TIG3 cells was seeded on a 12-well plate at a density of 1.0 × 10⁵ cells/well. On the next day, the hOct4/hSox2/hKlf4/hc-Myc sustained expression-inducing Sendai virus vector prepared in Example 3, or the hOct4/hSox2/hKlf4/hc-Myc sustained expression-inducing Sendai virus vector Version 3 prepared in Example 13, was added to culture medium to induce human iPS cells according to Example 8.

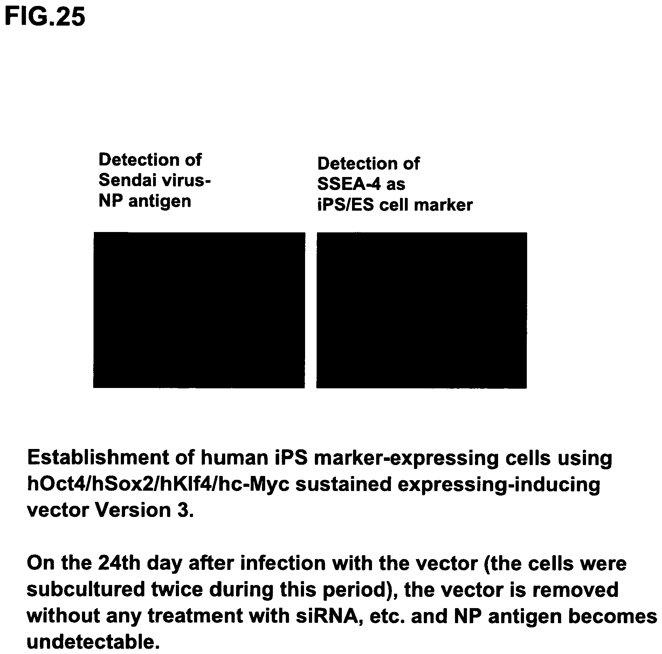

Then, on the 24th day after subculturing emerged colonies twice, the colonies were fluorescently stained using an antibody against NP protein. As a result, in the process using the hOct4/hSox2/hKlf4/hc-Myc sustained expression-inducing Sendai virus vector Version 3, the removal of the vector was ascertained in many colonies (FIG. 25). On the other hand, in the process using the hOct4/hSox2/hKlf4/hc-Myc sustained expression-inducing Sendai virus vector, the vector still remained.

The above result clearly shows that, when human iPS cells are induced using the hOct4/hSox2/hKlf4/hc-Myc sustained expression-inducing Sendai virus vector Version 3, the vector is automatically removed by the action of mir-302a expressed in the induced iPS cells.

### Example 18: Establishment of iPS cells from Human Peripheral-Blood Mononuclear Cells

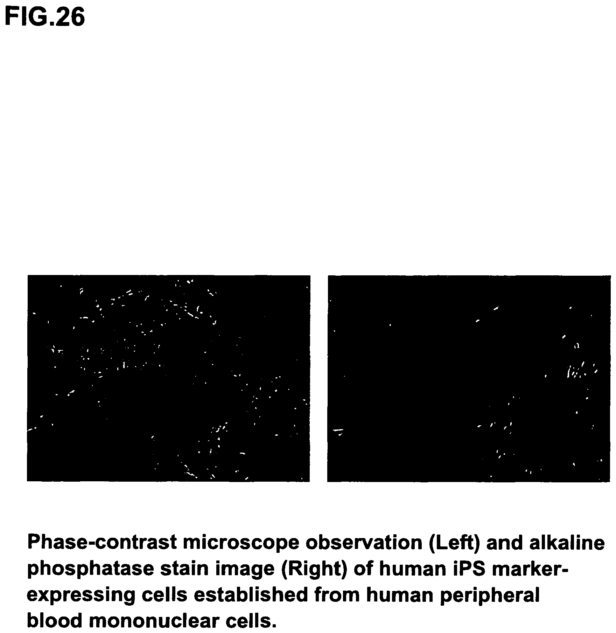

20 mL of adult blood was diluted with 20 mL of PBS (-), and layered on 6 mL of Lymphoprep. The blood was then centrifuged at 1.800 r.p.m. for 30 minutes to separate an upper layer of platelets, an intermediate layer including mononuclear cells and a lower layer including red blood cells. The intermediate layer was washed with PBS (-) to obtain human peripheral-blood mononuclear cells. In accordance with the technique described in Example 8, the hOct4/hSox2/hKlf4/hc-Myc sustained expression-inducing Sendai virus vector was transfected into the cells, and then the infected cells were cultured. As a result, alkaline phosphatase-positive iPS cells positive having a similar configuration to that of a human ES cell emerged (FIG. 26). Such cell colonies did not emerge in a negative control into which the vector was not transfected.

### Example 19: Comparison between Gene Expression Patterns in Human iPS Cells Produced using Sustained Expression-Type Recombinant Sendai virus vector

### (1) Preparation of RNA Sample to be Analyzed

human iPS cells prepared using the hOct4/hSox2/hKlf4/hc-Myc sustained expression-inducing Sendai virus vector according to the process in Example 8 were cultured on matrigel (Becton, Dickinson and Company) in MEF conditioned medium without any feeder cells, and 1.0 × 10⁶ cells were collected each time. Then, whole cell RNA was extracted using ISOGEN (Nippon Gene Co. Ltd.). As a control, five human ES cell lines established at the Institute for Frontier Medical Sciences, Kyoto University, were cultured in the same manner, and whole cell RNA was extracted.

### (2) Analysis of Gene Expression

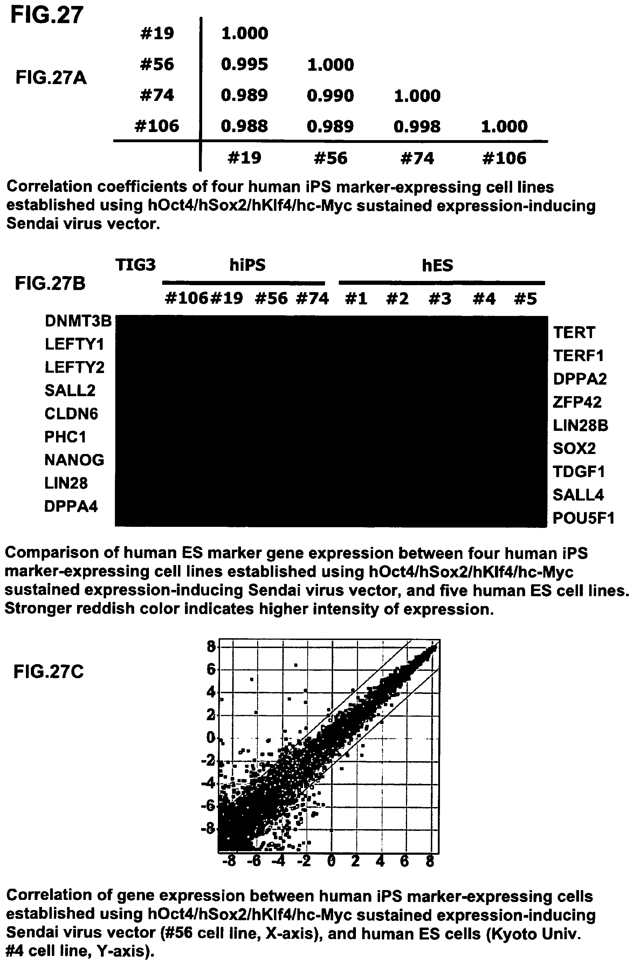

0.5 µg of whole cell RNA was labeled with Cy3, using Quick Amp Labeling Kit (Agilent Technologies, Inc.). The labeled RNA was hybridized with Whole Human Genome (4x44k) DNA array (Agilent Technologies, Inc.), using a Gene Expression Hybridization Kit (Agilent Technologies, Inc.), and a signal was acquired using Agilent DNA Microarray Scanner. The acquired signal was analyzed using GeneSpringGX10 software (Agilent Technologies, Inc.) to obtain a correlation coefficient between respective gene expression patterns of cell clones (FIG. 27A). As a result, the gene expression patterns of human iPS cells produced using the hOct4/hSox2/hKlf4/hc-Myc sustained expression-inducing Sendai virus vector were significantly similar to each other, as evidenced by a correlation coefficient of 0.98 or more. This shows that iPS cells having with significantly uniform properties can be established by the method of the present invention. In addition, each of the iPS cells subjected to this analysis expressed a marker gene which was strongly expressed in human ES cells and at the same expression level as that observed in ES cells (FIG. 27B), which means that the gene expression of iPS cells has high correlativity with that of human ES cells (FIG. 27C).

## Claims

1. A reprogramming gene-loaded Sendai virus, **characterized in that** the reprogramming gene-loaded Sendai virus comprises, as its genome:
(i) Sendai virus genes including NP gene, P/C gene and L gene: and
(ii) mammalian reprogramming genes comprising a combination of Oct3/4, Sox2 and Klf4, or a combination of Oct3/4, Sox2, Klf4 and c-Myc;
wherein at least one gene from M gene, F gene and HN gene in the genome is functionally deleted by replacement with the mammalian reprogramming genes;
wherein the L gene is a mutated gene and encodes an amino-acid sequence of the L-protein in which the amino-acid residue at position 1618 is valine; and
wherein each of the M gene, the F gene and the HN gene is a gene derived from a Sendai virus strain Cl.151.

2. The Sendai virus of claim 1, **characterized in that** all of the M gene, the F gene and the HN gene are replaced by the mammalian reprogramming genes.

3. The Sendai virus of claim 1 or 2, **characterized in that** it is a virus particle.

4. The Sendai virus of any one of claims 1 to 3, **characterized in that** the at least one gene from M, F and HN gene is subjected to insertion or replacement by a marker gene.

5. A reprogramming gene-loaded Sendai virus vector for generating an induced pluripotent stem cell, **characterized in that** it comprises the Sendai virus of any one of claims 1 to 4.

6. The reprogramming gene-loaded Sendai virus vector for generating an induced pluripotent stem cell of claim 5, wherein the Sendai virus vector comprises, on its genome, a target sequence for a specific microRNA added into a protein-noncoding region of L gene, NP gene or P gene, wherein the specific microRNA appears only in a reprogrammed cell.

7. A template vector for preparing a reprogramming gene-loaded Sendai virus, **characterized in that** the vector is a recombinant cloning vector for the Sendai virus of any one of claims 1 to 4 comprising the modified Sendai virus genome as defined in any one of claims 1 to 4.

8. The template vector of claim 7, **characterized in that** all of the M gene, the F gene and the HN gene are replaced by the mammalian reprogramming genes.

9. The template vector of claims 7 or 8, **characterized in that** the cloning vector is a phage vector.

10. The template vector of claim 9, **characterized in that** the phage vector is a λ phage vector.

11. The template vector of any one of claims 7 to 10, **characterized in that** it comprises DNA.

12. The template vector of claim 11, **characterized in that** it comprises a target sequence for a specific microRNA,
wherein the target sequence is added into a protein-noncoding region of L gene, NP gene or P gene,
wherein the specific microRNA appears only in a reprogrammed cell.

13. A cell **characterized in that** it is transfected with the template vector of any one of claims 7 to 12 with the proviso that the cell is not a human embryonic cell.

14. The cell of claim 13, **characterized in that** T7 RNA polymerase is expressed therein.

15. The cell of claim 14, **characterized in that** it is further transfected with NP gene, P gene and L gene.

16. The cell of claim 15, **characterized in that** it is further transfected with at least one gene from M gene, F gene and HN gene wherein the at least one gene includes gene(s) which is/are replaced by mammalian reprogramming genes in the template vector of any one of claims 7 to 12.

17. A method of producing a reprogramming gene-loaded Sendai virus vector, **characterized in that** it comprises cultivating the cell of claim 16 at a virus-particle production temperature of 32°C, in a culture medium, to collect a Sendai virus particle which comprises, as its genome:
(i) Sendai virus genes including NP gene, P/C gene and L gene; and
(ii) mammalian reprogramming genes comprising a combination of Oct3/4, Sox2 and Klf4, or a combination of Oct3/4, Sox2, Klf4 and c-Myc;
wherein at least one gene from M gene, F gene and HN gene in the genome is replaced by the mammalian reprogramming genes;
wherein the L gene is a mutated gene and encodes an amino acid sequence of the L-protein in which the amino-acid residue at position 1618 is valine; and
wherein each of the M gene, the F gene and the HN gene is a gene derived from a Sendai virus strain Cl.151; and
wherein the cell of claim 16 is a viral vector-producing cell.

18. An in vitro method for producing an induced pluripotent stem cell, **characterized in that** it comprises the steps of: infecting a differentiated mammalian cell with the reprogramming gene-loaded Sendai virus vector of claim 5 to reprogram the differentiated mammalian cell; and transfecting the reprogrammed cell with a siRNA and then allowing siRNA to act on the vector to remove the reprogramming gene-loaded Sendai virus vector from the cell,
wherein the siRNA is designed to target the L gene of the Sendai virus vector.

19. An in vitro method of producing an induced pluripotent stem cell, **characterized in that** it comprises the steps of: infecting a differentiated cell with the reprogramming gene-loaded Sendai virus vector of claim 6 to reprogram the differentiated cell; and then automatically removing the reprogramming gene-loaded Sendai virus vector, after forming an induced pluripotent stem cell which is the specific microRNA-expressing cell.

20. A method of producing an induced pluripotent stem cell, **characterized in that** it comprises the steps of: infecting a differentiated cell with the reprogramming gene-loaded Sendai virus vector of claim 5 or 6 to reprogram the differentiated cell; and then culturing the cell under high-temperature conditions to promote removal of the reprogramming gene-loaded Sendai virus vector from the cell.

## Patentansprüche

1. Umprogrammierendes Gen-beladenes Sendai-Virus, **dadurch gekennzeichnet, dass** das umprogrammierende Gen-beladene Sendai-Virus, als sein Genom, Folgendes umfasst:
(i) Sendai-Virus-Gene einschließlich NP-Gen, P/C-Gen und L-Gen; und
(ii) Säugetier-Umprogrammiergene, umfassend eine Kombination aus Oct3/4, Sox2 und Klf4 oder eine Kombination aus Oct3/4, Sox2, Klf4 und c-Myc;
wobei mindestens ein Gen aus M-Gen, F-Gen und HN-Gen in dem Genom funktionell deletiert ist durch das Ersetzen durch die Säugetier-Umprogrammiergene;
wobei das L-Gen ein mutiertes Gen ist und eine Aminosäuresequenz des L-Proteins kodiert, in dem der Aminosäurerest auf Position 1618 Valin ist; und
wobei jedes des M-Gens, des F-Gens und des HN-Gens ein Gen ist, das von einem Sendai-Virusstamm Cl.151 stammt.

2. Sendai-Virus nach Anspruch 1, **dadurch gekennzeichnet, dass** alle des M-Gens, des F-Gens und des HN-Gens durch die Säugetier-Umprogrammiergene ersetzt sind.

3. Sendai-Virus nach Anspruch 1 oder 2, **dadurch gekennzeichnet, dass** es ein Viruspartikel ist.

4. Sendai-Virus nach einem der Ansprüche 1 bis 3, **dadurch gekennzeichnet, dass** das mindestens eine Gen aus dem M-, F- und HN-Gen einem Einsetzen oder einem Ersetzen durch ein Markergen unterzogen wird.

5. Umprogrammierender Gen-beladener Sendai-Virusvektor zum Erzeugen einer induzierten pluripotenten Stammzelle, **dadurch gekennzeichnet, dass** er das Sendai-Virus nach einem der Ansprüche 1 bis 4 umfasst.

6. Umprogrammierender Gen-beladener Sendai-Virusvektor zum Erzeugen einer induzierten pluripotenten Stammzelle nach Anspruch 5, wobei der Sendai-Virusvektor in seinem Genom eine Zielsequenz für eine spezifische microRNA umfasst, die zu einem nicht-Protein-codierenden Bereich des L-Gens, NP-Gens oder P-Gens hinzugefügt ist, wobei die spezifische microRNA nur in einer umprogrammierten Zelle vorkommt.

7. Mustervektor für das Herstellen eines umprogrammierenden Gen-beladenen Sendai-Virus, **dadurch gekennzeichnet, dass** der Vektor ein rekombinanter Klonierungsvektor für das Sendai-Virus nach einem der Ansprüche 1 bis 4 ist, umfassend das Genom wie in einem der Ansprüche 1 bis 4 definiert.

8. Mustervektor nach Anspruch 7, **dadurch gekennzeichnet, dass** alle des M-Gens, F-Gens und HN-Gens durch die Säugetier-Umprogrammiergene ersetzt sind.

9. Mustervektor nach Ansprüchen 7 oder 8, **dadurch gekennzeichnet, dass** der Klonierungsvektor ein Phagenvektor ist.

10. Mustervektor nach Anspruch 9, **dadurch gekennzeichnet, dass** der Phagenvektor ein λ-Phagenvektor ist.

11. Mustervektor nach einem der Ansprüche 7 bis 10, **dadurch gekennzeichnet, dass** er DNA umfasst.

12. Mustervektor nach Anspruch 11, **dadurch gekennzeichnet, dass** er eine Zielsequenz für eine spezifische microRNA umfasst,
wobei die Zielsequenz zu einem nicht-Protein-codierenden Bereich des L-Gens, NP-Gens oder P-Gens hinzugefügt ist,
wobei die spezifische microRNA nur in einer umprogrammierten Zelle vorkommt.

13. Zelle, **dadurch gekennzeichnet, dass** sie mit dem Mustervektor nach einem der Ansprüche 7 bis 12 transfiziert ist, unter der Voraussetzung, dass die Zelle keine menschliche Embryonalzelle ist.

14. Zelle nach Anspruch 13, **dadurch gekennzeichnet, dass** T7-RNA-Polymerase darin exprimiert ist.

15. Zelle nach Anspruch 14, **dadurch gekennzeichnet, dass** sie ferner mit dem NP-Gen, P-Gen und L-Gen transfiziert ist.

16. Zelle nach Anspruch 15, **dadurch gekennzeichnet, dass** sie ferner mit mindestens einem Gen aus dem M-Gen, F-Gen und HN-Gen transfiziert ist, wobei das mindestens eine Gen Gen(e) enthält, das/die durch Säugetier-Umprogrammiergene in dem Mustervektor nach einem der Ansprüche 7 bis 12 ersetzt ist/sind.

17. Verfahren zum Herstellen eines umprogrammierenden Gen-beladenen Sendai-Virusvektors, **dadurch gekennzeichnet, dass** es das Kultivieren der Zelle nach Anspruch 16 umfasst, bei einer Viruspartikel-Herstellungstemperatur von 32 °C in einem Nährmedium, um ein Sendai-Viruspartikel zu erhalten, das als sein Genom Folgendes umfasst:
(i) Sendai-Virus-Gene einschließlich NP-Gen, P/C-Gen und L-Gen; und
(ii) Säugetier-Umprogrammiergene umfassend eine Kombination aus Oct3/4, Sox2 und Klf4 oder eine Kombination aus Oct3/4, Sox2, Klf4 und c-Myc;
wobei mindestens ein Gen aus M-Gen, F-Gen und HN-Gen in dem Genom durch die Säugetier-Umprogrammiergene ersetzt ist;
wobei das L-Gen ein mutiertes Gen ist und eine Aminosäuresequenz des L-Proteins kodiert, in dem der Aminosäurerest auf Position 1618 Valin ist; und
wobei jedes des M-Gens, des F-Gens und des HN-Gens ein Gen ist, das von einem Sendai-Virusstamm Cl.151 stammt; und
wobei die Zelle nach Anspruch 16 eine virale vektorproduzierende Zelle ist.

18. In-vitro-Verfahren für die Herstellung einer induzierten pluripotenten Stammzelle, **dadurch gekennzeichnet, dass** es die folgenden Schritte umfasst: das Infizieren einer differenzierten Säugetier-Zelle mit dem umprogrammierenden Gen-beladenen Sendai-Virusvektor nach Anspruch 5, um die differenzierte Säugetier-Zelle umzuprogrammieren; und das Transfizieren der umprogrammierten Zelle mit einer siRNA und dann das Wirkenlassen der siRNA auf den Vektor, um den umprogrammierenden Gen-beladenen Sendai-Virusvektor aus der Zelle zu entfernen,
wobei die siRNA ausgebildet ist, das L-Gen des Sendai-Virusvektors anzuzielen.

19. In-vitro-Verfahren zum Herstellen einer induzierten pluripotenten Stammzelle, **dadurch gekennzeichnet, dass** es die folgenden Schritte umfasst: das Infizieren einer differenzierten Zelle mit dem umprogrammierenden Gen-beladenen Sendai-Virusvektor nach Anspruch 6, um die differenzierte Zelle umzuprogrammieren; und dann das automatische Entfernen des umprogrammierenden Gen-beladenen Sendai-Virusvektors, nach dem Bilden einer induzierten pluripotenten Stammzelle, die die spezifischemicroRNA-exprimierende Zelle ist.

20. Verfahren zum Herstellen einer induzierten pluripotenten Stammzelle, **dadurch gekennzeichnet, dass** es die folgenden Schritte umfasst: das Infizieren einer differenzierten Zelle mit dem umprogrammierenden Gen-beladenen Sendai-Virusvektor nach Anspruch 5 oder 6, um die differenzierte Zelle umzuprogrammieren; und dann das Kultivieren der Zelle unter Bedingungen hoher Temperatur, um das Entfernen des umprogrammierenden Gen-beladenen Sendai-Virusvektors aus der Zelle zu unterstützen.

## Revendications

1. Virus Sendai chargé par un gène reprogrammant, **caractérisé en ce que** le virus Sendai chargé par un gène reprogrammant comprend, en tant que son génome:
(i) des gènes de virus Sendai incluant le gène NP, le gène P/C et le gène L; et
(ii) des gènes reprogrammants mammifères comprenant une combinaison de Oct3/4, Sox2 et Klf4 ou une combinaison de Oct3/4, Sox2, Klf4 et c-Myc;
dans lequel au moins un gène parmi le gène M, le gène F et le gène HN dans le génome est fonctionnellement délété par un remplacement avec les gènes reprogammants mammifères;
dans lequel le gène L est un gène muté et code pour une séquence d'acides aminés de la protéine L dans laquelle le résidu d'acide aminé à la position 1618 est une valine; et
dans lequel chacun du gène M, du gène F et du gène HN est un gène dérivé d'une souche de virus Sendai CI. 151.

2. Virus Sendai selon la revendication 1, **caractérisé en ce que** la totalité du gène M, du gène F et du gène HN est remplacée par les gènes reprogrammants mammifères.

3. Virus Sendai selon la revendication 1 ou 2, **caractérisé en ce que** c'est une particule virale.

4. Virus Sendai selon l'une quelconque des revendications 1 à 3, **caractérisé en ce que** au moins un gène parmi les gènes M, F et HN est soumis à une insertion ou un remplacement par un gène marqueur.

5. Vecteur de virus Sendai chargé par un gène reprogrammant pour générer une cellule souche pluripotente induite, **caractérisé en ce qu'**il comprend le virus Sendai selon l'une quelconque des revendications 1 à 4.

6. Vecteur de virus Sendai chargé par un gène reprogrammant pour générer une cellule souche pluripotente induite selon la revendication 5, où le vecteur de virus Sendai comprend, sur son génome, une séquence cible pour un microARN spécifique ajouté dans une région non codante de protéine du gène L, du gène NP ou du gène P, dans lequel le microARN spécifique apparaît seulement dans une cellule reprogrammée.

7. Vecteur matrice pour préparer un virus Sendai chargé par un gène reprogrammant, **caractérisé en ce que** le vecteur est un vecteur de clonage recombinant pour le virus Sendai selon l'une quelconque des revendications 1 à 4 comprenant le génome du virus Sendai modifié tel que défini dans l'une quelconque des revendications 1 à 4.

8. Vecteur matrice selon la revendication 7, **caractérisé en ce que** la totalité du gène M, du gène F et du gène HN est remplacée par les gènes reprogrammants mammifères.

9. Vecteur matrice selon la revendication 7 ou 8, **caractérisé en ce que** le vecteur de clonage est un vecteur phagique.

10. Vecteur matrice selon la revendication 9, **caractérisé en ce que** le vecteur phagique est un vecteur phagique λ.

11. Vecteur matrice selon l'une quelconque des revendications 7 à 10, **caractérisé en ce qu'**il comprend un ADN.

12. Vecteur matrice selon la revendication 11, **caractérisé en ce qu'**il comprend une séquence cible pour un microARN spécifique,
dans lequel la séquence cible est ajoutée dans une région non codante de protéine du gène L, du gène NP ou du gène P,
dans lequel le microARN spécifique apparaît seulement dans une cellule reprogrammée.

13. Cellule **caractérisée en ce qu'**elle est transfectée avec le vecteur matrice selon l'une quelconque des revendications 7 à 12 sous réserve que la cellule ne soit pas une cellule embryonnaire humaine.

14. Cellule selon la revendication 13, **caractérisée en ce qu'**une ARN polymérase T7 est exprimée à l'intérieur.

15. Cellule selon la revendication 14, **caractérisée en ce qu'**elle est en outre transfectée avec le gène NP, le gène P et le gène L.

16. Cellule selon la revendication 15, **caractérisée en ce qu'**elle est en outre transfectée avec au moins un gène parmi le gène M, le gène F et le gène HN, dans laquelle le au moins un gène inclut un ou des gène(s) qui est/sont remplacé(s) par des gènes reprogrammants mammifères dans le vecteur matrice selon l'une quelconque des revendications 7 à 12.

17. Méthode pour la production d'un vecteur de virus Sendai chargé par un gène reprogrammant, **caractérisée en ce qu'**elle comprend la culture de la cellule selon la revendication 16 à une température de production de particule virale de 32°C, dans un milieu de culture, pour récupérer une particule de virus Sendai qui comprend, en tant que son génome:
(i) des gènes de virus Sendai incluant le gène NP, le gène P/C et le gène L; et
(ii) des gènes reprogrammants mammifères comprenant une combinaison de Oct3/4, Sox2 et Klf4 ou une combinaison de Oct3/4, Sox2, Klf4 et c-Myc;
dans laquelle au moins un gène parmi le gène M, le gène F et le gène HN dans le génome est remplacé par les gènes reprogammants mammifères;
dans laquelle le gène L est un gène muté et code pour une séquence d'acides aminés de la protéine L dans laquelle le résidu d'acide aminé à la position 1618 est une valine; et
dans laquelle chacun du gène M, du gène F et du gène HN est un gène dérivé d'une souche de virus Sendai CI.151; et
dans laquelle la cellule selon la revendication 16 est une cellule produisant un vecteur viral.

18. Méthode in vitro pour la production d'une cellule souche pluripotente induite, **caractérisée en ce qu'**elle comprend les étapes de: infection d'une cellule mammifère différenciée avec le vecteur de virus Sendai chargé par un gène reprogrammant selon la revendication 5 pour reprogrammer la cellule mammifère différenciée; et transfection de la cellule reprogrammée avec un ARNsi et ensuite laisser l'ARNsi agir sur le vecteur pour retirer le vecteur de virus Sendai chargé par un gène reprogrammant de la cellule,
dans laquelle l'ARNsi est conçu pour cibler le gène L du vecteur de virus Sendai.

19. Méthode in vitro pour la production d'une cellule souche pluripotente induite, **caractérisée en ce qu'**elle comprend les étapes de: infection d'une cellule différenciée avec le vecteur de virus Sendai chargé par un gène reprogrammant selon la revendication 6 pour reprogrammer la cellule différenciée; et ensuite retrait automatique du vecteur de virus Sendai chargé par un gène reprogrammant, après la formation d'une cellule souche pluripotente induite qui est la cellule exprimant le microARN spécifique.

20. Méthode pour la production d'une cellule souche pluripotente induite, **caractérisée en ce qu'**elle comprend les étapes de: infection d'une cellule différenciée avec le vecteur de virus Sendai chargé par un gène reprogrammant selon la revendication 5 ou 6 pour reprogrammer la cellule différenciée; et ensuite culture de la cellule dans des conditions de température élevée pour favoriser le retrait du vecteur de virus Sendai chargé par un gène reprogrammant de la cellule.
